# EUROPEAN PATENT SPECIFICATION

(11) **EP 1 761 783 B1**
(45) Date of publication and mention of the grant of the patent: **29.07.2009**
(21) Application number: 05744506.6
(22) Date of filing: 25.05.2005
(51) Int. Cl.: G01N 33/68, C07K 5/00, G01N 33/543, C07K 7/00

(54) **IDENTIFICATION OF COMPOUNDS MODIFYING A CELLULAR RESPONSE**
IDENTIFIZIERUNG VON EINE ZELLULÄRE ANTWORT MODIFIZIERENDEN VERBINDUNGEN
IDENTIFICATION DE COMPOSES MODIFIANT UNE REPONSE CELLULAIRE

(30) Priority: 25.05.2004 DK 200400821; 25.05.2004 DK 200400822
(43) Date of publication of application: 14.03.2007
(73) Proprietor: 2cureX ApS, 3460 Birkerød (DK)
(72) Inventor: MELDAL, Morten, DK-2400 Copenhagen (DK); NIELSEN, Thomas, Eiland, DK-1663 Copenhagen (DK); HAGEL, Grith, DK-2791 Dragør (DK); KAZNELSON, Dorte, Wissing, DK-2200 Copenhagen N (DK); DINESS, Frederik, DK-2100 Copenhagen Ø (DK); THASTRUP, Ole, DK-3460 Birkerød (DK)
(74) Representative: HOEIBERG A/S
(86) International application number: PCT/DK2005/000348
(87) International publication number: WO 2005/116643

(56) References cited:
- WO-A-03/038431
- JUDICE, WAGNER A. S. ET AL: "Carboxydipeptidase activities of recombinant cysteine peptidases: cruzain of Trypanosoma cruzi and CPB of Leishmania mexicana" EUROPEAN JOURNAL OF BIOCHEMISTRY , 271(5), 1046-1053 CODEN: EJBCAI; ISSN: 0014-2956, 2004, XP002345206
- CHEMICAL ABSTRACTS, vol. 98, no. 13, 28 March 1983 (1983-03-28), Columbus, Ohio, US; abstract no.: 107766, DAMIROV, A. G. ET AL: "Fragmentary conformational analysis of the Arg6-Lys13 segment of the dynorphin molecule" XP002345203 & DOKLADY - AKADEMIYA NAUK AZERBAIDZHANSKOI SSR , 38(6), 53-7 CODEN: DAZRA7; ISSN: 0002-3078, 1982,
- PENNINGTON M E ET AL: "THE USE OF A COMBINATORIAL LIBRARY METHOD TO ISOLATE HUMAN TUMOR CELL ADHESION PEPTIDES" MOLECULAR DIVERSITY, ESCOM SCIENCE PUBLISHERS, LEIDEN, NL, vol. 2, no. 1-2, 1996, pages 19-28, XP001041521 ISSN: 1381-1991
- LAM KIT S ET AL: "A one-bead one-peptide combinatorial library method for B-cell epitope mapping" METHODS : A COMPANION TO METHODS IN ENZYMOLOGY, ACADEMIC PRESS INC., NEW YORK, NY, US, vol. 9, no. 3, 1996, pages 482-493, XP002231929 ISSN: 1046-2023
- CARDARELI PINA M ET AL: "The collagen receptor alpha-2-beta-1, from MG-63 and HT1080 cells, interacts with a cyclic RGD peptide" JOURNAL OF BIOLOGICAL CHEMISTRY, AMERICAN SOCIETY OF BIOLOGICAL CHEMISTS, BALTIMORE, MD, US, vol. 267, no. 32, 15 November 1992 (1992-11-15), pages 23159-23164, XP002231928 ISSN: 0021-9258

### SEQUENCE LISTING

<110> Carlsberg A/S
<120> Identification of compounds modifying a cellular response
<130> P791PC00
<160> 70
<170> Patent In version 3.1
<210> 1
   <211> 7
   <212> PRT
   <213> Artificial Sequence
<220>
   <223> Cell adhesion peptide
   <400>
   Ala Arg Ile Arg Ile Gln His
   1               5
<210> 2
   <211> 7
   <212> PRT
   <213> Artificial Sequence
<220>
   <223> Cell adhesion peptide
   <400> 2
   Ala Lys Cys Arg Trp Cys Met
   1               5
<210> 3
   <211> 7
   <212> PRT
   <213> Artificial Sequence
<220>
   <223> Cell adhesion peptide
   <400> 3
   Ala Lys Ala Arg Cys Lys Ser
   1               5
<210> 4
   <211> 7
   <212> PRT
   <213> Artificial Sequence
<220>
   <223> Cell adhesion peptide
   <400> 4
   Ala Lys Tyr Trp Ser Tyr Lys
   1 5
<210> 5
   <211> 7
   <212> PRT
   <213> Artificial Sequence
<220>
   <223> Cell adhesion peptide
   <400> 5
   Ala His Leu Tyr Arg Asn Lys
   1 5
<210> 6
   <211> 7
   <212> PRT
   <213> Artificial Sequence
<220>
   <223> Cell adhesion peptide
   <400> 6
   Ala Arg Arg Cys Phe Arg Asp
   1 5
<210> 7
   <211> 7
   <212> PRT
   <213> Artificial Sequence
<220>
   <223> Cell adhesion peptide
   <400> 7
   Ala Ala Arg His Cys Tyr Tyr
   1 5
<210> 8
   <211> 7
   <212> PRT
   <213> Artificial Sequence
<220>
   <223> Cell adhesion peptide
   <400> 8
   Ala Tyr Tyr Cys Gln Gln Arg
   1 5
<210> 9
   <211> 7
   <212> PRT
   <213> Artificial Sequence
<220>
   <223> Cell adhesion peptide
   <400> 9
   Ala Asp Leu Lys Arg Pro Met
   1 5
<210> 10
   <211> 7
   <212> PRT
   <213> Artificial Sequence
<220>
   <223> Cell adhesion peptide
   <400> 10
   Ala Gly Gly Lys Arg Lys Phe
   1 5
<210> 11
   <211> 7
   <212> PRT
   <213> Artificial Sequence
<220>
   <223> Cell adhesion peptide
   <400> 11
   Ala Pro Arg Lys Arg Cys Gly
   1 5
<210> 12
   <211> 7
   <212> PRT
   <213> Artificial Sequence
<220>
   <223> Cell adhesion peptide
   <400> 12
   Ala Thr Arg Arg Val Ala Arg
   1 5
<210> 13
   <211> 7
   <212> PRT
   <213> Artificial Sequence
<220>
   <223> Cell adhesion peptide
   <400> 13
   Ala Gly Lys Lys Asn Lys Asn
   1 5
<210> 14
   <211> 7
   <212> PRT
   <213> Artificial Sequence
<220>
   <223> Cell adhesion peptide
   <400> 14
   Ala Ala Lys Arg Trp Lys Phe
   1 5
<210> 15
   <211> 7
   <212> PRT
   <213> Artificial Sequence
<220>
   <223> Cell adhesion peptide
   <400> 15
   Ala Arg Trp Pro Tyr Arg Gly
   1 5
<210> 16
   <211> 7
   <212> PRT
   <213> Artificial Sequence
<220>
   <223> Cell adhesion peptide
   <400> 16
   Ala Leu Tyr Trp Thr Trp Arg
   1 5
<210> 17
   <211> 7
   <212> PRT
   <213> Artificial Sequence
<220>
   <223> Cell adhesion peptide
   <400> 17
   Ala Ala Tyr Arg Trp Tyr Arg
   1 5
<210> 18
   <211> 7
   <212> PRT
   <213> Artificial Sequence
<220>
   <223> Cell adhesion peptide
   <400> 18
   Ala Arg Cys Ile Arg Gly Asp
   1 5
<210> 19
   <211> 7
   <212> PRT
   <213> Artificial Sequence
<220>
   <223> Cell adhesion peptide
   <400> 19
   Ala Thr Lys Cys Lys Gly Arg
   1 5
<210> 20
   <211> 7
   <212> PRT
   <213> Artificial Sequence
<220>
   <223> Cell adhesion peptide
   <400> 20
   Ala Val Tyr Met Arg Asn Ile
   1 5
<210> 21
   <211> 7
   <212> PRT
   <213> Artificial Sequence
<220>
   <223> Cell adhesion peptide
   <400> 21
   Ala Arg Lys Arg Ile Arg Gln
   1 5
<210> 22
   <211> 7
   <212> PRT
   <213> Artificial Sequence
<220>
   <223> Cell adhesion peptide
   <400> 22
   Ala Lys Ile Arg Glu Lys Arg
   1 5
<210> 23
   <211> 7
   <212> PRT
   <213> Artificial Sequence
<220>
   <223> Cell adhesion peptide
   <400> 23
   Ala Arg Arg Phe Lys Met Tyr
   1 5
<210> 24
   <211> 4
   <212> PRT
   <213> Artificial Sequence
<220>
   <223> Cell adhesion peptide
   <400> 24
   Arg Arg Phe Lys
   1
<210> 25
   <211> 4
   <212> PRT
   <213> Artificial Sequence
<220>
   <223> Cell adhesion peptide
   <400> 25
   Arg Arg Ile Arg
   1
<210> 26
   <211> 6
   <212> PRT
   <213> Artificial sequence;
<400> 26
   Leu Arg His Arg Leu Lys
   1 5
<210> 27
   <211> 5
   <212> PRT
   <213> Artificial sequence;
<400> 27 <210> 28
   <211> 6
   <212> PRT
   <213> Artificial sequence;
<400> 28
<210> 29
   <211> 6
   <212> PRT
   <213> Artificial sequence;
<400> 29
   Ile Arg Tyr Arg Leu Arg
   1 5
<210> 30
   <211> 6
   <212> PRT
   <213> Artificial sequence;
<400> 30
   Ala Gln Arg Pro Arg Trp
   1 5
<210> 31
   <211> 6
   <212> PRT
   <213> Artificial sequence;
<400> 31
   Trp Tyr Ala Lys Arg Arg
   1 5
<210> 32
   <211> 8
   <212> PRT
   <213> Artificial sequence;
<400> 32
   Lys Arg Ile Arg Gln Arg Leu Arg
   1 5
<210> 33
   <211> 7
   <212> PRT
   <213> Artificial sequence;
<400> 33
   Lys Arg Ile Arg Gln Arg Lys
   1 5
<210> 34
   <211> 5
   <212> PRT
   <213> Artificial sequence;
<400> 34
   Arg Ile Arg Gln Arg
   1 5
<210> 35
   <211> 5
   <212> PRT
   <213> Artificial sequence;
<400> 35
   Arg Gln Arg Ile Arg
   1 5
<210> 36
   <211> 6
   <212> PRT
   <213> Artificial sequence;
<400> 36
   Lys Phe Gly Gln Lys Cys
   1 5
<210> 37
   <211> 6
   <212> PRT
   <213> Artificial sequence;
<400> 37
   Arg Arg Leu Leu Pro Ile
   1 5
<210> 38
   <211> 6
   <212> PRT
   <213> Artificial sequence;
<400> 38
   Pro Phe Arg Lys Lys Cys
   1 5
<210> 39
   <211> 6
   <212> PRT
   <213> Artificial sequence;
<400> 39
   Tyr Arg Trp Arg Ile Ala
   1 5
<210> 40
   <211> 6
   <212> PRT
   <213> Artificial sequence;
<400> 40
   Arg Ser Lys Arg Ile Asn
   1 5
<210> 41
   <211> 6
   <212> PRT
   <213> Artificial sequence;
<400> 41
   Arg Ser Ala Lys Arg Cys
   1 5
<210> 42
   <211> 6
   <212> PRT
   <213> Artificial sequence;
<400> 42
   Lys Lys Gln Phe Trp Phe
   1 5
<210> 43
   <211> 6
   <212> PRT
   <213> Artificial sequence;
<400> 43
   Arg Met Lys Leu His Lys
   1 5
<210> 44
   <211> 6
   <212> PRT
   <213> Artificial sequence;
<400> 44
   Arg His Trp Gly Arg Ile
   1 5
<210> 45
   <211> 6
   <212> PRT
   <213> Artificial sequence;
<400> 45
   Thr Lys Arg Leu Lys Thr
   1 5
<210> 46
   <211> 6
   <212> PRT
   <213> Artificial sequence;
<400> 46
   Thr Lys Gly Lys Ala Lys
   1 5
<210> 47
   <211> 6
   <212> PRT
   <213> Artificial sequence;
<400> 47
   Ala Lys Thr Arg His Arg
   1 5
<210> 48
   <211> 6
   <212> PRT
   <213> Artificial sequence;
<400> 48
   Asn Arg Pro Arg Val Arg
   1 5
<210> 49
   <211> 6
   <212> PRT
   <213> Artificial sequence;
<400> 49
   Val Pro Arg Lys Val Gln
   1 5
<210> 50
   <211> 6
   <212> PRT
   <213> Artificial sequence;
<400> 50
   Lys Met Arg Tyr Cys Gln
   1 5
<210> 51
   <211> 6
   <212> PRT
   <213> Artificial sequence;
<400> 51
   Ile Arg Lys His Leu Ile
   1 5
<210> 52
   <211> 6
   <212> PRT
   <213> Artificial sequence;
<400> 52
   Pro Arg Arg Val Val Ile
   1 5
<210> 53
   <211> 6
   <212> PRT
   <213> Artificial sequence;
<400> 53
   Lys Arg Glu Ser Lys Arg
   1               5
<210> 54
   <211> 6
   <212> PRT
   <213> Artificial sequence;
<400> 54
   Ser Arg Lys Asp Arg Lys
   1               5
<210> 55
   <211> 6
   <212> PRT
   <213> Artificial sequence;
<400> 55
   Arg Cys Lys Lys Leu Ile
   1               5
<210> 56
   <211> 6
   <212> PRT
   <213> Artificial sequence;
<400> 56
   Arg Lys Leu Arg Val Asn
   1               5
<210> 57
   <211> 6
   <212> PRT
   <213> Artificial sequence;
<400> 57
   Val Arg Thr Val Arg Val
   1 5
<210> 58
   <211> 6
   <212> PRT
   <213> Artificial sequence;
<400> 58
   Arg Ala Phe Lys Tyr Tyr
   1 5
<210> 59
   <211> 6
   <212> PRT
   <213> Artificial sequence;
<400> 59
   Ile Thr Arg Arg Thr Gln
   1 5
<210> 60
   <211> 6
   <212> PRT
   <213> Artificial sequence;
<400> 60
   Lys Met Pro Lys Lys Asn
   1 5
<210> 61
   <211> 6
   <212> PRT
   <213> Artificial sequence;
<400> 61
   Lys Pro Lys Met Met Cys
   1 5
<210> 62
   <211> 6
   <212> PRT
   <213> Artificial sequence;
<400> 62
   Lys Lys Met Arg Phe Trp
   1 5
<210> 63
   <211> 6
   <212> PRT
   <213> Artificial sequence;
<400> 63
   Lys Lys Lys Phe Tyr Tyr
   1 5
<210> 64
   <211> 6
   <212> PRT
   <213> Artificial sequence;
<400> 64
   Lys Ser Asn Lys Val Arg
   1 5
<210> 65
   <211> 6
   <212> PRT
   <213> Artificial sequence;
<400> 65
   Lys Trp Pro His His Arg
   1 5
<210> 66
   <211> 6
   <212> PRT
   <213> Artificial sequence;
<400> 66
   Arg His Ile Gln Trp Tyr
   1 5
<210> 67
   <211> 6
   <212> PRT
   <213> Artificial sequence;
<400> 67
   Leu Arg Leu Lys Pro Lys
   1               5
<210> 68
   <211> 6
   <212> PRT
   <213> Artificial sequence;
<400> 68
   Glu Arg Lys Arg Cys Thr
   1               5
<210> 69
   <211> 6
   <212> PRT
   <213> Artificial sequence;
<400> 69
   Arg Arg Ala Arg Gln Asp
   1               5
<210> 70
   <211> 6
   <212> PRT
   <213> Artificial sequence;
<400> 70
   Arg Glu Lys Gly Ala Arg
   1               5

## Description

### Field of invention

The present invention relates to a method and tools for extracting information relating to an influence, for example on a surface receptor, in particular an influence caused by contacting a receptor with a substance linked to a solid support to which a cell expressing the surface receptor is attached. In particular the method related to a solid support that allow chemical synthesis of individual substances on beads of the solid support

The method of the invention may be used as a very efficient procedure for testing or discovering the influence of a library of substances on a physiological process, for example in connection with screening for new drugs, testing of substances for toxicity, identifying drug targets for known or novel drugs. Other valuable uses of the method and technology of the invention will be apparent to the skilled person on the basis of the following disclosure

### Background of invention

Combinatorial synthesis of peptide as well as small-molecule libraries has proven very useful as a method for generating vast numbers of highly diverse compounds (see for example Comprehensive Survey of Combinatorial Library Synthesis: 2002 Roland E. Dolle J. Comb. Chem.,2003, pp 693 - 753). To fully exploit this high capacity of Combinatorial chemistry to produce huge numbers of compounds several technologies have been developed that allow screening directly on the solid support (M.Meldal, 1994, METHODS: A companion to methods of enzymology 6:417-424). In the field of drug discovery such methods have successfully been applied for example for the identification of enzyme modulators. The library can be synthesized on resin beads that each carry one specific compound, and these "one-bead-one compound" libraries are then screened against the purified biological component of interest (e.g. cellular proteins or peptides),
Before progressing active compounds, identified though such procedure, further in the drug discovery process, the compound will have to be re-synthesized and tested for efficacy In a cell-based or in-vivo test system.
Novel ways to screen combinatorial libraries in a physiological more correct way are assumed to greatly accelerate the drug discovery process, and show importance in areas like chemo-genomics and chemo-proteomics.
Screening of combinatorial libraries in intact cells have been done by capturing mammalian or yeast cells together with a limited number of resin-beads in a "nanodroplet" (Borchart et al. Chem Biol 1997 4:961). Compounds immobilized on the resin are released through disruption of a photo-cleavable linker and the compound-associated effects on the intact cells are monitored.
In an alternative method the compounds are released through acidolysis resin-beads carrying the library members area are spread out an a lawn of mammalian calls, and the spatial localization of a cellular response is monitored and beads in that region is isolated, and the remaining compound is structure elucidated Jayawickreme et al, 1998, Combinatorial peptide Library Protocols, Ed. Shmuel Cabilly, Humana Press, p. 107-128).
WO03/038431 describes methods for screening combinatorial bead libraries by capturing cells from body fluids. Beads comprising a compound enabling cells to adhere to said bead may be selected.
US2003/0059764 describes multiplexed cell analysis systems using non-positional or positional arrays of coded carriers.

Wagner et all., 2004, European Journal of Biochemistry, 271 (5), 1046-1053 describes - carboxypeptidase activities of recombinant cysteine peptidases. Examples of peptidase substrates are described including for example Abz-RRFK-OH.

Damirov et al., 1983, Chemical Abstracts, vol. 98, no. 13, abstract no. 107766 describes conformational analysis of dynorphin fragments for example of CaCO-Arg6-Arg7-Ile8-Arg9-NHCα.

### Summary of invention

It is of great importance to provide new and efficient methods for identification of compounds influencing specific cellular processes. In particular, such methods wherein a very large quantity of candidate compounds may be tested for a specific effect on a cell within a relatively short period of time.

It is therefore an object of the present invention to provide very efficient procedures for testing or discovering the influence of compounds of a library on a physiological process in a cell. In particular, the methods provides means for testing very large numbers of different compounds for one or more physiological effects within a rather short time period. This may be obtained by attaching living cells to resin beads coupled to a test compound. The test compounds may thus influence physiological processes in said cells. Said influence(s) may be detected and beads containing cells displaying the desired influence(s) may be selected. Once selected the compounds coupled to the selected beads may be identified. These methods may for example be very useful in connection with screening for new drugs, testing of substances for toxicity, identifying drug targets for known or novel drugs.

Accordingly, it is a first objective of the invention to provide methods of identifying a compound modifying at least one cellular response, wherein each cellular response is linked to different reporter systems generating detectable outputs, said method comprising the steps of:
(a) Providing multiple resin beads capable of supporting growth of cells, wherein each resin bead comprises one member of a library of test compounds and wherein at least two beads comprise different library members; and
(b) Attaching cells comprising said reporter system(s) onto said solid support wherein
   - cells are directly attached to the solid support, and/or
   - at least 10% of the solid supports comprise cell adhesion molecules as well as said library member, and cells adhere to said cell adhesion molecules; and
(c) Screening said resin beads for beads comprising cells meeting at least one predetermined selection criterion, wherein said selection criterion is linked directly or indirectly to a detectable output; and
(d) Selecting beads comprising cells meeting said at least one selection criterion; and
(e) Identifying said the library member, thereby identifying a compound modifying said at least one cellular response.

### Description of Drawings

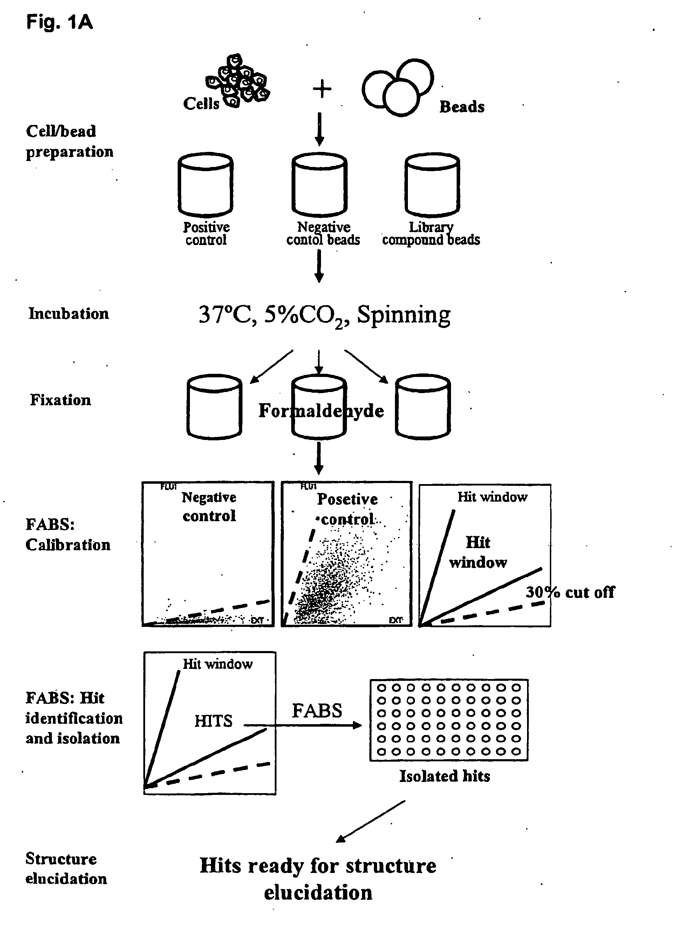

Figure 1A illustrates a method of identifying a resin bead comprising a compound influencing a cellular response linked to a reporter system generating a fluorescent output. The method involves cultivating cells on resin beads, fixing cells, FABS calibration using a positive and a negative control, identification and isolation of positive hits.
   A method of identifying a resin bead comprising a compound influencing a cellular response linked to a reporter system generating a detectable fluorescent output uses a plate reader or image acquisition analysis. The method involves 1) Grow cells on beads for 24 hrs and Fix cells in EtOH, 2) Add app. 20 beads to each well and identify hit wells using plate reader or image acquisition/analysis and 3) Transfer beads from hit wells to a new 384 well plate - one bead/well and identify hit wells using plate reader or image acquisition. If for example 500.000 beads are screened with 20 beads/well, approx. 25.000 wells, i.e. approx. 68 plates must be screened. With a 0.1% hit rate, there will be approx. 500 hit wells comprising approx. 10.000 beads, which amounts to analysis of approx. 27 plates in the second round. Alternatively, positive beads may be picked directly (preferably without fixation) after the first identification using image acquisition analysis. The method may for example be used for analysis of expression of a Cre-YFP construct.
Figure 2A illustrates a multiplexed screen involving FABS and microscopy. The screen involves I) Identification of positive hits by FABS as displayed in figure 1, followed by II) a step of microscopy identifying resin beads comprising cells with an internal fluorescent signal. The screen could for example be a screen for Cre-YFP and MC4R-GFP or HA-MC4R internalisation, wherein I) Cre-YFP reporter hits are identified and isolated by FABS and II) MC4R-GFP or HA-GFP Internalisation positive hits are picked.
Figure 2B illustrates a multiplexed screen involving two FABS analysis. The screen involves I) Identification of positive hits by FABS as displayed in figure 1, followed by II) a second FABS analysis. The screen could for example be a screen for Cre-YFP and HA-MC4R Internalisation, wherein I) Cre-YFP reporter hits are identified and isolated by FABS into a 10 ml. tube (see figure 1) and II) HA-MC4R internalisation hits are isolated (=low fluorescence).
Figure 3 illustrates a plasmid map of pCRE-d2EGFP
Figure 4A illustrates synthesis of Ac-His-(D)phe-Arg-Trp-NH₂,
Figure 4B illustrates synthesis of Ac-His-(D)phe-Arg-Trp-Gly-PEGA₁₉₀₀
Figure 4C illustrates synthesis of Fmoc-Dap(N₃)
Figure 5 illustrates synthesis of the cyclic peptide of example 3
Figure 6a illustrates synthesis of a combinatorial library (6a) via an intramolecular N-acyliminium Pictet-Spengler reaction
Figure 6b illustrates synthesis of a combinatorial library (6b) via an intramolecular N-acyliminium Pictet-Spengler reaction
Figure 7 illustrates spectra and structure determination by accurate mass differences from single beads
Figure 8 illustrates structure determination by accurate mass differences from single beads
Figure 9 illustrates a fragmentation pathway

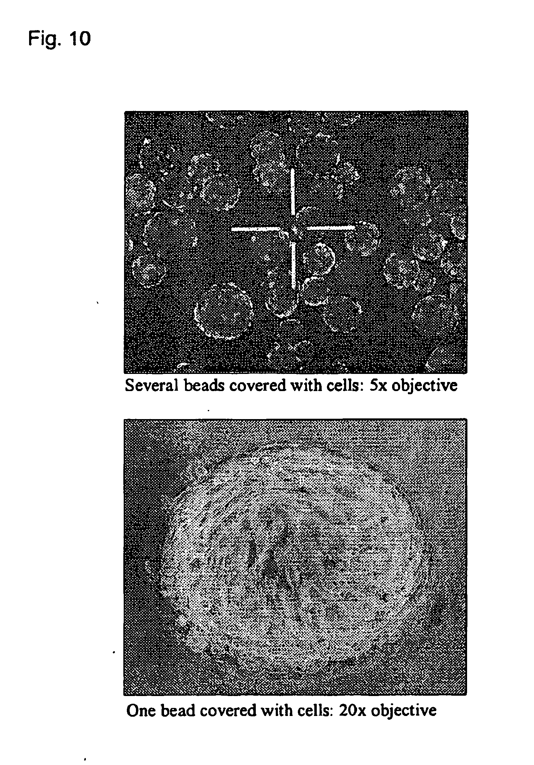

Figure 10 illustrates examples of an adhesion peptide displaying bead covered with cells (U2OS).
Figure 11 illustrates quantification of MC4R-GFP internalization on beads
Figure 12 illustrates intracellular Ca²⁺ mobilization as visualised using the Flou4 probe.
Figure 13 illustrates the aMSH induced CRE-YFP transcription in HEK293 and U2OS cells, respectively, expressing MC4.
Figure 14 illustrates signal obtained from a subfraction of identified hits after functional screening (CRE-YFP) of a library.
Figure 15a is a picture of a bead with cells screened as described in Example 14a comprising the compound designated TEN-636-36-36.
Figure 15b illustrates QTOF MSMS analysis of the compound designated TEN-636-33-26.
Figure 16 illustrates MSMS analysis of material cleaved from a single bead of a library prepared as described in Examples 6a or 6b. Structure elucidation is given by [M+H]⁺, [M-Gly-AA1]⁺, and [M-Gly-AA₁-AA₂]⁺.

### Definitions

Naturally occurring amino acids are named herein using either their 1-letter or 3-letter code according to the recommendations from IUPAC, see for example http://www.chem.qmw.ac.uk/iupac. If nothing else is specified amino acids may be of D or L-form. In the description (but not in the sequence listing) 3-letter codes starting with a capital letter indicate amino acids of L-form, whereas 3-letter codes in small letters indicate amino acids of D-form.

The term "a" as used herein, can mean one or more, depending on the context in which it is used.

In the present context, the term "green fluorescent protein" or (GFP) is intended to indicate a protein which, when expressed by a cell, emits fluorescence upon exposure to light of the correct excitation wavelength (cf. [(Chalfie et al. 1994)]). "GFP" as used herein means any protein or fragment thereof capable of fluorescing when excited with appropriate radiation. This includes fluorescent proteins that are either naturally occuring or engineered and proteins that have been modified to be fluorescent. Naturally occuring fluorescent proteins have been isolated from the jellyfish, Aequorea vistoria, the sea pansy, Renilla reniformis, Phialidium gregarium and Discosoma coral (W.W. Ward et al. (1982) Photochem. Photobiol, 35:803-808; Levine et al. (1982) Biochem. Physiol., 72B:77-85; Fradkov et al. (2000), FEBS Lett. 479:127-130). GFPs have also been engineered to emit different colors and to fluoresce more intensely in mammalian organisms (U.S. Pat. Np. 5,625,048; WO 97/28261; WO 96/23810; EP0851874; US6,172,188; WO01/98338).
A variety of Aequorea-related fluorescent proteins have been engineered to have different excitation and emission spectra by modifying the naturally occuring amino acid sequence (D.C. Prasher et al. (1992) Gene 111:229-233; Heim et al. (1994) Proc. Natl. Acad. Sci. USA 91: 12501-12504; US. Pat. No. 5,625,048; WO 96/23810 and PCT/US97/14593).

The term "living cell" is used to indicate a cell which is considered living according to standard criteria for that particular type of cell such as maintenance of normal membrane potential, cell membrane integrity and energy metabolism

The terms "image processing" and "image analysis" are used to describe a large family of digital data analysis techniques or combination of such techniques which reduce ordered arrays of numbers (images) to quantitative information describing those ordered arrays of numbers. When said ordered arrays of numbers represent measured values from a physical process, the quantitative information derived is therefore a measure of the physical process.

The term "fluorescent probe" is used to indicate a fluorescent fusion polypeptide comprising a GFP or any functional part thereof which is N- or C-terminally fused to a biologically active polypeptide as defined herein, optionally via a peptide linker consisting of one or more amino acid residues, where the size of the linker peptide in itself is not critical as long as the desired functionality of the fluorescent probe is maintained. A fluorescent probe according to the invention is expressed in a cell and basically mimics the physiological behaviour of the biologically active polypeptide moiety of the fusion polypeptide.

The term "determining the fluorescence" is used to describe the process used to monitor a change in fluorescence properties.

The term "bioluminescence" is used to describe a process where light is produced through a chemical reaction that natively is occuring in a biological system. For the reaction to occur at least two chemicals are required: the one that produces the light (called "luciferin") and the other (called "luciferase") that catalyzes the reaction. Sometimes the luciferin and luciferase are brought together in one single unit (called "photoprotein" an example of the last group is aequorin.

The term "FRET" is used to describe the occurrence of Fluorescence resonance energy transfer between a fluorophore donor and an acceptor chromophore. It is a distance-dependent interaction between the electronic excited states of two fluorophores in which excitation is transferred from a donor fluorophore to an acceptor chromophore without emission of a photon. The efficiency of FRET is dependent on the inverse sixth power of the intermolecular separation, making it useful over distances comparable with the dimensions of biological macromolecules. Thus, FRET is an important technique for investigating interactions between cellular molecules for example complex formation.

The term "BRET" is used to describe a process that is related to FRET, but differs from FRET in that donor is a bioluminescent protein like luciferase that generates its own luminescence emission in the presence of a substrate, and that can pass the energy to an acceptor fluorophore. For either BRET or FRET to work, the donor's emission spectrum must overlap the acceptor's absorption spectrum, their transition dipoles must be in an appropriate orientation, and the donor and acceptor must be in close proximity (usually within 30-80 A of each other, depending on the degree of spectral overlap).

The term "Scintillation Proximity Assay" is used to describe an assay determining the distance between two compounds, wherein one compound (bound to a bead) will emit light when radiation from an isotope occurs in close proximity and the other compound is containing a radioactive isotope.

The term "mammalian cell" is intended to indicate any cell of mammalian origin. The cell may be an established cell line, many of which are available from The American Type Culture Collection (ATCC, Virginia, USA) or a primary cell with a limited life span derived from a mammalian tissue, including tissues derived from a transgenic animal, or a newly established immortal cell line derived from a mammalian tissue including transgenic tissues, or a hybrid cell or cell line derived by fusing different celltypes of mammalian origin e.g. hybridoma cell lines. The cells may optionally express one or more non-native gene products, e.g. receptors.

The phrase "fluorescence properties" means absorption properties, such as wavelength and extension, or spectral properties of the emitted light, such as wavelength, fluorescence lifetime, intensity or polarisation, or the intracellular localisation of the fluorophore. It may thus be localised to a specific cellular component (e.g. organelle, membrane, cytoskeleton, molecular structure) or it may be evenly distributed throughout the cell or parts of the cell.

The term "fixed cells" is meant to cover cells treated with a cytological fixative such as glutaraldehyde, methanol, acetone or formaldehyde, treatments which serve to chemically cross-link and/or stabilize soluble and insoluble proteins within the structure of the cell or to dehydrate cells. Once in this state, such proteins cannot be lost from the structure of the now-dead cell.

The term "cell line" is meant to cover a group of cells, wherein the cells of that group are essentially genetically indistinguishable from each other. The cells of a cell line are thus all progeny of the same cell.

The term "comprising" should be understood in an inclusive manner. Hence, by way of example, a composition comprising compound X, may comprise compound X and optionally additional compounds.

The term "multiple" should be understood as "at least two".

The term "library of test compounds" should be understood as a collection of test compounds comprising at least 2 different test compounds.

The term "small organic molecules or compounds" refers herein to non-oligomeric, carbon containing compounds producible by chemical synthesis and generally having a size of less than 600 mass units.

The term "one bead-one compound library" refers to libraries immobilised on resin beads, wherein each individual resin bead does not comprise more than one library member in one or multiple copies. In a particular form of such libraries each member is represented by multiple fragments of said member obtained by ladder synthesis encoding.

The term "one bead-two compound library" refers to libraries immobilised on resin beads, wherein each individual resin bead does not comprise more than one library member in one or multiple copies and wherein each individual resin bead in addition to said library member also comprises an adhesion compound. All beads may comprise identical adhesion compounds.

### Detailed description of the invention

### Library of test compounds

In the present invention, libraries of compounds are used to screen for compounds having a desired physiological influence on a living cell. As used herein, the term "library" means a collection of molecular entities or test compounds, herein also designated "library members" obtained after a series of chemical transformation.

In preferred embodiments of the present invention the library is a combinatorial library. Non-limiting examples of combinatorial libraries that may be used with the present invention and methods of producing such libraries are given in: Comprehensive Survey of Combinatorial Library Synthesis: 1998 Roland E. Dolle and Kingsley H. Nelson, Jr. J. Comb. Chem., 1999, pp 235 - 282; Comprehensive Survey of Combinatorial Library Synthesis: 1999 Roland E. Dolle J. Comb. Chem., 2000, pp 383 - 433; Comprehensive Survey of Combinatorial Library Synthesis: 2000 Roland E. Dolle J. Comb. Chem.,2001, pp 477 - 517; Comprehensive Survey of Combinatorial Library Synthesis: 2001 Roland E. Dolle J. Comb. Chem.,2002, pp 369 - 418 and Comprehensive Survey of Combinatorial Library Synthesis: 2002 Roland E. Dolle J. Comb. Chem.,2003, pp 693 - 753. The skilled person will appreciate that these protocols may be easily be adapted to specific need of a particular embodiment of the present invention.

In one embodiment, these molecular entities can be natural oligomers (oligomers of building blocks occurring in Nature) such as peptides, glycopeptides, lipopeptides, nucleic acids (DNA or RNA), or oligosaccharides. By way of example, a natural oligomer may be any peptide consisting of naturally occurring amino acid, even if said peptide comprises a sequence not present in nature. The libraries may comprise different natural oligomers or the libraries may comprise only one kind of natural oligomer, for example the library may be a peptide library. In another embodiment, they can be unnatural oligomers (oligomers comprising one or more building blocks not occurring in Nature) such as chemically modified peptides, glycopeptides, nucleic acids (DNA or RNA), or, oligosaccharides, and the like. Said chemical modification may for example be the use of unnatural building blocks connected by the natural bond linking the units (for example, a peptide amide linkage), the use of natural building blocks with modified linking units (for example, oligoureas as discussed in Boeijen et al, 2001, J. Org. Chem., 66: 8454-8462; oligosulfonamides as discussed in Monnee et al, 2000, Tetrahedron Lett., 41: 7991-95), or combinations of these (for example, statine amides as discussed in Dolle et al, 2000, J. Comb. Chem., 2: 716-31.). Preferred unnatural oligomers include oligomers comprising unnatural building blocks connected to each other by a naturally occurring bond linking. Said oligomers may thus comprise a mixture of naturally occurring and unnatural building blockslinked to each other by naturally occurring bonds. By way of example, the oligomer may comprise naturally occurring amino acids and unnatural building blocks linked by peptide bonds f.x. PNA or LNA. Thus, in one embodiment of the invention preferred oligomers comprise modified amino acids or amino acid mimics). Other preferred unnatural oligomers include, for example oligoureas, poly azatides, aromatic C-C linked oligomers and aromatic C-N linked oligomers. Still other preferred oligomers comprise a mixture of natural and unnatural building blocks and natural and unnatural linking bonds. For example, the unnatural oligomer may be any of the oligomers mentioned in recent reviews see: Graven et al., 2001, J. Comb. Chem., 3: 441-52; St. Hilaire et al., 2000, Angew. Chem. Int. Ed. Engl., 39: 1162-79; James, 2001, Curr. Opin. Pharmacol., 1: 540-6; Marcaurelle et al., 2002, Curr. Opin. Chem. Biol., 6: 289-96; Breinbauer et al., 2002. Angew. Chem. Int. Ed. Engl., 41: 2879-90. The libraries of the invention may also comprise cyclic oligomers, for example cyclic natural oligomers, such as cyclic peptides or cyclic unnatural oligomers. In certain embodiments of the invention, libraries of cyclic oligomers may be advantegous to use due to the rigid structure. This may result in higher selectively and affinity.

In yet another embodiment, the molecular entities may comprise non-oligomeric molecules such as peptidomimetics or other small organic molecules. Peptidomimetics are compounds that mimic the action of a peptidic messenger, such as bicyclic thiazolidine lactam peptidomimetics of L-proplyl-L-leucyl-glycinamide (Khalil et al, 1999, J. Med. Chem., 42: 2977-87). In a preferred embodiment of the invention, the library comprises or even more preferably consists of small organic molecules. Small organic molecules are non-oligomeric compounds of less than about 600 mass units containing any of a variety of possible functional groups and are the product of chemical synthesis, or isolated from nature, or isolated from nature and then chemically modified, and include, for example, Bayer's urea-based kinase inhibitors (Smith et al., 2001, Bioorg. Med. Chem. Lett., 11: 2775-78). Small organic compounds may for example be selected from the group consisting of alcohols, ethers, carboxylic acids, aryloxy, acyloxy, thiol, alkylthio, arylthio, heteroarylthio, sulphonyl, sulphoxy, amino, alkylamino, dialkylamino, acylamino, diacylamino, alkoxycarbonylamino, amides, alkyl, branched alkyl, aryl, heteroaryl, nitro, cyano, halogeno, silyloxy, keto, heterocycles, fused ring systems, fused heterocycles and mixtures thereof, wherein each of the aforementioned may be substituted independently on each position with one or more groups selected from the group consisting of -H, -OH, -SH, halogen, carboxyl, carbonyl, alkoxy, aryloxy, acyloxy, alkylthio, arylthio, heteroarylthio, sulphonyl, sulphoxy, amino, alkylamino, dialkylamino, acylamino, diacylamino, alkoxycarbonylamino, amides, alkyl, aryl, heteroaryl, nitro, cyano, halogeno, silyloxy, keto, heterocycles, fused ring systems, and fused heterocycles.
Non-limiting examples of small organic molecule libraries that may be used with the present invention and methods of producing them may for example be found in the reviews Thompson et al., 1996, Chem. Rev., 96: 555-600; Al-Obeidi et al., 1998, Mol. Biotechnol., 9: 205-23; Nefzi et al., 2001, Biopolymers, 60: 212-9; Dolle, 2002, J. Comb. Chem., 4: 369-418.

The libraries according to the invention may comprise at least 20, such as at least 100, for example at least 1000, such as at least 10,000, for example at least 100,000, such as at least 1,000,000 different test compounds. Preferably, the libraries comprises in the range of 20 to 10⁷, more preferably 50 to 7,000,000, even more preferably 100 to 5,000,000, yet more preferably 250 to 2,000,000 different compounds. In a very preferred embodiment of the present invention the libraries comprises in the range of 1000 to 20,000, such as in the range of 20,000 to 200,000 different test compounds. In preferred embodiments of the invention the library comprises in the range of 10,000 to 1,000,000 different test compounds.

Preferably, the libraries to be used with the present invention are immobilised on resin beads. Said resin beads may be any of the beads described herein below. At least 2, preferably at least 20, more preferably at least 100, even more preferably at least 1000, yet more preferably at least 10,000, for example at least 100,000, such as at least 1,000,000 resin beads comprising different library members, i.e. different test compounds may be used with the methods according to the invention. Preferably, the in the range of 20 to 10⁷, more preferably 100 to 7,000,000, even more preferably 1000 to 5,000,000, yet more preferably 5000 to 2,000,000, even more preferably 10,000 to 1,000,000 resin beads comprising different library members, are used with the methods according to the invention.

In one very preferred embodiment of the invention, each resin bead does not comprise more than one library member in one or more copies, i.e. each resin bead only comprises on kind of test compound, however said test compound may be present on the resin bead in multiple copies. Such libraries may also be designated one-bead-one-compound libraries. Preferably, each resin beads comprises sufficient copies of said library member in order to exert the desired influence of cells attached to said resin bead and in order to analyse the chemical structure of the compound. Such libraries may be prepared by different methods, for example by a split/mix method or by coupling individually a specific compound to a bead. One-bead-one compound libraries offer the advantage that once a resin bead has been selected according to the methods described herein, the desired compound may easily be identified (see useful methods herein below).

The libraries may in one preferred embodiment be synthesized directly on resin beads using a split/mix method (vide infra) which gives rise to one-bead-one-compound libraries. Split/mix methods in general comprise the steps of:
1. Providing several pools of resin beads
2. Performing one or more different chemical synthesis steps on each pool of rein beads,
3. Splitting said pools to obtain fractions
4. Mixing fractions from different pools, thereby obtaining new pools
5. Optionally repeating step 1 and 4

Alternatively steps 3 and 4 may be as follows:
3. Mixing all pools of resin beads, thereby obtaining a mixed pool
4. Splitting the mixed pool of resin beads into reaction containers thereby obtaining new pools.

One-bead-one-compound libraries may for example be prepared as described in M. Meldal, Multiple column synthesis of quenched solid-phase bound fluorogenic substrates for characterization of endoprotease specificity in Methods: A Companion to Methods in Enzymology 6:417-424, 1994 or in M. Meldal, The One-bead Two-Compound Assay for Solid Phase Screening of Combinatorial Libraries in Biopolymers, Peptide Science 66:93-100, 2002; or in Combinatorial peptide library protocols, Ed. by Shmuel Cabilly, Humana Press, 1998, p. 1-24 and 51 to 82.

In another embodiment of the invention the library may be a one-bead-two-compounds library. Each individual resin bead of such a library comprises only one library member in one or more copies. In addition each individual resin bead comprises a second compound, such as a cell adhesion compound. The cell adhesion compound could for example be any of the cell adhesion compounds mentioned herein below. It is comprised within the invention that several library resin beads, such as all library resin beads comprises identical adhesion compound(s) in one or more copies. One-bead-two-compound libraries may for example be prepared by a method involving the steps of:
1. Providing resin beads comprising a plurality of reactive groups
2. Reacting said reactive groups with two chemical moeities comprising different and preferably orthogonal protective groups
3. Deprotecting a subset of the reactive groups by removal of one kind of protective groups, preferably selective removal of one kind of protective group,
4. Attaching or synthezising a split/mix library of test compounds to the deprotected reactive group
5. Deprotecting the remaining reactive groups by removal the other kind of protective group
6. Attaching the second compound to the deprotected reactive groups

The method may also be performed by first attaching the second compound and then synthezising the library. Accordingly, the steps of the method may be performed in the following order: 1, 2, 3, 6, 5 and 4. The library of test compounds may be first synthesized and then attached to the resin beads or it may be synthesized directly into the resin bead. Similarly, the second compound may be first synthesized and then attached to the resin beads or it may be synthesized directly into the resin bead.

Preferred resin beads are described in the section "resin beads" herein below. The reactive group may be any suitable reactive group, preferably however, the reactive group is either a hydroxyl group, a thiol or a primary amino group. The reactive may also preferably be an azido or a secondary amino group. The protective group may be any suitable protective group known to the person skilled in the art, such as acid labile, alkaline labile or photolabile protective groups, preferably the protective group is selected from the group consisting of Fmoc, Boc, Alloc and N₃. It is preferred that the different protective groups may be removed by different treatment, for example that if one protective group is acid labile, then the other is not acid labile, but instead for example alkaline labile or photo labile. In an preferred embodiment one protective group is Fmoc and the other protective group is Alloc or N₃. Step 3 may for example be performed by a split/mix method as described herein above, thereby generating a one-bead-one-compound library. The second compound is preferably a cell adhesion compound.

In one embodiment the library may be linked to the resin bead via a linker, which may be a cleavable linker. This may for example be achieved by synthesizing the linker directly on resin beads or coupling the linker to the resin beads and subsequently coupling or synthesizing the library onto the resin beads. Thus, before coupling of the library the linker preferably comprises a protective group as described herein above. The cleavable linker may be any of the cleavable linkers described herein below. If the resin beads are coupled to an adhesion compound via a cleavable linker it is preferred that the cleavable linker linking the library is different to the cleavable linker linking the adhesion compound. It is in particularly preferred that the linker are not cleavable by the same mechanism. Thereby, the library may be specifically released from the resin beads, without release of adhesion compounds.

In yet another embodiment of the invention the library may be a mixed compound library, wherein each individual resin bead comprises a plurality of library members.

Selection of an appropriate library is dependent upon the specific embodiment of the invention. For example, a totally random library designed to contain interesting and greatly diverse compounds may be used with the invention. An advantage of this approach is that the outcome of the screening is not prejudiced in any specific manner. Since the invention permits screening of millions of diverse compounds, for example, immobilized on resin beads, a large number, for example in the range of 3 to 5 million, of random molecules can be used in the ligand library.

Alternatively, a smaller, targeted library (hundreds to thousands of compounds) can be used, for example, starting with a known compound or compounds, and providing numerous variations of these known compounds for targeted screening. For example, in embodiments of the invention wherein compounds modulating the activity of a specific cell surface molecule, a compound known to modulate said specific cell surface molecule may be used as starting compound for the preparation of a targeted library. Alternatively, a smaller targeted library of compounds mimicking a compound known to modulate the activity of said cell surface molecule may be prepared, for example using computer aided modelling followed by chemical synthesis. The smaller, targeted library can also comprise random molecules. Examples of libraries and methods of preparing such libraries, which may useful in embodiments of the invention, wherein the cellular response is mediated through a G-protein coupled receptor are described in C. Haskell-Luevano, A. Rosenquist, A. Souers, K. C. Khong, J. A. Ellman, and R. D. Cone, 1999, J.Med.Chem. 42:4380-4387. Compounds that activate the mouse melanocortin-1 receptor identified by screening a small molecule library based upon the b-tum. J.Med.Chem. 42:4380-4387, 1999; A. J. Souers, A. A. Virgilio, Å. Rosenquist, W. Fenuik, and J. A. Ellman. Identification of a potent heterocyclic ligand to somatostatin receptor subtype 5 by the synthesis and screening of b-tum mimetic libraries. J.Am.Chem.Soc. 121 (9):1817-1825, 1999; J. Bondebjerg, Z. Xiang, R. M. Bauzo, C. Haskell-Luevano, and M. Meldal. A solid phase approach to mouse melanocortin receptor agonists derived from a novel thioether cyclized peptidomimetic scaffold. J.Am.Chem.Soc. 124:11046-11055, 2002; B. A. Harrison, G. W. Pasternak, and G. L. Verdine. 2,6-dimethyltyrosine analogues of a stereodiversified ligand library: highly potent, selective, non-peptidic m opioid receptor agonists. J.Med.Chem. 46:677-680, 2003; G. R. Marshall. Peptide interactions with G-protein coupled receptors. Peptide Science 60:246-277, 2003; P.N.Arasasingham, C.Fotsch, X.Ouyang, M.H.Norman, M.G.Kelly, K.L.Stark, B.Karbon, C.Hale, J.W.Baumgartner, M.Zambrano, J.Cheetham, N.A.Tamayo, and Structure-Activity relationship of (1-aryl-2-piperazinylethyl) piperazines: Antagonists for the AGRP/Melanocortin receptor binding. J.Med.Chem. 46:9-11, 2003. Further useful libraries are described in examples 4, 5 and 6 herein below. The person skilled in the art will appreciate that other libraries may be prepared by adapting the protocols described in the aforementioned referecences. The library may contain a parallel array of random modifications of one or more test compounds. In one embodiment, the library may be formed as a parallel array of random modifications to a known compound or compounds. The term "parallel array" is meant to cover synthesis of a library by subjecting a given compound to a known set of reactions in an isolated vessel or well. Thus, the nature of a compound in a given container or well is known. The array of test compounds is preferably prepared directly on resin beads using techniques known by those skilled in the art. Briefly, the resin may be portioned into a number of vessels or wells, usually less than 500 and the reagents added. There is in general no mixing step and after the appropriate washing steps, subsequent reactions are carried out by addition of additional reagents to the wells. There is no exponential increase in the number of compounds generated and that is equal to the number of vessels used. The compound can be easily identified by keeping track of the reagent added to each well.

The library may also have been prepared by parallel synthesis using a tag to enable identification of, what chemical synthesis steps the individual resin bead has been submitted to. This may for example be done by IRORI or radiofrequency tag. Alternatively, chemical synthesis steps may be performed in parallel to preparing a polymeric tag. Identification of the tag will thus provide knowledge of the compound.

Attachment of a label to a compound may alter the properties of said compound. Hence, in one embodiment of the present invention, the compounds of the library are not labelled, i.e. the compounds are not connected to a detectable label, such as a fluorescent component, a nucleic acid or a nucleic acid homologue such as PNA, a dye, a probe comprising a reactive moiety or the like. In particular it is preferred that all compounds are not connected to the same detectable label.

The present invention also describes to methods of synthezising libraries of test compounds, wherein said libraries are in particular useful for the screening methods of the invention.

The invention thus describes methods of synthesising a cyclic peptide or peptide mimetic library, comprising the steps
i) Providing a plurality of peptides or peptide mimetics, (preferably peptides) covalently linked to an azide moiety and an acetylene moeity; and
ii) cyclizing said peptide or peptide mimetic through a Cu(I) catalysed reaction between said azide- and said acetylene moiety; and
iii) thereby obtaining a library of cyclic peptides or peptide mimetics.

Each peptide preferably only comprises one azide moeity and one acetylene moiety. An example of a method of preparing such a library is given in example 4 herein below.

The invention also describes methods of synthesising a library of heterocyclic ureas, comprising the steps of
i) Providing a plurality of urea containing peptide aldehydes; and
ii) Subjecting said urea containing peptides to an intramolecular Pictet-Spengler reaction; and
iii) Whereby obtaining a library of heterocyclic ureas

Said urea containing peptide aldehydes are preferably peptides covalently linked to at least one urea moeity and one aldehyde moeity. The intramolecular Pictet-Spengler reaction may for example be performed as described in WO2004/113362 claiming priority from Danish patent application PA 2003 00967, both are hereby incorporated by reference.

An example of a method of preparing such libaries is given in examples 5 and 5a herein below.

The peptides used for preparation of any of the libraries mentioned above may be oligomers of naturally occurring or not naturally occurring amino acids or a mixture of both, preferably they are oligomers of the 20 amino acids naturally present in proteins, wherein said amino acids may be in either D- or L-form. It is preferred that each peptide (or peptide mimetic) is immobilised on a solid support, such as any of the solid supports mentioned herein below. More preferably the solid support is resin beads and it is preferred that each resin bead comprises only one library member in one or more copies.

Preferably at least 2, such as at least 10, for example at least 100, such as at least 1000, for example at least 10.000 different peptides and/or peptide mimetics are provided. Each peptide may comprise in the range of 2 to 100 amino acids, such as in the range of 2 to 50 amino acids, for example 2 to 25 amino acids, such as in the range of 2 to 15 amino acids, for example 2 to 10 amino acids, such as in the range of 3 to 8 amino acids, for example 4 to 6 amino acids,

The invention also relates to libraries prepared by any of the methods described above.

Libraries of heterocyclic compounds obtained by cyclisation of a peptide aldehyde through an intramolecular Pictet-Spengler reaction may also be used with the present invention. Such libraries may for example be any of the libraries described in WO2004/113362 claiming priority from Danish patent application PA 2003 00967.

### Resin beads

The library members of this invention are preferably bound to a solid support. Preferred solid supports to be used with the present invention are resin beads (see herein below).

The solid support may however also be a spot or region on a surface or a plated gel or a membrane. A spot or a region is a defined area on said surface, to which the library member is covalently bound. One can therefore envisage one surface comprising a plurality of spots or regions, wherein each such spot or region is covalently attached to only one library member in one or more copies. Said surface could for example be a silicium wafer, a glass surface, a plastic surface or a gel.
Plastic surface may for example be prepared from polystyrene, polycarbonate polypropylene, ethylene and/or teflon. Gels could be prepared from for example poly acrylamid or PEGA.

In this invention however, the compounds of the library are preferably bound to a resin bead, conferring the advantage of compartmentalized "mini-reaction vessels" for attachment of cells.

In general more compounds may be screened and several of the steps in the procedure may be performed on one bead with sufficient material. Hence, preferably, the library is bound to resin beads. Each member of the library is a unique compound and is physically separated in space from the other compounds in the library, preferably, by immobilizing the library on resin beads, wherein each bead at the most comprises one member of the library. Depending on the mode of library synthesis, each library member may contain, in addition, fragments of the library member. Since ease and speed are important features of this process invention, it is preferred that the screening step take place on the same solid support used for synthesis of the library, and also that identification of the members of the binding pair can take place on the same support, such as on a single resin bead. Thus, preferred solid supports useful in the process invention satisfy the criteria of not only being suitable for organic synthesis, but are also suitable for screening procedures, such as "on-bead" screening as well as suitable for attachment of cells. It is furthermore preferred that the resin bead is suitable for "on-bead" identification of library members as described herein below. The resin bead may be prepared from any suitable material such as polystyrene, polyethylene polyacrylamide,controlled pore glass or PEG. The resin bead could thus for example be selected from the group consisting of Toyopearl, sepharose, sephadex, CPG, silica, POPOP, PEGA, SPOCC, Expansin, Tentagel, Argogel, Polystyrene, Jandagel, polydimethylacrylamide resin, Polyacrylamide resin, kieselghur supported resins and polystyrene supported resins. Hydrophilic supports are preferred. Examples of preferred hydrophilic resin beads includes TentaGel (commercially available from Rapp polymere, Tübingen, Germany), ArgoGel (commercially available from Argonaut Technologies Inc., San Carlos, CA), PEGA (commercially available from VersaMatrix, Copenhagen), POEPOP (Renil et al., 1996, Tetrahedron Lett., 37: 6185-88; available from Versamatrix, Copenhagen, Denmark) and SPOCC (Rademann et al, 1999, J. Am. Chem. Soc., 121: 5459-66; available from Versamatrix, Copenhagen, Denmark). Examples of on-bead screening attempts are described in the following references: Chen et al., 1996, Methods Enzymol., 267: 211-19; Leon et al., 1998, Bioorg. Med. Chem. Lett., 8: 2997-3002; St. Hilaire et al., 1999, J. Comb. Chem., 1: 509-23; Smith et al., 1999, J. Comb. Chem., 1: 326-32; Graven et al., 2001. J. Comb. Chem. 3: 441-52; Park et al., 2002, Lett. Peptide Sci., 8: 171-78). TentaGel and ArgoGel are made up of polyethylene glycol chains grafted on to a polystyrene core. However, use of these supports in biological screening is limited by a size restriction, and by denaturation of certain proteins, particularly enzymes.

Preferred resin beads according to the present invention are resin beads, useful for on-bead library synthesis, screening and identification of ligand/protein. Hence, preferred resins according to the present invention are resin comprising polyethylene glycol. More preferably, the resin is PolyEthyleneGlycol Acrylamide copolymer (PEGA), Super Permeable Organic Combinatorial Chemistry (SPOCC) or PolyOxyEthylene-PolyOxyPropylene (POEPOP) resin. Another preferred resin comprises a crosslinked polyacrylamide resin.

PEGA (PolyEthyleneGlycol Acrylamide copolymer; Meldal M., 1992, Tetrahedron Lett., 33: 3077-80), POEPOP (PolyOxyEthylene-PolyOxyPropylene; Renil et al., 1996, Tetrahedron Lett., 37: 6185-88) and SPOCC (Super Permeable Organic Combinatorial Chemistry; Rademann et al, 1999, J. Am. Chem. Soc., 121: 5459-66) resins are made primarily of polyethylene glycol and swell well in organic as well as aqueous solvents. Because they have very reduced or no non-specific binding, PEGA and SPOCC resins have been effectively used in the screening of myriad proteins including enzymes of different classes. Furthermore, these resins are available in different pore sizes and can allow large proteins to enter while retaining activity. For example, PEGA6000 resins allow proteins up to 600 kDa to enter. In the Examples below, PEGA4000 and PEGA1900 resin with a molecular weight cut off of 200 and 90 kDa, respectively, are used for screening. In principle, any hydrophilic support that is useful for compartmentalized synthesis, retains the activity of the proteins, and has minimal non-specific binding, may be used in this process invention.

One aspect of the invention relates to a method comprising the step of providing multiple resin beads capable of supporting growth of cells. Preferably, all resin beads provided are capable of supporting growth of cells. In one preferred embodiment all resin beads are similar and each is capable of supporting growth of cells, wherein the resin beads only differs by comprising different library members. In embodiments of the invention wherein the resin beads comprise a cell adhesion molecule, it is preferred that at least 10%, more preferably at least 20%, even more preferably at least 30%, yet more preferably at least 40%, even more preferably at least 50%, yet more preferably at least 60%, %, even more preferably at least 70%, yet more preferably at least 90%, even more preferably essentially all, yet more preferably all resin beads comprise the cell adhesion molecule as well as a library member.

### Cells

The cells to be used with the present invention may be any useful cells available or prepared for the purpose. Preferably, the cells are selected from the group consisting of mammalian cells. For example the cells may be human cells. The cells may be cells capable of growing in suspension or they may be adherent cells. Adherent cells may preferably be cultivated directly on the resin beads used with the invention (see also herein below). It is preferred that the cells are adherent cells. Cells with a better adherence are preferred over cells with a poorer adherence. Cells which adhere well to resin beads comprising an adhesion compound as described herein above are very preferred.

Cells could for example be primary cells or established cell lines. Preferred cell lines include but are not limited to those mentioned in Table 1.

**Table 1**

| **Cell line** | **Species** | **Tissue** | **Morphology** |
|---|---|---|---|
| 3T3-L1 | Mouse | Embryonic fibroblast | Fibroblast |
| 3T3-Swiss albino (CCL-92) | Mouse | Embryo | fibroblast |
| A10 | Rat | thoracic aorta | Myoblast |
| Att 20 | Mouse | Pituitary | Small round cells |
| BAE | Cow | Aorta | Endothelial |
| Balb/c | Mouse | Embryonic fibroblast | Fibroblast |
| BHK:R P.1#4aa PTP1B fl | | | |
| BHK-21 | Hamster | Kidney | Fibroblast |
| BHK467 | Hamster | Kidney | |
| BHK570 | Hamster | Kidney | Fibroblast |
| BJ | Human | Foreskin | Fibroblast |
| C2C12 | Mouse | Muscle | Myoblast |
| Caki-1 | Human | Kidney | Epithelial |
| CAL-54 | Human | Kidney | Epithelial |
| CHOh1R | Chinese hamster | Ovary | Fibroblast |
| CHO-K1 | Hamster | Ovary | Epithelial |
| COS 1 | Monkey | Kidney | Fibroblast |
| COS 7 | Monkey | Kidney | Fibroblast |
| G-8 | Mouse | Muscle | Myoblast |
| GT1-7 | | | |
| HCT 116 | Human | Colorectal | Epithelial |
| HEK293 | Human | Embryonic kidney | Epithelial |
| Hela | Human | Cervix adenocardnoma | Epithelial |
| HEP-G2 | Human | Liver | Epithelial |
| HT-1080 | Human | Fibrosarcoma | Epithelial |
| HT-29 | Human | Colon | Epithelial |
| HUVEC | Human | Umbilical vein | Endothelial |
| Ins-1 | | | |
| Jurkat done E6-1 | Human | T lymphocyte | Lymphoblastoid |
| K-562 | Human | Bone marrov | Lymphoblastoid |
| L-6 | Rat | Muscle | Myoblast |
| MCF 7 | Human | Mammary Gland | Epithelial |
| MDA-MB-231 | Human | Adenocardnoma | Epithelial |
| MDA-MB-468 | Human | Mammary Gland | Epithelial |
| MDCK | Canine | Kidney | Epithelial |
| Min 6 | | | |
| Mv 1 Lu (NBL-7) | Mink | Lung | Epithelial |
| NIH-3T3 | Mouse | Embryo | Fibroblast |
| PAE | Pig | Aorta | |
| PC 12 | Rat | Adrenal gland | |
| PC-3 | Human | Prostate | Epithelial |
| RAT2 | Rat | Normal | Fibroblast |
| RAW 264.7 | Mouse | | Monocyte |
| RIN | Rat | | Epithelial |
| SK-ML-28 | Human | Melanoma | |
| SK-N-AS | Human | Neuroblastoma | Epithelial |
| SK-N-DZ | Human | Neuroblastoma | Epithelial |
| SK-N-F1 | Human | Brain | Epithelial |
| SK-NM-C | Human | Neuroepithelioma | Epithelial |
| SK-N-SH | Human | Caucasian neuroblastoma | Epithelial |
| SW480 | Human | Colorectal | Epithelial |
| U-2 OS | Human | Bone, osteosarcoma | Epithelial |
| U-87 MG | Human | Brain | Epithelial |
| U937 | Human | Lymphoma | Monocyte |
| VERO | Monkey | Kidney | Fibroblast-like |
| WI-38 | Human | Lung | Fibroblast |
| WM-266-4 | Human | Skin | Epithelial |
| WEHI | Human | | |

In one embodiment of the invention the cells have been genetically or otherwise modified in order to enhance their usability with the present invention. The modification may be stable or only transient or a mixture of both. For example, the cells may have been modified to contain one or more of the reporter systems described herein below. Depending on the nature of the reporter system this may be achieved by a number of different methods. For example, if the reporter system comprises a nucleic acid, said nucleic acid may be inserted into said cell by conventional recombinant techniques (see below).

In another preferred example the cell comprises a nucleic acid comprising a first nucleotide sequence encoding a cell surface molecule operably linked to a second nucleotide sequence not naturally associated therewith directing expression of said first sequence. The cell surface molecule may be any of the cell surface molecules described herein below. Such cells are in particular useful for identification of compounds modulating the activity of said cell surface molecule. Said nucleic acid may be introduced transiently or stably into said cells.

Useful second sequences includes for example promoters active in the particular cells, for example mammalian promoters, viral promoters or synthetic promoters. A large number of useful eukaryotic promoters are known to the person skilled in the art, useful promoters are for example described in "Mechanism of Transcription" (1998) Cold Spring Harbor Symposia on Quantitative Biology Vol. LXIII; Cold Spring Harbor Laboratory Press
Such promoters may be constitutively active or they may be active only temporarily.
In one example the promoter may be regulated by an external signal, for example the promoter may be inducible or repressable.

The nucleic acid may be inserted into the cells by any useful method, for example by conventional recombinant techniques, such as any of the techniques described in Sambrook et al., Molecular Cloning: A Laboratory Manual, 1989, Cold Spring Harbor Laboratory, New York, USA

In another embodiment the cells are primary cells. Primary cells are cells with a limited life span that preferably are derived from a mammalian tissue. Preferred primary cells are cells which are adherent. The mammalian tissue may for example be a human tissue, such as healthy or diseased tissue. In one embodiment the tissue is or comprises a neoplastic tissue, for example tissue removed from a cancer patient by surgery, for example from a patient suffering from melanoma, breast cancer or colon cancer. The tissue may also be hypertrophic cells, such as cardiac myocytes. Preferably said cancer patient has not been subjected to radiotherapy prior to surgery. In embodiments of the invention wherein the cells are primary cells it is preferred that the reporter system is endogenous to said primary cells.

### Cell attachment to resin beads and cell cultivation

The present invention relates to methods comprising the step of attaching cells comprising a reporter system(s) to resin beads. The cells may for example attach to said resin beads directly or by attaching a second compound conferring adhesion to the resin bead.

The resin beads useful for the present invention should preferably be able to support cell growth. The resin beads may per se be able to support cell growth, however frequently the resin beads will comprise a cell adhesion compound that enables the resin beads to support growth of cells. Said cell adhesion compound may be coupled to said resin beads by any useful means known to the person skilled in the art depending on the nature of the cell adhesion compound.

Any cell adhesion compound known to the person skilled in the art may be used with the present invention. It is frequently an advantage if the cell adhesion compound comprises at least one positively charged moiety at neutral pH, more preferably the cell adhesion compound has a positive overall netcharge at neutral pH.

In one preferred embodiment of the invention the cell adhesion compound comprises a peptide or a polypeptide, more preferably the cell adhesion compound consists of a peptide. Such peptides are herein also designated "adhesion peptides".

Said peptide preferably consists of in the range of 4 to 100, preferably in the range of 4 to 75, more preferably in the range of 4 to 50, even more preferably in the range of 4 to 30, yet more preferably in the range of 4 to 25, even more preferably in the range of 4 to 20, yet more preferably in the range of 4 to 15, such as in the range of 4 to 10, for example in the range of 4 to 8, for example in the range of 6 to 7 amino acids. In general, it is sufficient if the peptide comprises at least 4 amino acids.

It is preferred that the peptide comprises at least one amino acid selected from the group consisting of arginine and lysine, more preferably the peptide comprises at least 2 basic amino acids, such as 3 basic amino acids selected from the group consisting of Arg and Lys, even more preferably the peptide has an overall positive netcharge. In one preferred embodiment the peptide comprises the following sequence of 4 amino acids: basic-basic-lipophilic-basic. Basic amino acids may for example be selected from the group consisting of arginine and lysine, whereas the lipophilic amino acid may be selected from the group consisting of Gly, Ala, Val, Leu, Ile, Phe, Trp, Pro and Met of either D or L-form. Preferably, the peptide comprise at least 1, preferably at least 2, more preferably at least 3, even more preferably at least 4 amino acid on the D-form, yet more preferably all amino acids are on the D-form. Preferably D-amino acids are used to enhance the metabolic stability but also L-amino acids may be used.

Preferred examples of peptides useful as cell adhesion compounds are given in table 2 herein below:

**Table 2**

| No | 1 | 2 | 3 | 4 | 5 | 6 | 7 | | **SEQ ID NO** |
|---|---|---|---|---|---|---|---|---|---|
| 1 | ala | arg | ile | arg | ile | gln | his | | SEQ ID:1 |
| 2 | ala | lys | cys | arg | trp | cys | met | | SEQ ID:2 |
| 3 | ala | lys | ala | arg | cys | lys | ser | | SEQ ID:3 |
| 4 | ala | lys | tyr | trp | ser | tyr | lys | | SEO ID:4 |
| 5 | ala | his | leu | tyr | arg | asn | lys | | SEQ ID:5 |
| 6 | ala | arg | arg | cys | phe | arg | asp | | SEQ ID:6 |
| 7 | ala | ala | arg | his | cys | tyr | tyr | | SEQ ID:7 |
| 8 | ala | tyr | tyr | cys | gln | gln | arg | | SEQID:8 |
| 9 | ala | asp | leu | lys | arg | pro | met | | SEQ ID:9 |
| 10 | ala | gly | gly | lys | arg | lys | phe | | SEQ ID:10 |
| 11 | ala | pro | arg | lys | arg | cys | gly | | SEQ ID:11 |
| 12 | ala | thr | arg | arg | val | ala | arg | | SEQ ID:12 |
| 13 | ala | gly | lys | lys | asn | lys | asn | | SEQ ID:13 |
| 14 | ala | ala | lys | arg | trp | lys | phe | | SEQ ID:14 |
| 15 | ala | arg | trp | pro | tyr | arg | gly | | SEQ ID:15 |
| 16 | ala | leu | tyr | trp | thr | trp | arg | | SEQ ID:16 |
| 17 | ala | ala | tyr | arg | trp | tyr | arg | | SEQ ID:17 |
| 18 | ala | arg | cys | ile | arg | gly | asp | | SEQ ID:18 |
| 19 | ala | thr | lys | cys | lys | gly | arg | | SEQ ID:19 |
| 20 | ala | val | tyr | met | arg | asn | ile | | SEQ ID:20 |
| 21 | ala | arg | lys | arg | ile | arg | gln | | SEQ ID:21 |
| 22 | ala | lys | ile | arg | glu | lys | arg | | SEQ ID:22 |
| 23 | ala | arg | arg | phe | lys | met | tyr | | SEQ ID.23 |
| 24 | | arg | arg | phe | lys | | | | SEQ ID:24 |
| 25 | | arg | arg | ile | arg | | | | SEQ ID:25 |
| 26 | leu | arg | his | arg | leu | lys | | | SEQ ID:26 |
| 27 | lys | phe | gly | gln | lys | | | | SEQ ID:27 |
| 28 | lys | val | tyr | met | his | lys | | | SEQ ID.28 |
| 29 | ile | arg | tyr | arg | leu | arg | | | SEQ ID:29 |
| 30 | ala | gln | arg | pro | arg | trp | | | SEQ ID:30 |
| | trp | tyr | ala | lys | arg | arg | | | SEQ ID:31 |
| | lys | arg | ile | arg | gln | arg | leu | arg | SEQ ID:32 |
| | lys | arg | ile | arg | gln | arg | lys | | SEQ ID: 33 |
| | | arg | ile | arg | gln | arg | | | SEQ ID: 34 |
| | | arg | gln | arg | ile | arg | | | SEQ ID: 35 |
| | lys | phe | gly | gln | lys | cys | | | SEQ ID:36 |
| | arg | arg | leu | leu | pro | ile | | | SEQ ID:37 |
| | pro | phe | arg | lys | lys | cys | | | SEQ ID:38 |
| | tyr | arg | trp | arg | ile | ala | | | SEQ ID:39 |
| | arg | ser | lys | arg | ile | asn | | | SEQ ID:40 |
| | arg | ser | ala | lys | arg | cys | | | SEQ ID:41 |
| | lys | lys | gln | phe | trp | phe | | | SEQ ID:42 |
| | arg | met | lys | leu | his | lys | | | SEQ ID:43 |
| | arg | his | trp | gly | arg | ile | | | SEQ ID:44 |
| | thr | lys | arg | leu | lys | thr | | | SEQ ID:45 |
| | thr | lys | gly | lys | ala | lys | | | SEQ ID:46 |
| | ala | lys | thr | arg | his | arg | | | SEQ ID:47 |
| | asn | arg | pro | arg | val | arg | | | SEQ ID:48 |
| | val | pro | arg | lys | val | gln | | | SEQ ID:49 |
| | lys | met | arg | tyr | cys | gln | | | SEQ ID:50 |
| | ile | arg | lys | his | leu | ile | | | SEQ ID:51 |
| | pro | arg | arg | val | val | ile | | | SEQ ID:52 |
| | lys | arg | glu | ser | lys | arg | | | SEQ ID:53 |
| | ser | arg | lys | asp | arg | lys | | | SEQ ID:54 |
| | arg | cys | lys | lys | leu | ile | | | SEQ ID:55 |
| | arg | lys | leu | arg | val | asn | | | SEQ ID:56 |
| | val | arg | thr | val | arg | val | | | SEQ ID:57 |
| | arg | ala | phe | lys | tyr | tyr | | | SEQ ID:58 |
| | ile | thr | arg | arg | thr | gln | | | SEQ ID:59 |
| | lys | met | pro | lys | lys | asn | | | SEQ ID:60 |
| | lys | pro | lys | met | met | cys | | | SEQ ID:61 |
| | lys | lys | met | arg | phe | trp | | | SEQ ID:62 |
| | lys | lys | lys | phe | tyr | tyr | | | SEQ ID:63 |
| | lys | ser | asn | lys | val | arg | | | SEQ ID:64 |
| | lys | trp | pro | his | his | arg | | | SEQ ID:65 |
| | arg | his | ile | gln | trp | tyr | | | SEQ ID:66 |
| | leu | arg | leu | lys | pro | lys | | | SEQ ID:67 |
| | glu | arg | lys | arg | cys | thr | | | SEQ ID:68 |
| | arg | arg | ala | arg | gln | asp | | | SEQ ID:69 |
| | arg | glu | lys | gly | ala | arg | | | SEQ ID:70 |

Furthermore, preferred peptide may be any of the peptides identified by any of SEQ ID: 1 to 70, preferably any of SEQ ID: 1 to 23 and 26 to 35, such as SEQ ID: 1 to 23, for example SEQ ID: 25 to 35, wherein 3 amino acids, preferably 2 amino acids, more preferably 1 amino acid have been substituted for another amino acid. Preferably, said substition is a conservative substitution, i.e. substition for an amino acid with similar characteristics. Said characteristic could for example be acidic/basic properties, polarity or lipophilicity. It is also comprised within the invention that the peptide may be a peptide of above mentioned size comprising any of the peptides identified by SEQ ID: 1 to 70. In particular, in order to immoblised the peptide on a resin bead it may be useful to synthesise the adhesion peptide on an amino acid immobilized on the resin bead, for example a Gly.

In one embodiment the peptide is preferably selected from the group consisting of peptides identified by SEQ ID: 21 to 23 and 36 to 35, more preferably from the group consisting of 26 to 35, even more preferably SEQ ID:35. In another embodiment the peptide defined by SEQ ID:21 is preferred.

In one embodiment of the invention it is preferred that the peptide has low or essentially no fluorescent properties. It is particularly preferred that the peptide has low or essentially no fluorescent properties when attached to a solid support, such as a resin bead. By "essentially no fluorescent properties" is meant that the peptide does not emit any detectable fluorescence. This is in particularly relevant for embodiments of the invention wherein the detectable output is fluorescence (see herein below). Preferred peptides to use with this embodiment of the invention may be selected from the group consisting of SEQ ID:26 to 35.

Peptides useful as cell adhesion compounds may be identified using any suitable method. Said method may for example include the steps of
i) coupling a test peptide to a resin bead;
ii) incubating said resin bead with cells under cell cultivation conditions;
iii) testing whether said cells attach to said resin bead
iv) identification of the peptide sequence
wherein the test peptide is useful as cell adhesion compound if more cells attach to said resin bead in the presence, than in the absence of said test peptide. Preferably, the test peptide is useful as cell adhesion compound if at least 200, more preferably at least 500, even more preferably at least 1000 cells attach to said resin bead after incubation. This is in particular the case in embodiments of the invention, wherein the resin beads are PEGA beads. For example useful test peptides may be identified as described in example 1 herein below.

In embodiments of the invention wherein it is preferred that the peptide has no or low fluorescence it is preferred that the method comprises an additonal step performed at any point subsequent to step i), such as immediately subsequent to step i) prior to step ii). Said additional step comprises testing whether said peptide has fluorescent properties. This may for example be performed by sorting resin beads in a FABS or manually with the aid of a fluorescence microscope. If this is done prior to step ii) then only resin beads with no or low fluorescence properties are incubeted with cells, A non-limiting example of a useful method is described in example 1a.

The peptide may be coupled to the resin bead by any useful method, for example by synthesising the peptide directly onto an amino functionalised resin bead using a standard Fmoc-protocol for peptide synthesis. Other protective groups may be used instead of Fmoc, for example N₃ or Alloc. In one embodiment Alloc is the preferred protective group. It is preferred that different protecting gorup are used for synthesis of the adhesion peptide or for library synthesis. The peptide may also be synthesised by anchoring an Fmoc amino acid to a hydroxyl functionalised resin bead, such as a hydroxymethylbenzoic acid derivatised PEGA resin followed by peptide assembly using standard Fmoc technology as described in B. Blankemeyer-Menge, M. Nimtz, and R. Frank, An Efficient method for ancoring Fmoc-amino acids to hydroxyl- functionalised solid supports. Tetrahedron Lett. 31:1701-1704, 1990. Sidechains may be protected with acid labile protecting groups such as t-Bu, Trt, Pmc, Boc etc. The protected peptide may for example be cleaved off the resin using alkaline conditions or hydrazine and the structure may be determined e.g. by on bead Edman Degradtion. An non-limiting example of a method for synthesizing an adhesion peptide is given in example 5a, "Synthesis of adhesion peptide" herein below.

In one embodiment the adhesion compound may be linked to the resin bead via a linker, which may be a cleavable linker. This may for example be achieved by synthesizing the linker directly on resin beads or coupling the linker to the resin beads and subsequently coupling or synthesizing the library onto the resin beads. Thus, before coupling of the library the linker preferably comprises a protective group as described herein above. The cleavable linker may be any of the cleavable linkers described herein below. If the resin beads are coupled to the library via a cleavable linker it is preferred that the cleavable linker linking the adhesion compound is differentially cleavable.

In embodiments wherein cells adhere to the resin bead via the adhesion compound and the adhesion compound is attached to the resin bead via a cleavable linker, cells may be at least partially or even essentially fully released from the resin bead by cleavage of the cleavable linker.

Testing whether cells attach to resin beads may be done by any conventional methods, such as by manual inspection with the aid of a light microscope. If the cells have fluorescent properties, for example if the cells express a fluorescent protein, then resin beads with attached cells may be identified using a fluorescent microscope or a FABS, preferably a fluorescent microscope.

In one preferred embodiment of the invention, the cells may be cultivated directly on the resin beads. In general, a method of cultivating cells on resin beads may comprise the steps of
- Providing resin beads capable of supporting growth of cells
- Seeding cells onto said resin bead
- Incubating said resin beads comprising said cells in a cell culture medium under cell cultivation conditions
- Optionally allowing said cells to divide on said resin bead
- Thereby cultivating cells on resin beads

The cells may adhere actively to the resin beads and will then generally be referred to as adherent cells.

Cells cultivation conditions depends on the specific cells. For a large number of mammalian cells, such conditions comprise high humidity, preferably close to 100%, approximately 5% CO₂ and around 37°C. It is often desirable to keep the resin beads immersed in a suitable cultivation medium and frequently it is also desirable that the resin beads can be circulated within said medium, for example by stirring or rotation. Said stirring or rotation may be continuous or in intervals. It is also possible the container comprising the resin beads is simply rocked gently a few times every now and then.

In another embodiment of the invention cells may be attached to resin beads, without active adherence. For example, this may be the case for non-adherent cells, i.e. cells that may be cultivated in suspension.

In one embodiment of the invention more than one cell line or type of primary cell is attached to or cultivated on the beads. Hence for example 2, such as 3, for example 4, such as 5, for example 6, such as 7, for example 8, such as 9, for example 10, such as in the range of 10 to 20, for example in the range of 20 to 50, such as more than 50 different cell lines may be attached to or cultivated on said beads. Also different specific primary cells may be attached to the cultivated beads.

It is possible that a subgroup of resin beads only comprise one cell line or a specific kind of primary cells and another subgroup of resin beads comprises another cell line or another specific kind of primary cell and so forth. However, it is also possible that in principle every resin beads comprises all the different cell lines.
Intermediates between these two extremes may also be envisaged. Preferably, said different cell lines and/or primary cells comprise different reporter systems, hence it is possible that the different cell lines are derived from the same parent cell lined by insertion of different reporter systems. However, the different cell lines may also be unrelated.

### Cleavable linkers

The library of test compounds and/or the adhesion compound may in one embodiment be linked to the resin beads or solid supports by a cleavable linker.

The cleavable linker may be any chemical moiety which may be used to attach a molecule to a solid support either covalently or via complex formation, and thereafter is capable of releasing said molecule by the action of either acid, base, electrophiles, nucleophiles, oxidative agents, reductive agents, metals or light. Preferably, the cleavable linker attaches the library member/adhesion molecule to the solid support covalently. A comprehensive review describing *state of the art* for "cleavable linkers" is "Linkers and Cleavage Strategies in Solid-Phase Organic Synthesis and Combinatorial Chemistry", F. Guillier, D. Orain, and M. Bradley, Chem. Rev. 2000, 100, 2091-2157. Any of the cleavable linkers described therein may be used with the present invention.

Examples of useful acid labile linkers include the most commonly used linkers for acidic detachment from a solid support, the Wang and Rink linkers. Examples of useful base-labile linkers includes Wang and HMBA linkers, which may be cleaved under alkaline conditions. Light sensitive cleavable linkers are linkers which, upon the action of light with a given wave length and intensity, may release the library member/adhesion compound from the solid support. Photo-labile linkers cleavable by irradiation with UV-light may be *o*-nitrobenzyl type of linkers (nitrated analogs of the Wang linker), NBA type linkers or Holmes-type linkers. Paladium linkers may also be used with the invention.

### In one embodiment photolabile linkers are preferred

### Cell surface molecules

In one particularly preferred embodiment of the invention the methods of the invention involve identification of compounds modulating a cellular response, which is mediated through a cell surface molecule. Hence, the invention, for example may be useful for identifying compounds modulating the activity of a cell surface molecule, preferably a cell surface molecule capable of activating/repressing a signal transduction pathway. Within the context of the present invention the term "signal transduction pathway" should be understood in its common cell biological meaning, i.e. modulation of an intracellular event triggered by a cell surface receptor.

Signal transduction pathways may for example involve steps of phosphorylation, cleavage of proteins, synthesis of cAMP, activation of transcription, inhibition of transcription, change i intracellular Ca²⁺ concentration, change in membrane potential, subcellular relocalisation of cellular components, complex formation of cellular components, degradation of cellular components and/or change in energy metabolism

The cell surface molecule is preferably a protein, more preferably a protein that is accessible from the extracellular surface. Yet more preferably, the cell surface molecule is a cell surface protein receptor (herein also merely designated "receptor"). A "receptor" within the meaning of the present invention, is a molecule, which at least sometimes is localised at the cell surface and which is capable or associating with at least one ligand. The ligand binding site is accessible from the extracellular surface. Frequently, association with said ligand may alter the activity of the receptor.

In a preferred embodiment the cell surface molecule is a G-protein coupled receptor (GPCR). GPCR is a family of receptors coupled to a trimeric G-protein. GPCR to be used with the invention preferably have 7 transmembrane domains. Examples of useful GPCR are given in table 3.

GPCR may be divided into subfamilies, accordingly the GPCR may selected from the group consisting of GPCR belonging the rhodopsin like family, the secretin family or the metabotropic family, preferably from the group consisting of GPCR belonging the rhodopsin like family or the secretin family.

Rhodopsin like GPCR are also referred to as Class I GPCR. They are charaterised by a structurally similarity to the Rhodopsin receptor. Preferred examples of members of this family includes receptors for the following ligands: Acetylcholine (muscarinic & nicotinic), Adrenoceptors, Alpha Adrenoceptors, Beta Adrenoceptors, Dopamine, Histamine, Serotonin (5-HT), Angiotensin, Bradykinin, C5a anaphylatoxin, Fmet-leu-phe, Interleukin-8, ochernokine, Orexin, Nociceptin, CCK (Gastrin), Endothelin, Melanocortin including any of melanocortin 1 to 5 receptors, Neuropeptide Y, Neurotensin, Opioid, Somatostatin, Tachykinin (Substance P, NKA₁ ), Thrombin, vasopressin-like, Galanin, Follicle stimulating hormone, Lutropin-choriogonadotropic, Thyrotropin, Rhodopsin, Opsin, Prostaglandin, Lysophosphatidic Acid, Sphingosine- 1 -phosphate, Leukotriene, Prostacyclin, Thromboxane, Adenosine, Purinoceptors, Cannabis, Platelet activating factor, Gonadotropin-releasing Hormone, Thyrotropin-releasing hormone, Growth hormone-inhibiting factor or Melatonin.

Secretin like GPCR are also referred to as Class II GPCR. They are charaterised by a structurally similarity to the Secretin receptor. (Acession No NM_002980) Preferred examples of members of this family includes receptors for the following ligands: Secretin, calcitonin, Corticotropin releasing factor/urocortin, Gastric inhibitory peptide (GIP), Glucagon, Glucagon-like Peptide 1 (GLP- 1), Growth hormone-releasing hormone, Parathyroid hormone, PACAP or Vasoactive intestinal polypeptide (VIP).

Metabotropic GCPR are also referred to as class III GPCR. Preferred examples of members of this group includes receptors for the following ligands: Metabotropic Glutamate, GABA₈, or Extracellular Calcium Sensing.

In another preferred embodiment of the invention the GPCR is coupled to a G-protein, such as Gₛ, that stimulates adenylate cyclase. In yet another preferred embodiment of the invention the GPCR is coupled to a G-protein, such as Gₗ, that inhibits adenylate cyclase. Examples of GPCRs coupled to Gₛ or Gₗ are given in table 3.

### Gene Ontology Blast ServerFull

**Table 3**

| Gene symbol* Gene Ontology Blast Server | Full name |
|---|---|
| G-protein signaling, coupled to cyclic nucleotide second messenger | |
| NEUY_HUMAN | Neuropeptide Y precursor [Contains: Neuropeptide Y |
| ACM2_HUMAN | Muscarinic acetylcholine receptor M2 |
| SY02_HUMAN | Small inducible cytokine A2 precursor |
| B3AR_HUMAN | Beta-3 adrenergic receptor |
| TSHR_HUMAN | Thyrotropin receptor precursor |
| CB1R_HUMAN | Cannabinoid receptor 1 |
| DADR_HUMAN | D(1A) dopamine receptor |
| LSHR_HUMAN | Lutropin-choriogonadotropic hormone receptor precursor |
| HH2R_HUMAN | Histamine H2 receptor |
| NY1R_HUMAN | Neuropeptide Y receptors type 1 |
| 5H1D_HUMAN | 5-hydroxytryptamine 1D receptor |
| 5H1B_HUMAN | 5-hydroxytryptamine 1 B receptor |
| 5H1E_HUMAN | 5-hydroxytryptamine 1E receptor |
| SSR1_HUMAN | Somatostatin receptor type 1 |
| SSR2_HUMAN | Somatostatin receptor type 2 |
| 5H1F_HUMAN | 5-hydroxytryptamine 1F receptor |
| SSR4_HUMAN | Somatostatin receptor type 4 |
| VIPR_HUMAN | Vasoactive intestinal polypeptide receptor 1 precursor |
| CKR1_HUMAN | C-C chemokine receptor type 1 |
| SSR3_HUMAN | Somatostatin receptor type 3 |
| MC5R_HUMAN | Melanocortin-5 receptor |
| 5H7_HUMAN | 5-hydroxytryptamine 7 receptor |
| CB2R_HUMAN | Cannabinoid receptor 2 |
| CRF1_HUMAN | Corticotropin releasing factor receptor 1 precursor |
| SSR5_HUMAN | Somatostatin receptor type 5 |
| OPRM_HUMAN | Mu-type opioid receptor |
| OPRD_HUMAN | Delta-type opioid receptor |
| MC3R_HUMAN | Melanocortin-3 receptor |
| PI2R_HUMAN | Prostacyclin receptor |
| CXC1_HUMAN | Chemokine XC receptor 1 |
| ML1A_HUMAN | Melatonin receptor type 1A |
| ML1B_HUMAN | Melatonin receptor type 1B |
| 5H6_HUMAN | 5-hydroxytryptamine 6 receptor |
| ACTR_HUMAN | Adrenocorticotropic hormone receptor |
| MSHR_HUMAN | Melanocyte stimulating hormone receptor |
| PTRR_HUMAN | Parathyroid hormone/parathyroid hormone-related Peptide receptor precursor |
| 5H4_HUMAN | 5-hydroxytryptamine 4 receptor |
| CGRR_HUMAN | Calcitonin gene-related peptide type 1 receptor precursor |
| EDG7_HUMAN | Lysophosphatidic acid receptor Edg-7 |
| HH3R_HUMAN | Histamine H3 receptor |
| Htr7 RGD | 5-hydroxytryptamine (serotonin) receptor 7 |
| G-protein signaling, coupled to cAMP nucleotide second messenger | |
| PE23_MOUSE | Prostaglandin E2 receptor, EP3 subtype |
| CYA4_MOUSE | Adenylate cyclase, type IV |
| P2YC_MOUSE | P2Y purinoceptor 12 |
| GALS_HUMAN | Galanin receptor type 2 |
| GLP2_HUMAN | Glucagon-like peptide 2 receptor precursor |
| CAL0_HUMAN | Calcitonin precursor [Contains: Calcitonin; Katacalcin |
| SLIB_HUMAN | Somatoliberin precursor |
| CAL1_HUMAN | Calcitonin gene-related peptide I precursor |
| B2AR_HUMAN | Beta-2 adrenergic receptor |
| ACM2_HUMAN | Muscarinic acetylcholine receptor M2 |
| B3AR_HUMAN | Beta-3 adrenergic receptor |
| FMLR_HUMAN | fMet-Leu-Phe receptor |
| A1AD_HUMAN | Alpha-1D adrenergic receptor |
| AA2A_HUMAN | Adenosine A2a receptor |
| V2R_HUMAN | Vasopressin V2 receptor |
| PE23_MOUSE | Prostaglandin E2 receptor, EP3 subtype |
| MC4R_HUMAN | Melanocortin-4 receptor |
| GRK5_HUMAN | G protein-coupled receptor kinase GRK5 |
| CRF1_HUMAN | Corticotropin releasing factor receptor 1 precursor |
| A1AB_HUMAN | Alpha-1B adrenergic receptor |
| PE24_HUMAN | Prostaglandin E2 receptor, EP4 subtype |
| GLR_HUMAN | Glucagon receptor precursor |
| CKR3_HUMAN | C-C chemokine receptor type 3 |
| CRF2_HUMAN | Corticotropin releasing factor receptor 2 precursor |
| Q8BZV8 | P2Y12 platelet ADP receptor homolog |
| CYA4_MOUSE | Adenylate cyclase, type IV |
| Q99188 | ORF OR107W from chromosome XV |
| P2YC_MOUSE | P2Y purinoceptor 12 |
| WAS2_HUMAN | Wiskott-Aldrich syndrome protein family member 2 |
| Adcy2 MGI | adenylate cyclase 2 |
| Adcy4 MGI | adenylate cyclase 4 |
| P2ry12 MGI | With purinergic receptor P2Y, G-protein coupled 12 |
| Ptger3 MGI | prostaglandin E receptor 3 (subtype EP3) |
| Crhr1 RGD I | corticotropin releasing hormone 1 |
| CYR1 SGD | adenylate cyclase |
| RGS2 SGD | GTPase activating protein (GAP) |
| acy-1 | IMP - [cgc3038] |
| acy-2 | IMP - [cgc3207] |
| C44F1.5 | [cgc3038] |
| acy-4 | [cgc3207] |
| G-protein signaling, adenylate cyclase activating pathway | |
| GBQ_MOUSE | Guanine nucleotide-binding protein G(q), alpha subunit |
| Q9D1X2 | Thyroid stimulating hormone, receptor |
| TSHR_MOUSE | Thyrotropin receptor precursor |
| Q9WUC0 | Extra large alpha stimulating guanine-nucleotide Binding |
| | polypeptide |
| Q9Z0H2 | Neuroendocrine-specific golgi protein P55 isoform 2 |
| Q9Z0L1 | G protein-coupled receptor precursor |
| Q9Z1N8 | G-protein XLalphas |
| Q9Z1R7 | Guanine nucleotide-binding protein |
| AA2A_MOUSE | Adenosine A2a receptor |
| LGR8_MOUSE | Relaxin receptor 2 |
| UCN3_MOUSE | Urocortin III precursor |
| RAS1_YEAST | Ras-like protein 1 |
| GBQ_MOUSE | Guanine nucleotide-binding protein G(q), alpha subunit |
| CALR_HUMAN | Calcitonin receptor precursor |
| GPR3_HUMAN | Probable G protein-coupled receptor GPR3 |
| TSHR_MOUSE | Thyrotropin receptor precursor |
| Q14455 | Alpha subunit of GsGTP binding protein (Fragment) |
| AA2A_MOUSE | Adenosine A2a receptor |
| GBAF_MOUSE GB10_MOUSE | Guanine nucleotide-binding protein G(OLF), Alpha subunit (Fragment) Guanine nucleotide-binding protein, alpha-10 subunit Fragment) |
| Q80ZK6 | Similar to GNAS (Fragment) |
| Q8BIR3 | XLALPHAS protein homolog |
| Q8BM77 | Similar to G protein coupled receptor AFFECTING |
| | testicular DESCENT |
| Q8BUB2 | GNAS |
| Q8BXD1 | Similar to G protein coupled receptor AFFECTING |
| | testicular DESCENT |
| Q8C6E2 | Inferred: endothelial differentiation |
| Q8CAU3 | Adenosine A2a receptor (Fragment) |
| LGR8_MOUSE | Relaxin receptor 2 |
| UCN3_MOUSE | Urocortin III precursor |
| Q9D1X2 | Thyroid stimulating hormone, receptor |
| Q9D697 | Thyroid stimulating hormone, receptor |
| Q9JJX0 | Xlalphas protein (Fragment) |
| Q9QXW5 | Nesp |
| Q9QYZ0 | Extra large alpha stimulating guanine-nucleotide binding |
| | protein (Fragment) |
| Q9WUC0 | Extra large alpha stimulating guanine-nucleotide binding |
| | polypeptide |
| Q9Z0H2 | Neuroendocrine-specific golgi protein P55 isoform 2 |
| Q9Z0L1 | G protein-coupled receptor precursor |
| Q9Z1 N8 | G-protein XLalphas |
| Q9Z1R7 | Guanine nucleotide-binding protein |
| Adora2a | adenosine A2a receptor |
| Edg6 | endothelial differentiation, G-protein-coupled receptor 6 |
| Gnal | guanine nucleotide binding protein, alpha stimulating, |
| olfactory type | |
| Gnaq | guanine nucleotide binding protein, alpha q polypeptide |
| Gnas | GNAS (guanine nucleotide binding protein, alpha stimu- |
| lating) complex locus | |
| Gnas | GNAS (guanine nucleotide binding protein, alpha stimu- |
| lating) complex locus | |
| Gpr106 | G protein-coupled receptor 106 |
| Ptger4 | prostaglandin E receptor 4 (subtype EP4) |
| Tshr | thyroid stimulating hormone receptor |
| Ucn3 | urocortin 3 |
| RAS1 | ras homolog |
| adenylate cyclase activation | |
| piaA | cytosolic regulator of adenylyl cyclase pianissimo |
| G-salpha60A | G-salpha60A |
| Pacap38 F | Pacap38 |
| DADR_MOUSE | D(1A) dopamine receptor |
| 043190 | Not Available |
| CAL0_HUMAN | Calcitonin precursor [Contains: Calcitonin; Katacalcin |
| GBAS_HUMAN | Guanine nucleotide-binding protein G(S), alpha subunit |
| CAL1_HUMAN | Calcitonin gene-related peptide I precursor |
| B2AR_HUMAN | Beta-2 adrenergic receptor |
| B1AR_HUMAN | Beta-1 adrenergic receptor |
| PACA_HUMAN | Pituitary adenylate cyclase activating polypeptide precur- |
| sor | |
| ET1R_HUMAN | Endothelin-1 receptor precursor |
| AA2A_HUMAN | Adenosine A2a receptor |
| AA2B_HUMAN | Adenosine A2b receptor |
| V2R_HUMAN | Vasopressin V2 receptor |
| CALR_HUMAN | Calcitonin receptor precursor |
| AA3R_HUMAN | Adenosine A3 receptor |
| CRF1_HUMAN | Corticotropin releasing factor receptor 1 precursor |
| CAP2_HUMAN | Adenylyl cyclase-associated protein 2 |
| GLP1_HUMAN | Glucagon-like peptide 1 receptor precursor |
| GIPR_HUMAN | Gastric inhibitory polypeptide receptor precursor |
| CAP1_HUMAN | Adenylyl cyclase-associated protein 1 |
| GRFR_HUMAN | Growth hormone-releasing hormone receptor precursor |
| GBAF_MOUSE | Guanine nucleotide-binding protein G(OLF), alpha sub- |
| unit (Fragment) | |
| GB10_MOUSE | Guanine nucleotide-binding protein, alpha-10 subunit |
| (Fragment) | |
| DADR_MOUSE | D(1A) dopamine receptor |
| B2AR_ONCMY | Beta-2 adrenergic receptor |
| Adcy1 | adenylate cyclase 1 |
| Adcy2 | adenylate cyclase 2 |
| Adcy3 | adenylate cyclase 3 |
| Adcy4 | adenylate cyclase 4 |
| Adcy5 | adenylate cyclase 5 |
| Adcy6 | adenylate cyclase 6 |
| Adcy7 | adenylate cyclase 7 |
| Adcy9 | adenylate cyclase 9 |
| Adcyap1 | adenylate cyclase activating polypeptide 1 |
| Drd1a | dopamine receptor D1A |
| GnaI | guanine nucleotide binding protein, alpha stimulating, |
| olfactory type | |
| RAS2 | small GTP-binding protein |
| dopamine receptor, adenylate cyclase activating pathway | |
| Q8CH75 | Mu opioid receptor variant P |
| Q8VI69 | Mu opioid receptor variant BII |
| OPRM_MOUSE | Mu-type opioid receptor |
| Q9JIY1 | Mu opioid receptor variant F |
| Q9R0D1 | Mu opioid receptor variant C |
| Q9R1L9 | Mu opioid receptor MOR1 E |
| DADR_MOUSE | D(1A) dopamine receptor |
| DADR_HUMAN | D(1A) dopamine receptor |
| DBDR_HUMAN | D(1B) dopamine receptor |
| OPRM_MOUSE | Mu-type opioid receptor |
| DADR_MOUSE | D(1A) dopamine receptor |
| Q8CAN5 | Opioid receptor |
| Q8CGW2 | Mu opioid receptor variant MOR-1 R |
| Q8CH73 | Mu opioid receptor variant R |
| Q8CH74 | Mu opioid receptor variant Q |
| Q8CH75 | Mu opioid receptor variant P |
| Q8VI69 | Mu opioid receptor variant BII |
| Q8VI70 | Mu opioid receptor variant BI |
| Q8VI71 | Mu opioid receptor variant A |
| OPR2_MOUSE | Mu-type opioid receptor, isoforms 1G to 1M |
| Q9JIY1 | Mu opioid receptor variant F |
| Q9R0D1 | Mu opioid receptor variant C |
| Q9R0D2 | Mu opioid receptor variant 110222 (Fragment) |
| Q9R1L9 | Mu opioid receptor MOR1E |
| Q9R1M0 | Mu opioid receptor MOR1D |
| Drd1a | dopamine receptor D1A |
| NOT Oprd1 | opioid receptor, delta 1 |
| Oprm | opioid receptor, mu |
| Tar1 | trace amine receptor 1 |
| Serotonin receptor, adenylate cyclase activating pathway | |
| 5-HT7 | 5-HT7 |
| 5-HT7 | 5-HT7 |
| 5-HT7 | 5-HT7 |
| Htr7 | 5-hydroxytryptamine (serotonin) receptor 7 |
| G-protein signaling, adenylate cyclase inhibiting pathway | |
| GBI2_MOUSE | Guanine nucleotide-binding protein G(i), alpha-2 subunit |
| Q8CH75 | Mu opioid receptor variant P |
| Q8VI69 | opioid receptor variant BII |
| SSR2_MOUSE | Somatostatin receptor type 2 |
| OPRD_MOUSE | Delta-type opioid receptor |
| Q9DC35 | Endothelial differentiation sphingolipid G-protein-coupled |
| receptor 1 | |
| OPRM_MOUSE | Mu-type opioid receptor |
| Q9JIY1 | Mu opioid receptor variant F |
| Q9R0D1 | Mu opioid receptor variant C |
| Q9R1L9 | Mu opioid receptor MOR1E |
| Q9Z0U9 | LYSOPHOSPHOLIPID receptor B3 |
| EDG1_MOUSE | Probable G protein-coupled receptor Edg-1 |
| CORT_HUMAN | Cortistatin precursor [Contains: Cortistatin-29; Cortistatin- |
| 17] | |
| EDG1_MOUSE | Probable G protein-coupled receptor Edg-1 |
| GB12_MOUSE | Guanine nucleotide-binding protein G(i), alpha-2 subunit |
| NY1R_HUMAN | Neuropeptide Y receptor type 1 |
| SSR2_MOUSE | Somatostatin receptor type 2 |
| OPRD_MOUSE | Delta-type opioid receptor |
| OPRK_HUMAN | Kappa-type opioid receptor |
| OPRX_HUMAN | Nociceptin receptor |
| OPRM_MOUSE | Mu-type opioid receptor |
| NY2R_HUMAN | Neuropeptide Y receptor type 2 |
| RGS1_HUMAN | Regulator of G-protein signaling 1 |
| Q8BLP9 | Delta-type opioid receptor |
| Q8BP20 | Endothelial differentiation |
| Q8C4A3 | Endothelial differentiation sphingolipid G-protein-coupled |
| receptor 1 | |
| Q8CAN5 | Opioid receptor |
| Q8CGW2 | Mu opioid receptor variant MOR-1R |
| Q8CH73 | Mu opioid receptor variant R |
| Q8CH74 | Mu opioid receptor variant Q |
| Q8CH75 | Mu opioid receptor variant P |
| Q8JZT4 | Similar to guanine nucleotide binding protein, alpha inhib- |
| iting 2 | |
| Q8VI69 | Mu opioid receptor variant BII |
| Q8VI70 | Mu opioid receptor variant BI |
| Q8VI71 | Mu opioid receptor variant A |
| OPR2_MOUSE | Mu-type opioid receptor, isoforms 1G to 1M |
| Q922Y6 | Hypothetical protein (Fragment) |
| MCR1_HUMAN | Melanin-concentrating hormone receptor 1 |
| Q9DC35 | Endothelial differentiation sphingolipid G-protein-coupled |
| receptor 1 | |
| Q9JIY1 | Mu opioid receptor variant F |
| Q9R0D1 | Mu opioid receptor variant C |
| Q9R0D2 | Mu opioid receptor variant 110222 (Fragment) |
| Q9R1L9 | Mu opioid receptor MOR1 E |
| Q9R1M0 | Mu opioid receptor MOR1 D |
| Q9R235 | Lysophospholipid receptor B1 |
| Q9Z0U9 | LYSOPHOSPHOLIPID receptor B3 |
| Edg1 | endothelial differentiation sphingolipid G-protein-coupled |
| receptor 1 | |
| Edg3 | endothelial differentiation, sphingolipid G-protein-coupled |
| receptor, | |
| Gnai2 | guanine nucleotide binding protein, alpha inhibiting 2 |
| Npb | neuropeptide B |
| Oprd1 | opioid receptor, delta 1 |
| Oprm | opioid receptor, mu |
| Sstr2 | somatostatin receptor 2 |
| Oprm1 | "Opioid receptor, mu 1" |
| dopamine receptor, adenylate cyclase inhibiting pathway | |
| D2DR_HUMAN | D(2) dopamine receptor |
| muscarinic acetyl choline receptor, adenylate cyclase inhibiting pathway | |
| ACM2_HUMAN | Muscarinic acetylcholine receptor M2 |
| ACM5_HUMAN | Muscarinic acetylcholine receptor M5 |
| Negative regulation of adenylate cyclase activity | |
| MGR8_HUMAN | Metabotropic glutamate receptor 8 precursor |
| GALT_HUMAN | Galanin receptor type 3 |
| GBR2_HUMAN | Gamma-aminobutyric acid type B receptor, subunit 2 |
| precursor | |
| GBI2_HUMAN | Guanine nucleotide-binding protein G(i), alpha-2 subunit |
| GBAK_HUMAN | Guanine nucleotide-binding protein G(k), alpha subunit |
| A2AA_HUMAN | Alpha-2A adrenergic receptor |
| ETBR_HUMAN | Endothelin B receptor precursor |
| CKR2_HUMAN | C-C chemokine receptor type 2 |
| GALR_HUMAN | Galanin receptor type 1 |
| MGR2_HUMAN | Metabotropic glutamate receptor 2 precursor |
| MGR7_HUMAN | Metabotropic glutamate receptor 7 precursor |
| MGR3_HUMAN | Metabotropic glutamate receptor 3 precursor |
| MGR4_HUMAN | Metabotropic glutamate receptor 4 precursor |
| Q9NPE5 | Not Available |
| GBR1_HUMAN | Gamma-aminobutyric acid type B receptor, subunit 1 |
| precursor | |
| positive regulation of adenylate cyclase activity | |
| dagA | cytosolic regulator of adenylyl cyclase |
| serotonin receptor, adenylate cyclase inhibiting pathway | |
| 5-HT1A | 5-HT1A |
| 5-HT1B | 5-HT1 B |
| 5HTA_DROME | 5-hydroxytryptamine receptor 2A |
| 5HTB_DROME | 5-hydroxytryptamine receptor 2B |
| Q9V8Q3 | CG15113-PA |
| Q9V8Q9 | CG16720 protein |
| G-protein signaling, coupled to cGMP nucleotide second messenger | |
| TBL3_HUMAN | WD-repeat protein SAZD |
| 4933400B15Rik | RIKEN cDNA 4933400B15 gene |
| Gnat1 | guanine nucleotide binding protein, alpha transducing 1 |
| Gnat2 | guanine nucleotide binding protein, alpha transducing 2 |
| TbI3 | transducin (beta)-like 3 |
| Nos2 | nitric oxide synthase 2 |
| G-protein signaling, coupled to IP3 second messenger (phospholipase C activating) | |
| GB15_MOUSE | Guanine nucleotide-binding protein, alpha-15 subunit |
| Q9ERT2 | Thyrotropin-releasing hormone receptor 2 |
| GBGD_MOUSE | Guanine nucleotide-binding protein G(I)/G(S)/G(O) |
| | gamma-13 subunit |
| 043190 | Not Available |
| 076067 | Not Available |
| ETBR_HUMAN | Endothelin B receptor precursor |
| IL8B_HUMAN | High affinity interleukin-8 receptor B |
| NK1 R_HUMAN | Substance-P receptor |
| NMBR_HUMAN | Neuromedin-B receptor |
| AG2R_HUMAN | Type-1 angiotensin II receptor |
| PE23_MOUSE | Prostaglandin E2 receptor, EP3 subtype |
| OXYR_HUMAN | Oxytocin receptor |
| GB15_MOUSE | Guanine nucleotide-binding protein, alpha-15 subunit |
| CCKR_HUMAN | Cholecystokinin type A receptor |
| GASR_HUMAN | Gastrin/cholecystokinin type B receptor |
| HH1R_HUMAN | Histamine H1 receptor |
| P2Y2_HUMAN | P2Y purinoceptor 2 |
| 5H2B_HUMAN | 5-hydroxytryptamine 2B receptor |
| MC3R_HUMAN | Melanocortin-3 receptor |
| P2YR_HUMAN | P2Y purinoceptor 1 |
| P2Y4_HUMAN | P2Y purinoceptor 4 |
| P2Y6_HUMAN | P2Y purinoceptor 6 |
| L4R1_HUMAN | Leukotriene B4 receptor 1 |
| Q61621 | G-protein beta subunit (Fragment) |
| Q9ERT1 | Thyrotropin-releasing hormone receptor 2 (Fragment) |
| Q9ERT2 | Thyrotropin-releasing hormone receptor 2 |
| GBGD_MOUSE | Guanine nucleotide-binding protein G(I)/G(S)/G(O) |
| | gamma-13 subunit |
| Q9NYK7 | CCK-B/gastrin receptor |
| Gna15 | guanine nucleotide binding protein, alpha 15 |
| Gnb1 | guanine nucleotide binding protein, beta 1 |
| Gng13 | guanine nucleotide binding protein 13, gamma |
| Ptger3 | prostaglandin E receptor 3 (subtype EP3) |
| Trhr2 | thyrotropin releasing hormone receptor 2 |
| Agtr1a | angiotensin receptor 1a |
| cytosolic calcium ion concentration elevation | |
| norpA | norpA |
| Q99L49 | Similar to transient receptor protein 2 |
| PE23_MOUSE | Prostaglandin E2 receptor, EP3 subtype |
| TRP6_MOUSE | Short transient receptor potential channel 6 |
| JPH2_MOUSE | Junctophilin 2 |
| SY28_MOUSE | Small inducible cytokine A28 precursor |
| CKRA_MOUSE | C-C chemokine receptor type 10 |
| TRP2_MOUSE | Short transient receptor potential channel 2 |
| 043431 | Not Available |
| GALS_HUMAN | Galanin receptor type 2 |
| SZ13_HUMAN | Small inducible cytokine B13 precursor |
| 095977 | Putative G-protein coupled receptor, EDG6 precursor |
| CAL0_HUMAN | Calcitonin precursor [Contains: Calcitonin; Katacalcin |
| CAL1_HUMAN | Calcitonin gene-related peptide I precursor |
| DADR_HUMAN | D(1A) dopamine receptor |
| C5AR_HUMAN | C5a anaphylatoxin chemotactic receptor |
| ET1R_HUMAN | Endothelin-1 receptor precursor |
| BRB2_HUMAN | B2 bradykinin receptor |
| AG2R_HUMAN | Type-1 angiotensin II receptor |
| PE23_MOUSE | Prostaglandin E2 receptor, EP3 subtype |
| CCR4_HUMAN | C-X-C chemokine receptor type 4 |
| CCKR_HUMAN | Cholecystokinin type A receptor |
| GASR_HUMAN | Gastrin/cholecystokinin type B receptor |
| CKR1_HUMAN | C-C chemokine receptor type 1 |
| CKR7_HUMAN | C-C chemokine receptor type 7 precursor |
| V1AR_HUMAN | Vasopressin V1a receptor |
| CKR2_HUMAN | C-C chemokine receptor type 2 |
| CXC1_HUMAN | Chemokine XC receptor 1 |
| BRB1_HUMAN | B1 bradykinin receptor |
| V1BR_HUMAN | Vasopressin V1b receptor |
| CCR3_HUMAN | C-X-C chemokine receptor type 3 |
| P2Y4_HUMAN | P2Y purinoceptor 4 |
| CKR3_HUMAN | C-C chemokine receptor type 3 |
| CKR4_HUMAN | C-C chemokine receptor type 4 |
| CKR5_HUMAN | C-C chemokine receptor type 5 (CCR5) |
| CKR6_HUMAN | C-C chemokine receptor type 6 |
| CKR8_HUMAN | C-C chemokine receptor type 8 |
| CKR9_HUMAN | C-C chemokine receptor type 9 |
| PAR2_HUMAN | Proteinase activated receptor 2 precursor |
| C3AR_HUMAN | C3a anaphylatoxin chemotactic receptor |
| Q61057 | Trp-related protein 2 (Fragment) |
| TRP6_MOUSE | Short transient receptor potential channel 6 |
| Q8BRU2 | Transient receptor protein 2 |
| Q8CDC6 | Transient receptor protein 2 |
| Q8CEM7 | Transient receptor protein 2 (Fragment) |
| EDG2_HUMAN | Lysophosphatidic acid receptor Edg-2 |
| EDG3_HUMAN | Sphingosine 1-phosphate receptor Edg-3 |
| MCR1_HUMAN | Melanin-concentrating hormone receptor 1 |
| Q99L49 | Similar to transient receptor protein 2 |
| JPH2_MOUSE | Junctophilin 2 |
| SY28_MOUSE | Small inducible cytokine A28 precursor |
| CKRA_MOUSE | C-C chemokine receptor type 10 |
| Q9NYK7 | CCK-B/gastrin receptor |
| TRP2_MOUSE | Short transient receptor potential channel 2 |
| EDG7_HUMAN | Lysophosphatidic acid receptor Edg-7 |
| CLT1_HUMAN | Cysteinyl leukotriene receptor 1 |
| Ccl28 | chemokine (C-C motif) ligand 28 |
| Edg3 | endothelial differentiation, sphingolipid G-protein-coupled |
| | receptor, 3 |
| Gpr2 | G protein-coupled receptor 2 |
| Jph2 | junctophilin 2 |
| Ptger3 | prostaglandin E receptor 3 (subtype EP3) |
| Trpc2 | transient receptor potential cation channel, subfamily C, |
| | member 2 |
| Trpc6 | transient receptor potential cation channel, subfamily C, |
| | member 6 |
| ltpr3 | inositol 1, 4, 5-triphosphate receptor 3" |
| Trrp6 | " transient receptor potential cation channel, subfamily C, |
| | member 6 " |
| itr-1 | Not Available |
| Metabotropic glutamate receptor, phospholipase C activating pathway | |
| 096003 | SYN47 protein |
| MGR5_HUMAN | Metabotropic glutamate receptor 5 precursor |
| Grm5 | "glutamate receptor, metabotropic 5" |
| Muscarinic acetyl choline receptor, phospholipase C activating pathway | |
| ACM2_HUMAN | Muscarinic acetylcholine receptor M2 |
| ACM1_HUMAN | Muscarinic acetylcholine receptor M1 (herein also |
| | designated Muscarinic M1) |
| GB15_HUMAN | Guanine nucleotide-binding protein, alpha-15 subunit |
| Chrm3 | cholinergic receptor, muscarinic 3, cardiac |
| phospholipase C activation | |
| Galpha49B | Galpha49B |
| Gbeta76C | Gbeta76C |
| 043431 | Not Available |
| 095977 | Putative G-protein coupled receptor, EDG6 precursor |
| CAL0_HUMAN | Calcitonin precursor [Contains: Calcitonin; Katacalcin |
| CAL1_HUMAN | Calcitonin gene-related peptide I precursor |
| C5AR_HUMAN | C5a anaphylatoxin chemotactic receptor |
| GBQ_DROME | Guanine nucleotide-binding protein G(q), alpha subunit |
| ET1 R_HUMAN | Endothelin-1 receptor precursor |
| GBB2_DROME | Guanine nucleotide-binding protein beta subunit 2 |
| GB15_HUMAN | Guanine nucleotide-binding protein, alpha-15 subunit |
| CALR_HUMAN | Calcitonin receptor precursor |
| GASR_HUMAN | Gastrin/cholecystokinin type B receptor |
| V1AR_HUMAN | Vasopressin V1a receptor |
| V1BR_HUMAN | Vasopressin V1b receptor |
| GBQ_HUMAN | Guanine nucleotide-binding protein G(q), alpha subunit |
| PIB2_HUMAN | 1-phosphatidylinositol-4,5-bisphosphate phospho |
| | diesterase beta 2 |
| EDG2_HUMAN | Lysophosphatidic acid receptor Edg-2 |
| Q9I7C8 | G protein alpha 49B |
| Q9NYK7 | CCK-B/gastrin receptor |
| Q9TXA4 | Signal-transducing G protein alpha Q subunit |
| Q9VW29 | GBETA76C protein |
| Adra1a | "adrenergic receptor, alpha 1a" |
| Adcyap1r1 | adenylate cyclase activating polypeptide 1 receptor 1 |
| protein kinase C activation | |
| PF14_0681 Pfalciparum | diacylglycerol kinase, putative |
| GBLP_HUMAN | Guanine nucleotide-binding protein beta subunit-like |
| | protein 12.3 |
| PIC1_MOUSE | PRKCA-binding protein |
| GBLP_HUMAN | Guanine nucleotide-binding protein beta subunit-like |
| | protein 12.3 |
| PIC1_RAT | PRKCA-binding protein |
| KPCN_HUMAN | Protein kinase C, nu type |
| ACM1_HUMAN | Muscarinic acetylcholine receptor M1 |
| NEUM_HUMAN | Neuromodulin |
| CAP7_HUMAN | Azurocidin precursor |
| ET2_HUMAN | Endothelin-2 precursor |
| GBLP_HUMAN . | Guanine nucleotide-binding protein beta subunit-like |
| | protein 12.3 |
| 143F_HUMAN | 14-3-3 protein eta |
| PIC1_MOUSE | PRKCA-binding protein |
| Q80VC8 | Similar to protein that interacts with C kinase 1 |
| Q8C1W2 | Protein that interacts with C kinase 1 |
| PIC1_RAT | PRKCA-binding protein |
| PIC1_HUMAN | PRKCA-binding protein |
| C130010K08Rik | RIKEN cDNA C130010K08 gene |
| Cerk | ceramide kinase |
| Gnb2-rs1 | guanine nucleotide binding protein, beta 2, related |
| | sequence 1 |
| F13G24.120 | diacylglycerol kinase 1 (DGK1) |
| F17I23.320 | diacylglycerol kinase family |
| F18E5.160 | diacylglycerol kinase family |
| F26K10.10 | diacylglycerol (DAG) kinase accessory domain protein |
| F5H14.13 | diacylglycerol kinase, putative |
| K19M13.8 | diacylglycerol kinase family |
| MBK5.25 | diacylglycerol kinase, putative |
| MRI1.5 | diacylglycerol kinase, putative |
| MSF3.11 | diacylglycerol kinase, putative |
| F13G24.120 | diacylglycerol kinase 1 (DGK1) |
| F26K10.10 | diacylglycerol (DAG) kinase accessory domain protein |
| T3F17.26 | diacylglycerol kinase family |
| serotonin receptor, phospholipase C activating pathway | |
| 5-HT1A | 5-HT1A |
| 5-HT1A | 5-HT1A |
| 5-HT1 B | 5-HT1 B |
| 5-HT1 B | 5-HT1 B |
| 5HTA_DROME | 5-hydroxytryptamine receptor 2A |
| 5HTB_DROME | 5-hydroxytryptamine receptor 2B |
| Q9V8Q3 | CG15113-PA |
| Q9V8Q9 | CG16720 protein |
| Htr2b | 5-hydroxytryptamine (serotonin) receptor 2B |

Gene symbol refers to the symbol used in the Gene Ontology Blast server available 25 May 2005 at http:// godatabase.org/cgi- bin/go.cgi?view=blast&session_id=87201075891145.

In an even further embodiment of the invention the GPCR is coupled to a G-protein, such as G_{Q}, that activates phospholipase C. Examples of such GPCRs are given in table 3.

Other receptors than GPCR may be used with the present invention, for example the cell surface molecule may be a receptor selected from the group consisting of receptors belonging to the family of protein kinase coupled receptors and receptors belonging to the family of receptor kinases.

The family of Protein kinase coupled receptors for example includes receptors for cytokines, interferons and HGF. These receptors do not have intrinsic kinase actvity, but are associated with a kinase.

Activation of preferred protein kinase coupled receptors results in activation of AP-1, i.e. in increased transcription from genes comprising one or more AP-1 sites in their regulatory sequences. This is in particular true for receptors activated by a cytokine.

Receptor kinases are receptors having an intrinsic kinase activity. Frequently said activity may be modulated by association of a ligand. The family for example includes receptors for Insulin, NGF, PDGF, FGF, EGF and GH.

Activation of preferred receptor kinases results in activation of SRE, i.e. in an increase in transcription from genes comprising one or more SRE in their regulatory sequences. This is in particular true for receptor kinases activated by growth hormones.

The receptor may also be an orphan receptor, i.e. a receptor for which no ligand has yet been identified. The methods of the present invention may also be useful for identifying ligands of orphan receptors.

The cell surface molecule may in one embodiment of the invention be a channel which is accessible from the extracellular surface, such as a transmembrane channel. Examples of channels are ion-channels, such as Ca²⁺ channels.

### Cellular response

The invention relates to methods of identifying compounds modulating, such as activating or inhibiting, a cellular response linked to a reporter system. The reporter system may be any of the reporter systems described herein below. The methods disclosed by the present invention may be used to identify compounds modifying any cellular response, which is or may be linked to a reporter system generating a detectable output. The person skilled in the art will appreciate that the specific methods disclosed herein may be adapted to any such cellular response. Below, non-limiting examples of cellular responses are described.

In a particularly preferred embodiment of the invention, the cellular response is mediated through a cell surface molecule, for example the cellular response may be activation of a receptor. Hence, the cellular response may for example be modulation of a signal transduction pathway within a cell, such as modulation of a signal transduction pathway mediated by a cell surface molecule. By "activation of a receptor" is meant that the receptor is influenced in a manner that it activates downstream signalling events. Accordingly, the methods according to the present invention may be employed to identify agonists or antagonist of a receptor.

Examples includes:
- Upregulation or downregulation of the level of a member of the pathway
- Relocalisation of a member of the pathway
- Complex formation between members of the pathway or between members of the pathway with other cellular compounds
- Enhanced or reduced transcription from genes regulated by the pathway
- Modification by for example phosphorylation of a member of the pathway
- Activation or inhibition of an enzyme of the pathway
- Degradation of a cellular compounds due to upregulation or downregulation of the pathway
- Altered secretion of a compound
- Change in ion-flux
- Morphological changes
- Change in viability

In a preferred embodiment the signal transduction pathway is a pathway modulated by any of the receptors described in the section herein above. Hence, the cellular response may for example be any of the following:
- Activation of adenylate cyclase; i.e. increase in adenylate cyclase activity
- Increased levels of cAMP
- Increased transcription of genes regulated by a CRE
- Inhibition of adenylate cyclase; i.e. decrease in adenylate cyclase activity
- Decreased levels of cAMP
- Decreased transcription of genes regulated by a CRE
- Increased activity of phospholipase C
- Increased level of inositol 1,4,5-trisphosphate
- Increased activity of Protein kinase C (PKC)
- Phosphorylation of proteins, which are phosphorylated by protein kinase C

The cellular response may in one embodiment be modulation of transcriptional activity, such as activation or reduction of transcription of one or more genes. In particular, activation or reduction of transcription of genes regulated by a response element. Said response element could for example be selected from the group consisting of CRE, SRE, TRE and AP-1.

Hence, the cellular response may also be an increased or decreased level of a particular mRNA within a cell.

By the term "regulated by a response element" is meant that transcription is modulated by said response element, however other elements may also modulate transcription of said gene. By the term "activation of response element" is meant increased transcription of genes regulated by said response element and/or operably associated therewith.

In another embodiment of the invention the cellular response is:
- change in the intracellular level of a compound; or
- change in the level of a compound within a specific cellular compartment, for example within the cytoplasm, in the golgi, in the endoplasmatic reticulum, in lysosomes, in endosomes or in the nucleus

The compound may be any compound, preferably a naturally occurring compound. Frequently, the compound is a compound endogenous to the cell. The compound may thus for example be a salt, an ion, a nucleotide or a derivative thereof, a peptide, a saccharide, a lipid or a biomacromolecule. Biomacromolecules includes for example RNA such as mRNA, polypeptides and proteins. An example of an ion is Ca²⁺ and an example of a nucleotide derivative is cAMP.

In yet another embodiment of the invention the cellular response is relocalisation of a compound. Relocalisation may for example be
- concentration of a compound otherwise dispersed in one or more specific locations
- relocalisation from one cellular compartment to another, for example relocalisation from the cellular membrane to the cytoplasma.
- relocalisation from one location within a compartment to another location within the same compartment
- internalisation of an extracellular compound

The compound may be any compound, such as any of the compounds mentioned in the section above. In one preferred embodiment the compound, which is relocalised is a biomacromolecule, such as RNA, polypeptides or proteins. For example, the compound may be a cell surface receptor (receptor). The cellular response may thus be internalisation of said receptor or relocalisation of said receptor from the cellular membrane to the cytoplasma.

In one embodiment of the invention the cellular response is change in the activity of a compound, such as an increase or a decrease in the activity of a compound. Said compound may for example be an enzyme.

In another embodiment of the invention the cellular response is change in phosphorylation of a compound.

In another embodiment of the invention the cellular response is formation or disruption of a complex between compounds.

In another embodiment of the invention the cellular response is change in the concentration of a compound.

The cellular response may also be altered secretion of a compound, such as increased or decreased secretion of a compound. Said compound could for example be a biomacromolecule, such as a protein, a polypeptide, a peptide, a hormone, a cytokine, or the like.

In another embodiment of the invention the cellular response is change in pH in an intracellular compartment, for example in the cytoplasm.

In yet another embodiment the the cellular response is a change in a membrane potential, for example a change in membrane potential over the cell membrane or over the mitochondria membrane.

In an even further embodiment of the invention the cellular response is change in morphology, such as change in size or shape. The cellular response may also be change in viability (e.g. apoptosis or necrosis) under specific conditions.

The methods according to the invention may also include identification of compounds modulating more than one cellular response, such as 2, for example 3, such as 4, for example 5, such as more than 5 different cellular responses. Said cellular responses may be any of the responses discussed above.

### Reporter system

The reporter system to be used with the present invention should be selected according to the particular cellular response. The reporter system should be capable of generating a detectable output.

In some embodiments of the invention the reporter system may be identical to the cellular response. This is in particular true when the cellular response may be detected without the aid of an additional reporter system, for example when the cellular response is an increase/decrease in the level of a compound, relocalisation of a compound, change in membrane potential, change in pH, change in morphology or the like.

Hence, the reporter system may be a system endogenous to said cells. For example, the reporter system may comprise the endogenous system regulating the intracellular level of an endogenous compound. By way of example, the reporter system may be the endogenous system of a cell regulating the intracellular Ca²⁺ level.

In another example, the reporter system comprises the intracellular localisation of an endogenous compound.

However, the reporter system may also be heterologous to the cell, i.e. a reporter system which has been inserted into the cell for example by recombinant techniques.

In embodiments of the invention, wherein the cellular response is modulation of transcription from gene(s) regulated by a response element, it is preferred that the report system comprises a nucleic acid comprising a nucleotide sequence encoding a detectable polypeptide operably linked to a response element, the activity of which is modulated by the cellular response.

In embodiments of the invention, wherein the cellular response is modulation of a signal tranduction pathway, the reporter system may comprise a nucleic acid comprising a nucleotide sequence encoding a detectable polypeptide operably linked to a response element, the activity of which is modulated by said signal transduction pathway.

For example, if the cellular response is modulation of a signal transduction pathway influencing the activity of CRE and/or SRE, then the reporter system may comprise a nucleic acid comprising a nucleotide sequence encoding a detectable polypeptide operably linked to a response element selected from the group consisting of cAMP response element (CRE) and serum response element (SRE). Examples of such signal transduction pathways include the signal transduction pathways modulated by GPCR of the rhodopsin family or secretin family and by protein kinase receptors and receptors belonging to the family of receptor kinases.
By way of example: 1) If the cellular response is activation of a signal transduction pathway activated by a GPCR coupled to a G_{S} (see herein above) that stimulates adenylate cyclase, then the reporter system may be a nucleic acid comprising a nucleotide sequence encoding a detectable polypeptide operably linked to CRE. Activation of said GPCR may then be detected by detection of increased levels of said detectable polypeptide. 2) If the cellular response is activation of signal transduction pathway activated by a GPCR coupled to a G, (see herein above) that inhibits adenylate cyclase, then the reporter system may be a nucleic acid comprising a nucleotide sequence encoding a detectable polypeptide operably linked to CRE. Activation of said GPCR may then be detected by detection of decreased levels of said detectable polypeptide.

Similarly, if the cellular response is modulation of a signal transduction pathway that influences the activity of TRE, then the reporter system may comprise a nucleic acid comprising a nucleotide sequence encoding a detectable polypeptide operably linked to TPA response element (TRE). Examples are GPCRs that are linked to activation of Protein Kinase C such as Gq coupled receptors (see herein above).

Similarly, if the cellular response is modulation of a signal transduction pathway that influences the activity of SRE, then the reporter system may comprise a nucleic acid comprising a nucleotide sequence encoding a detectable polypeptide operably linked to SRE. Examples of such signal transduction pathways include the signal tranduction pathways modulated by growth hormones or cytokines through protein kinase receptors and receptors belonging to the family of receptor kinases.

Similarly, if the cellular response is modulation of a signal transduction pathway that influences the activity of AP-1, then the reporter system may comprise a nucleic acid comprising a nucleotide sequence encoding a detectable polypeptide operably linked to AP-1. Examples of such signal transduction pathways include the signal tranduction pathways modulated by cytokines or growth factors cytokines through protein kinase receptors and receptors belonging to the family of receptor kinases

The detectable polypeptide may be any detectable polypeptide, however preferably the detectable polypeptide is selected from the group consisting of fluorescent proteins and enzymes.

Fluorescent proteins may for example be green fluorescent protein (GFP) and fluorescent mutants thereof, such as yellow fluorescent protein (YFP) or cyan fluorescent protein (CFP). The fluorescent protein can also be a protein complex, e.g. a di- or tetramer of a fluorescent protein, such as dsRed. Enzymes may for example be selected from the group consisting of luciferase, CAT, galactosidase, alkaline phosphatase and beta-lactamase.

In one embodiment of the invention the reporter system may comprise a bioluminescent moiety. For example, if the cellular response is relocalisation of a compound, then the reporter system may for example be said compound linked to a luminiscent moiety, such as a fluorescent moeity. Hence, for example if the cellular response is relocalisation of a polypeptide the reporter system may be a chimeric protein made up of said polypeptide and a fluorescent protein, such as GFP, YFP or CFP. In one preferred embodiment said polypeptide may be receptor.

In one embodiment of the invention the reporter system may detect complex formation between two cellular proteins. This may for example be achieved by linking a bioluminescent moiety, such as luciferase, to the one protein and a fluorescent moiety, such as a fluorescent protein, to the other protein. Direct interaction between the proteins can after expression of the two chimeric proteins be detected through occurrence of BRET (Bioluminescence Resonance Energy Transfer). If the two proteins are linked to fluorescent moieties it is possible to detect the complex formation through the occurrence of FRET (Fluorescence Resonance Energy Transfer). Complex formation may also be detecting using scintillation proximity assays.

Hence, if the cellular response is relocalisation of a cell surface molecule, then the reporter system may comprise a fluorescent moiety covalently coupled to said cell surface molecule.

In some embodiments of the invention the cellular response is modulation of a signal transduction pathway involving activation of phospholipase C. Phospholipase C may for example be activated by GPCRs coupled to G_{Q} (see herein above). Activation of phospholipase C in general leads to increase in the intracellular level of Ca²⁺ and thus in such embodiments the reporter system may be the intracellular Ca²⁺ level. This reporter system may thus be endogenous to the cell.

### Detectable output

The detectable output may be any output, which is detectable directly or indirectly. For example the detectable output may be the concentration of a compound within a cell, localisation of a compound within a cell, luminiscense, activity of an enzyme or the like:

In preferred embodiments of the invention the detectable output is luminiscense, such as fluorescence, bioluminescence, FRET or BRET. Bioluminiscence may be detected by any conventional methods, for example with the aid of a Plate reader. BRET may be performed as described herein above. In one embodiment, BRET2 technology is used which is based on energy transfer between a bioluminescent donor (a Renilla luciferase (Rluc) fusion protein) and a fluorescent acceptor (a Green Fluorescent Protein (GFP2) fusion protein). In presence of its substrate DeepBlueC™ (a coelenterazine derivative), Rluc emits blue light (-395 nm). A protein-protein interaction between Rluc and GFP2 fusion proteins allows energy transfer to GFP2 which reemits green light (510 nm). Expression of Rluc alone, in the presence of the substrate DeepBlueC™, gives an emission spectrum with a peak at ~395 nm (solid line). With the Rluc:GFP2 fusion construct, there is efficient energy transfer between Rluc and GFP2 and the 510 nm signal represents a major peak (dashed line). The BRET2 signal is expressed as the 515 nm to 410 nm ratio, since filters centered at those wavelengths are used for detection. FRET technology is based on the distance-dependent energy transfer between two fluorescence groups that are each coupled to a protein.

Alternatively, the detectable output may preferably be linked (directly or indirectly) to a bioluminiscent signal.

However, the detectable output could also be radioactivity, a coloured compound or a colour signal, a heavy metal, an electrical potential, a redox potential, a temperature or the detectable output may be linked to a radioactive signal, a coloured compound or a colour signal or a heavy metal or an electrical potential, or a redox potential or a temperature. Said radioactive signal could for example be ³⁵S, ³²P, ³H. The coloured compound could for example be the product of any of the enzymatic reaction described herein elsewhere. The heavy metal could for example be gold.

In embodiments of the invention, wherein the cellular response is change in the intracellular level of a compound or change in the level of a compound within a specific cellular compartment, then the detectable output may be said level of said compound. Depending on the nature of the compound, said level may be detected directly or indirectly.

If the compound for example is a fluorescent compound, the level of said compound may be determined by determining the fluorescence properties. This may be done by any suitable means, for example by the aid of a fluorescence microscope, a FACS (Fluorescence Activated Cell Sorter), a FABS (Fluorescence Activated Bead Sorter), fluorescence plate-reader or a fluorescence spectrometer,

If the compound for example is an enzyme then the level of said compound may be determined by determining the activity of said enzyme. By way of example, if the enzyme catalyses a reaction leading to a product, which is directly detectable, for example by colorimetric or chemiluminescent detection techniques, the activity of said enzyme may be detected by detecting said compound. For example, if the enzyme is luciferase, the activity of said enzyme may be detected by detecting emmision of light upon oxidation of the added substrate, luciferin.

Several other enzymes such as CAT, β-galactosidase, alkaline phosphatase, horseradish peroxidase and beta-lactamase are, when provided with suitable substrates, capable of catalysing reactions leading to coloured or chemiluminescent products, which may be detected using any colorimetric or chemiluminescent detection technique.

If the compound for example is Ca²⁺, then the intracellular concentration of said ion can be measured by using any suitable method, for example by inserting into the cells Ca²⁺ binding fluorescent compounds like Fura-2, Fluo-3 or Fluo-4 (Molecular Probes), which change fluorescent properties according to a changed Ca²⁺ concentration. Non-limiting examples of methods of determining cytosolic free Ca²⁺ are given in examples 13 and 13a herein below. Other ion concentrations can be monitored using suitable fluorescent compounds, which for example are available from Molecular Probes Inc.

If the compound for example is a protein, then it may for example be detected using a first specific binding partner. Said first specific binding partner could be a second protein capable of specifically interacting with said protein, such as a specific antibody or said first specific binding partner could be an aptamer. Said first specific binding partner could be conjugated to a directly detectable compound, such as a fluorescent compound, a radioactive compound or a heavy metal or to an indirectly detectable compound, such as an enzyme, which for example could be any of the enzymes mentioned herein above. It is also possible that the first specific binding partner may be detected with a second specific binding partner, capable of interacting specifically with the first specific binding partner. Said second specific binding partner may be conjugated to a directly or indirectly detectable compound similarly to the first specific binding partner. Additional specific binding partners may be used.

In embodiments of the invention wherein the cellular response is relocalisation of a compound the detectable output could be a detectable label conjugated to said compound. In particular, the compound may be conjugated to a directly detectable label, such as a fluorescent label or a heavy metal. Thus the localisation of the compound may be directly detected, for example using a fluorescence microscope, Fluorescent plate-reader, fluorescence spectrometer, a FACS or a FABS instrument In one preferred embodiment the compound is a fusion protein comprising a protein of interest and a fluorescent protein, such as GFP. The compound may thus be a fluorescent probe. Thus the detectable output may be localisation of a fluorescent signal. Alternatively, the compound is a fusion protein comprising the protein of interest and a tag. Said tag could be a tag specifically interacting with a specific binding partner, for example the tag could be an HA-tag or a flash domain. Alternatively, localisation of a compound may be determined with the aid of a specific binding partner as outlined above. Intracellular localisation may also be detected using methods capable of detecting distance between two compounds, for example BRET or FRET.

In embodiments of the invention wherein the cellular response is change of activity of a compound, the detectable output may be a product of said activity. i.e. when said compound is an enzyme the detectable output could be a product of a reaction catalysed by said enzyme. Said product could thus be a coloured product or a chemiluminiscent product as discussed herein above.

In embodiments of the invention wherein the cellular response is enhanced or reduced transcription from one or more genes, then the cellular response could be mRNA transcribed from said gene, a protein encoded by said gene or in case the protein is an enzyme, the detectable output could be a product of a reaction catalysed by said enzyme. The enzyme and the products could be any of the enzymes or products discussed herein above.

mRNA may be detected by any useful means, for example with the aid of a probe capable of hybridising specifically with said mRNA. Said probe could be labelled with a directly detectable label, for example a radioactive compound, a fluorescent compound or a heavy metal or an indirectly detectable label such as an enzyme or a specific binding partner.

Said protein may be detected with the aid of specific binding partners as outlined herein above. However, in a preferred embodiment the protein is a fluorescent protein and may thus be detected directly. Hence, the detectable output could be bioluminescence, such as fluorescence.

In embodiments of the invention wherein the cellular response is modification by for example phosphorylation of a compound this can be detected through binding of a antibody that specifically bind the phosphorylated protein said antibody can then be quantified by specific fluorescence labelling.
In embodiments of the invention wherein the cellular response is change in pH in an intracellular compartment, the detectable output will in general be said pH. The pH may be determined using any suitable method, for example using a pH indicator or a pH-meter. For example the pH may be determined using a fluorescent indicator for intracellular pH. Suitable compounds are compounds with a fluorescent excitation profile which is pH-dependent, such as BCECF (available from Molecular Probes). In embodiments of the invention wherein the cellular response is a change in a membrane potential, the detectable output will in general be said membrane potential. The membrane potential may be determined using any suitable method such as applying a fluorescent molecule to cells that distribute over the membrane dependent upon the membrane potential. Examples of such compounds are DiBAC. various ANEP dyes, JC-1 and JC-9 (Molecular Probes). For example, JC-1 and JC-9 are cationic dyes that exhibit potential-dependent accumulation in mitochondria leading to a shift in fluorescence emmision from green to red. Thus mitochondrial depolarization may for example be determined by decrease in red/green fluorescence intensity ratio (see also product information from Molecular Probes). ANEP dyes are in particularly useful for detection of changes in membrane potential. The fluorescence can be readfor instance by a fluorescence microscope, a fluorescence plate-reader, a FACS, or a FABS instrument.

In embodiment of the invention wherein the cellular response is change in morphology, the detectable output will in general be the morphology of the cell. The morphology may be observed using any suitable method for example by the aid of a microscope, using a FACS or FABS,

Depending on the detectable output, it will frequently be an advantage to fix cells prior to detecting said detectable output. However, in some embodiments of the invention it is preferred that the cells are not fixed. Cells may be fixed according to any useful protocol (see also definitions herein above).

### Selection

The methods according to the invention involves screening resin beads for beads comprising cells meeting at least one predetermined selection criterion, wherein said selection criterion is linked directly or indirectly to said detectable output. Hence, the selection criterion will be dependent on the detectable output.

For example the predetermined selection criteria may be a quantitative criterium, such as a quantitative level of bioluminiscence above or below a specific threshold value.

In embodiments of the invention, wherein the detectable output is fluorescence or the detectable output may be linked to a fluorescent signal, then the predetermined selection criterion could be any fluorescence property. For example, the selection criterion could be intensity of said fluorescence above or below a predetermined threshold value or emission of light of a specific wavelength or absorption of light of a specific wavelength or intensity of emitted light of a specific wavelength above or below a predetermined threshold value. The selection criterion could also be based on Fluorescence lifetime and/or fluorescence polarization The selection criterion could also be a specific localisation of the fluorescent signal, such as intensity of a fluorescent signal in a specific cellular compartment above or below a predetermined threshold value. The selection criterion could also be a predetermined change in fluorescence lifetime or in fluorescence polarization. Fluorescence intensity and/or localisation may for example be determined using image processing and/or image analysis, a fluorescence microscope, FACS, FABS or fluorescence plate reader.

In one embodiment of the invention the selection criterion is high fluorescence intensity. This may for example be the case, when the cellular response is activation of a signal transduction pathway and the reporter system comprises a gene encoding a fluorescent protein, where activation of the signal transduction pathway leads to incresed expression of said gene. Then resin beads may be selected using a method comprising the steps of:
1. Determining the fluorescence intensity of positive control resin beads and setting this fluorescence intensity to 100%
2. Determining the fluorescence intensity of negative control resin beads and setting this fluorescence intensity to 0%
3. Selecting resin beads having a fluorescence intensity corresponding to at least 5%, preferably at least 10%, more preferably at least 20%, even more preferably at least 30%, such as at least 40%, for example at least 50%, such as at least 60%, for example at least 70&, such as at least 80%, for example at least 90%, such as in the range of 5 to 100%, for example in the range of 10 to 100%, such as in the range of 20 to 100%, for example in the range of 30 to 100%, such as in the range of 40 to 100%, for example in the range of 50 to 100%.

The positive control may for example be a resin bead (or optionally several resin beads kept in a separate container or well) comprising a compound known to influence the cellular response. By way of example, if the cellular response is activation of a signal transduction pathway through a cell surface receptor, then the positive control may be a resin bead comprising a known ligand of said receptor, for example a naturally occurring ligand. The negative control may be a resin bead (or optionally several resin beads kept in a separate container or well) optionally comprising a cell adhesion compound, but otherwise comprising no library member or other test compound.

In another embodiment of the selection criterion is low fluorescence. This may for example be the case, when the cellular response is inhibition of a signal transduction pathway and the reporter system comprises a gene encoding a fluorescent protein, where an active signal transduction pathway leads to expression of said gene. Then resin beads may be selected using a method comprising the steps of:
1. Determining the fluorescence intensity of positive control resin beads and setting this fluorescence intensity to 0%
2. Determining the fluorescence intensity of negative control resin beads and setting this fluorescence intensity to 100%
3. Selecting resin beads having a fluorescence intensity corresponding to at least 5%, preferably at least 10%, more preferably at least 20%, even more preferably at least 30%, such as at least 40%, for example at least 50%, such as at least 60%, for example at leasat 70&, such as at least 80%, for example at least 90%, such as in the range of 5 to 100%, for example in the range of 10 to 100%, such as in the range of 20 to 100%, for example in the range of 30 to 100%, such as in the range of 40 to 100%, for example in the range of 50 to 100%.

The positive control may for example be a resin bead (or resin beads) comprising a compound known to influence the cellular response. By way of example, if the cellular response is inhibition of a signal transduction pathway through a cell surface receptor, then the positive control may be a resin bead comprising a known antagonist of said receptor. The negative control may be a resin bead (or resin beads) optionally comprising a cell adhesion compound, but otherwise comprising no library member or other test compound.

One method of selecting resin beads using FABS is illustrated in figure 1A.

In one preferred embodiment selection is performed manually with the aid of a fluorescence microscope. In this embodiment the fluorescence intensity or other fluorescence properties are judged manually.

When the selection criterion is fluorescence intensity of localisation, the resin beads may also be analysed using a plate reader or image acquisition.

If the selection criterion is localisation, then resin beads are generally analysed by a fluorescence or imaging microscope. Said microscope may optionally be equipped with a micromanipulator capable of picking out single beads. Resin beads are scanned for cells where the fluorescence signal is located at the desired intracellular location and these resin beads are selected. The selection may be manually or it may be automated.

In embodiments of the invention, wherein the detectable output is light emission or the detectable output may be linked to a light signal, then the predetermined selection criterion could be any property of the light. For example the selection criterion could be light intensity above or below a predetermined threshold value. Light can be detected for example by the eye, in a microscope, and if the light is emitted via bioluminescence it can be measured by a luminometer.

In embodiments of the invention, wherein the detectable output is a radioactive signal or the detectable output may be linked to a radioactive signal, then the selection criterion could be any property of said radioactive signal, such as intensity above or below a predetermined threshold value or localisation of the radioactive signal.

In embodiments of the invention, wherein the detectable output is a colour signal or the detectable output may be linked to a colour signal, then the selection criterion could be any property or said colour signal. For example the predetermined selection criterion could be a colour intensity above or below a specific threshold value or it could be a specific colour. The colour signal could be detected using any suitable colorimetric method, such as a spectrophotometer,

Resin beads comprising cells meeting at least one selection criterion, such as any of the selection criteria mentioned herein above are selected. In certain embodiments of the invention resin beads comprising cells meeting at least two, for example 2, such as 3, for example 4, such as in the range of 5 to 10, for example of in the range of 10 to 25 selection criteria are selected.

It is also possible within the present invention to select resin beads comprising cells meeting one or more predetermined selection criteria and subsequently to subject said beads to one or more additional selection rounds, wherein resin beads comprising cells meeting one or more additional selection criteria are selected.

Resin beads meeting said at least one predetermined selection criteria may be selected by manually sorting for example with the aid of a microscope, for example by sorting by fluorescence or by colour or by morphology depending on the detectable output and the selection criterion. Positive beads may be picked directly under the microscope, such as under a fluorescence microscope for example manually or with the aid of a micromanipulator. Frequently, in the range of 100 to 1,000,000, for example in the range of 1000 to 100,000, such as in the range of 5000 to 50,000 resin beads may be placed on a suitable surface, such as in a dish or on a coverglass and subsequently examined by microscopy. Alternatively, the sorting process may be automated with the use of specially designed, commercially available bead sorters (Union Biometrica, Sommerville, Mass.) and detecting for example fluorescence intensity (Meldal, 2002, Biopolymers, 66: 93-100). In general, resin beads can be sorted at a rate of about 100 beads per second, or even faster depending on the equipment used and its reading capacity. A range of about 5-30 beads per second is generally used with known instruments. Slower rates may be used to increase accuracy, however any suitable rate may be used with the present invention, such as much higher rates. Preferred, is a rate where only one resin bead passes through the detector at a time. It is also comprised within the present invention to select resin beads using a plate reader. In general in the range of 1 to 1000, such as 10 to 500, for example 50 to 100 resin beads are placed in each well of a multiter plate and analysed. Beads from positive wells may then be further examined.

In one embodiment of the invention resin beads may be selected by comparing the detectable output, with the detectable output generated by control resin beads, for example positive and/or negative control resin beads. Positive control resin beads are beads comprising a compound capable of inducing the desired cellular response, whereas negative control resin beads comprises no such compound. By way of example, if the cellular response is activation of a cell surface receptor with a known natural ligand, the positive control resin bead may comprise said ligand, whereas the negative control resin bead comprises no compound except optionally a cell adhesion compound.

If the detectable output is a quantifiable signal, then resin beads may be selected, comprising cells where the detectable output is higher or lower than the detectable output from cells attached to the positive or negative control resin bead. By way of example, if the detectable output is fluorescence intensity, then resin beads comprising cells displaying a fluorescence intensity which is higher than the negative control and lower than the positive control could for example be selected.

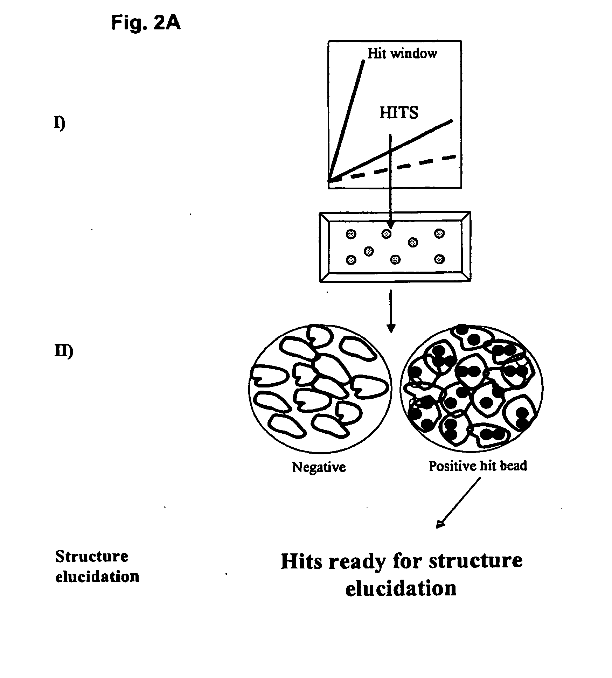

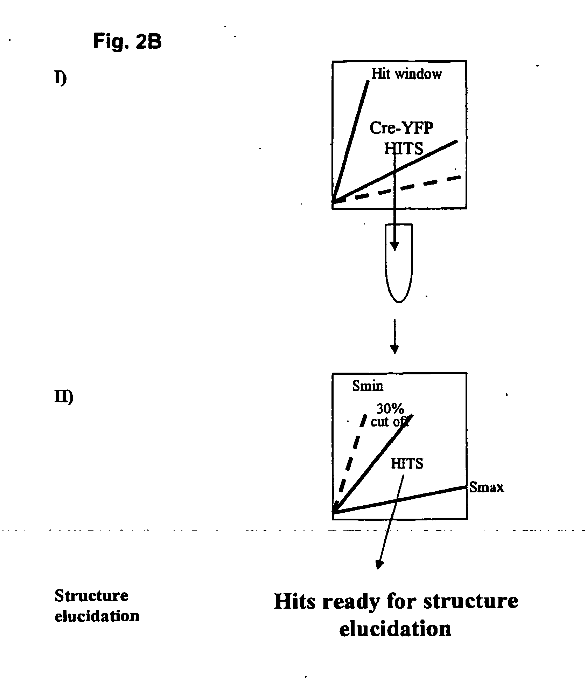

Non-limiting examples of methods of selecting resin beads are illustrates in figures 1 and 2.

### Identification of compound

Once a resin bead has been selected, the compound of said bead may be identified. Preferably, only one resin bead is used at a time. Thus if said resin bead only comprises one library member in one or more copies, then only one compound is identified at a time.

The process for identification of the library member depends on the type of library used. For a library of primarily oligomeric compounds, the library member can be analysed by Mass Spectroscopy (MS), particularly if the library was synthesized in such a way that the synthetic history of the compound is captured, for example, using a capping procedure to generate fragments of the compound that differ in mass by one building block (see, for example, Youngquist et al., 1995, J. Am Chem. Soc., 117: 3900-06). This capping procedure is most efficient when the cap and the building block are reacted at the same time. The capping agent can be any class of compound that has at least one functional group in common with the building block used to generate the oligomer, so that both the capping agent and the building block can react when added to the resin in an appropriate ratio. Alternatively, the capping agent can have two functional groups in common with the building block where one of the groups in common, such as the group in the building block that is used for the elongation of the oligomer, is orthogonally protected. For example, in a synthesis of a peptide using the Fmoc strategy, the capping agent could be the same as the building block but with a Boc group protecting the reactive amine instead of the Fmoc group (see St. Hilaire et al., 1998, J. Am. Chem. Soc., 120: 13312-13320). In another example, if the building block is a protected haloamine, the capping agent could be the corresponding alkylhalide.

Where the library is synthesized by parallel synthesis (a parallel array), the compound can be identified simply by the knowledge of what specific reaction components were reacted in a particular compartment. The structure can be confirmed by cleavage of a small portion of compound from the solid support and analyzed using routine analytical chemistry methods such as infrared (IR), nuclear magnetic resonance (NMR), mass spectroscopy (MS), and elemental analysis. For a description of various analytical methods useful in combinatorial chemistry, see: Fitch, 1998-99, Mol. Divers., 4: 39-45; and Analytical Techniques in Combinatorial Chemistry, M.E. Swartz (Ed), 2000, Marcel Decker: New York.

In a preferred embodiment however the library has been synthesised by a split-mix approach where the precise structure of the compound of a specific bead is unknown. In this embodiment, the library member can be identified using a variety of methods. The compound may be cleaved off the resin bead, and then analyzed using IR, MS, or NMR. If the library is attached to the resin bead by a cleavable linker, then the compound can be cleaved by cleaving said cleavable linker. For NMR analysis, larger beads containing approximately 5 nmoles of material are preferably used for the acquisition of 1-dimensional (1-D) and 2-dimensional (2-D) NMR spectra. Furthermore, these spectra can be attained using high-resolution MAS NMR (magic angle spinning nuclear magnetic resonance) techniques. Alternatively, high resolution-MAS NMR spectra can be acquired while the ligand is still bound to the solid support, as described for example, in Gotfredsen et al., 2000, J. Chem. Soc., Perkin Trans., 1: 1167-71. The compound may also be identified by release of the compound and fragmentation by MS-MS in MALDI or electrospray mode.

Frequently, resin beads used for library synthesis contain about 100 to 500 pmoles of material, which is generally insufficient for direct analysis using NMR techniques. In such situations, the libraries can be synthesised with special encoding to facilitate identification of the library member. For a review of encoding strategies employed in combinatorial chemistry see: Barnes et al., 2000, Curr. Opin. Chem. Biol., 4: 346-50. Most coding strategies include the parallel synthesis of the encoding molecule (for example, DNA, PNA, or peptide) along with the library compounds. This strategy requires a well-planned, time consuming, orthogonal protecting group scheme. Furthermore, the encoding molecule itself can sometimes influence the cell leading to false positives. Alternatively, the library members can be encoded using radiofrequency tags or using optical encoding, such as quantum dot encoding, spherical encoding or distance encoding. These methods alleviates the problem of false positives stemming from the coding tags, but is generally only useful for small libraries in a one-bead-one-compound system due to the sheer bulk of the radiofrequency tag. Alternatively, single beads can be analyzed in a non-destructive manner using infrared imaging. This method gives limited information and while useful for pre-screening, is not recommended for conclusive structural determination.

In a preferred embodiment of the invention the library member(s) comprised within selected resin beads are identified using mass spectrometry (MS). MS can be used alone to identify the library member. The library member can be cleaved from the resin bead, the molecular mass determined, and subsequently fragmented into subspecies to conclusively determine the structure. MS-based methods of compound identification are useful in this invention, as they require very little material, and can utilise pico- to femtomole amounts of compound. MS-based methods include for example QTOF MSMS, MSMS or QTOF LC/MSMS.

After identification of the compound it may be desirable to confirm the activity of said compounds by further in vitro and/or in vivo assays. For example, resin beads comprising the identified compound and optionally an adhesion compound may be synthesized and the cellular response confirmed. It is also possible to test identified compounds in in vitro assays in the absence of beads. Cells may for example be grown directly in a tissue culture dish, flask or coverglass and the identified compound can be added directly to the medium of said cells. If several reporter systems are available for the particular cellular response then preferably several different reporter assays may be tested in vitro, in order to identify very useful compounds. For example, induction of a signal transduction pathway by a G-protein coupled receptor frequently involves internalization of the G-protein coupled receptor as well as a transcriptional response. Reporter systems for both internalization and transcription may thus be tested.

### Multiplexing

The methods disclosed by the present invention may also be used in multiplexing methods.

For example, the methods may be used to identify compounds modifying at least two cellular responses, such as 2, for example 3, such as 4, for example in the range of 5 to 10, such as in the range of 10 to 25 cellular responses.

In such methods step c) of the method outlined above (see the section "Summary of the invention") preferably involves screening resin beads for beads comprising cells meeting at least two, such as 2, for example 3, such as 4, for example in the range of 5 to 10, such as in the range of 10 to 25 predetermined selection criteria, wherein each selection criterion is preferably related to a different detectable output.

In such a method more than one kind of cell may be attached to each resin bead and the different cellular responses may be detected in different kinds of cells. For example, a first cell line comprising a first reporter system linked to a first cellular response and a second cell line comprising a second reporter system linked to a second cellular response and optionally additional cell line(s) comprising additional reporter system(s) linked to additional cellular response(s) may all be attached to a single bead. Resin beads comprising cells meeting selection criteria linked to all the different reporter systems may then be selected.

Depending on the detectable outputs, said detectable output may be determined using any of the methods described herein above. In one preferred embodiment at least two detectable outputs are fluorescent outputs, preferably of different excitation and/or emmision. Thus resin beads meeting said at least two selection criteria may be selected in one step using a FABS with at least 2 channels in both excitation and emmision. Similarly, more than two different fluorescent properties may be selected for in an suitable FABS. The at least two detectable outputs may be in the same cell line or they may be in different cell lines.

Examples of multiplexing methods are illustrated in figure 2A and 2B.

### Examples

### Example 1

### Screening of adhesion peptide library

Approx. 100 adhesion peptide library beads were mixed with 1x10E6 cells (BHK, CHO, U2OS, Hek) in each well of a Falcon 12 well plate using 2ml growth medium. The adhesion peptide library was prepared using the general method for coupling amino acids outlined in example 5 below and involved
- Coupling HMBA linker to PEGA-resin
- Coupling amino acid to HMBA linker
- SPPS coupling
The library consisted of heptamers of D-amino acids. The peptide library beads were PEGA beads each coupled to a potential adhesion peptide. The cells and beads were mixed gently every 15 min for 2 hrs. Supernatant with non-attached cells were removed and new growth medium added. Cells/beads were incubating for another 16 hrs. (37°C, 5% CO₂).
Cell adhesive beads were identified using a microscope with 10 x objective and positive beads were transferred to a filter paper (to suck off medium). Peptides were identified by amino acid sequencing. Examples of useful peptides are given in table 2.

### Example 1a

### Identification of an adhesion peptide with low absorption of fluorescent components from growth medium and high adhesion properties:

An adhesion D-amino peptide library was synthesized (500.000 members) as described above in Example 1 and screened for low fluorescence/high adherence properties. This was done in 4 steps:
1) Selection of low fluorescent beads by Fluorescence Activated Bead Sorting (FABS).
   The 500.000 member adhesion peptide library was FABSorted and 150.000 low fluorescent beads were isolated.
2) Selection of beads with good cell adhesion properties.
   The 150.000 low fluorescent beads were incubated with GFP expressing U2OS cells followed by FABS sorting for high fluorescence (high cell adhesion). 536 beads were isolated.
3) Identification and isolation of beads with high Hek293 cell adherence properties. The 536 beads were cleared for U2OS cells and incubated with GFP expressing Hek293 cells. 47 beads with high cell adhesion properties were isolated using a fluorescence microscope.
4) Sequence elucidation and re-synthesis of selected peptides.
   22 peptides were sequenced and six of them were re-synthesized. Based on Structure-Activity of the six peptides, four additional ones (AP-7 and AP-10) were synthesized. The peptide defined by SEQ IS 35 showed the best overall performance.

An example of a method of preparing a resin bead comprising a useful adhesion peptide is described in example 5, section "Synthesis of adhesion peptide".

### Example 2

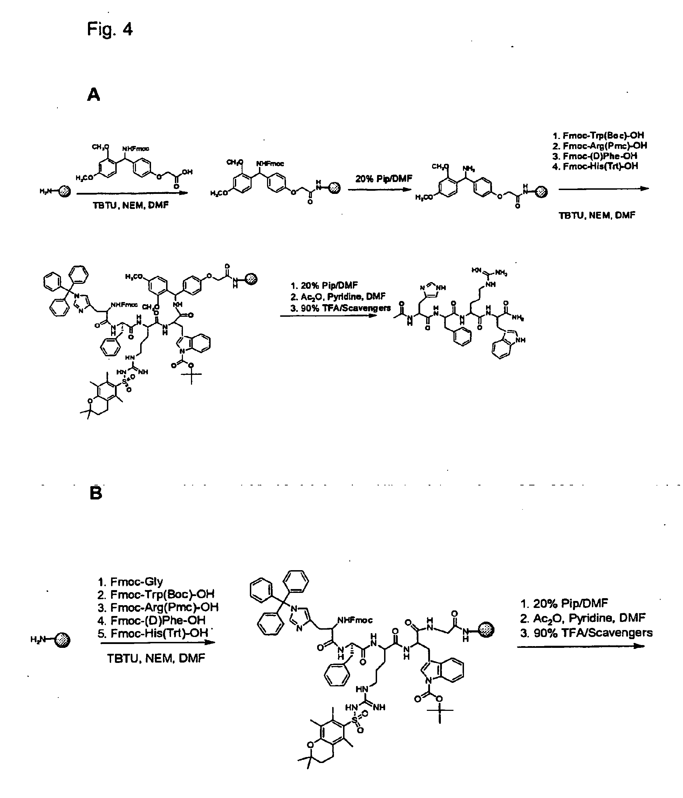

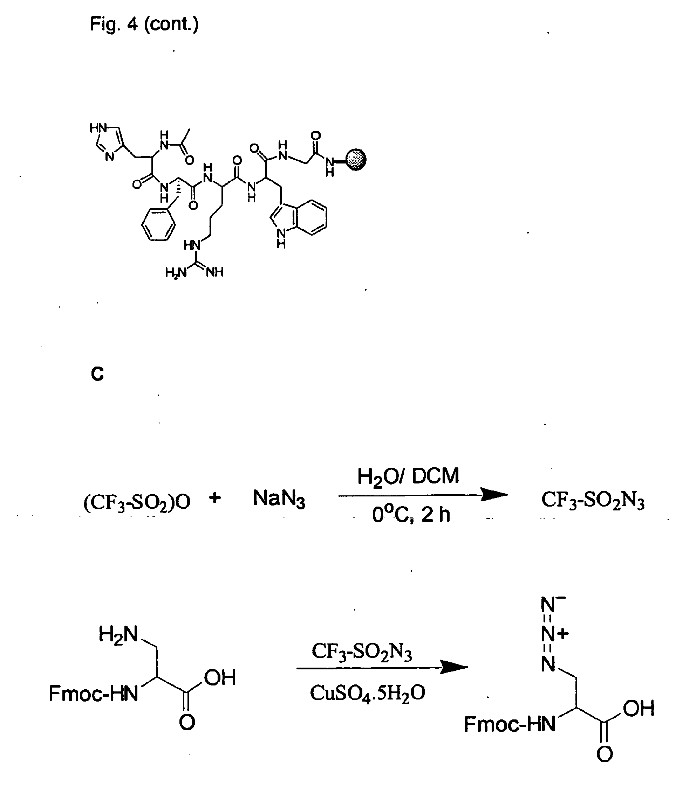

This example describes preparation of resin beads comprising His-(D)phe-Arg-Trp. These beads are for example useful as positive control for in methods for identification of compounds modulating a cellular response mediated through the melanocortin 4 receptor (MC4R). The synthesis is shown in figure 4.

### Synthesis of Ac-His-(D)phe-Arg-Trp-NH₂

An overview of the synthesis is given in figure 4A.

PEGA resin (35 mg, 0.056 mmol) was swollen in dry DMF (1 mL) and treated with Fmoc-Rink amide linker (90.65 mg, 0.168 mmol, 3 equiv) in presence of TBTU (51.77 mg, 0.224 mmol, 2.88 equiv) and NEM (28.3 µL, 0.224 mmol, 4 equiv). After 3 h at room temperature, the resin was washed with DMF (10×), MeOH (10×), DCM (10×) and dried in vacuo. The resin was negative to Kaiser amine test and a quantitative reaction was observed by measuring the Fmoc group on the resin (5 mg) with 20% Piperidine/DMF solution (8 mL) for 30 min at room temperature.

The resin was swollen in dry DMF (1 mL) and the Fmoc group was removed by 20% Piperidine/DMF (1 mL) for 20 min at room temperature. The resin was washed with DMF (10×) and the amino acids Fmoc-Trp(Boc), Fmoc-Arg(Pmc), Fmoc-(D)Phe and Fmoc-His(Trt) (3 equiv) were attached successively in presence of TBTU (2.88 equiv) and NEM (4 equiv). After the incorporation of all amino acids, the Fmoc protection was removed by 20% piperidine in DMF (1 mL, 20 min) and the resin was washed with DMF (10×). The peptide on the resin was then acetylated with aceticanhydride/pyridine/DMF (2:4:4) (1 mL) and washed with DMF (10×), MeOH (10×), DCM (10×) and dried in vacuo. The peptide was cleaved from the resin by treating with a solution of TFA (90%), water (5%), ethanedithiol (2%), triisopropyl silane (2%) and thioanisole (1%) for 3 h at room temperature. The resin was filtered off and washed with TFA (2 ×) and DCM (2 ×). The combined filtrate was concentrated under vacuum and the peptide was precipitated by ether. The peptide was washed with ether (10 ×) and dried in vacuo to afford 36.93 mg (96%) of pure peptide. 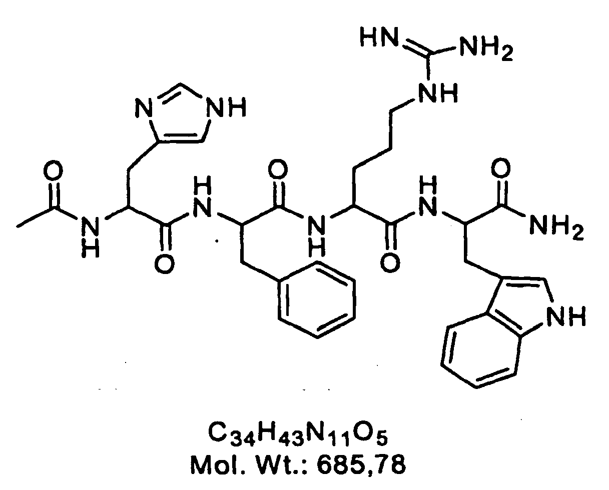 HPLC: t_{R} = 9.61 min.
ESI-MS: calcd (M+H)⁺ = 686.78 Da; found (M+H)⁺ = 686.4
MALDI TOF MS: calcd (M+H)⁺ = 686.78 Da; found (M+H)⁺ = 686.98
¹H NMR (600 MHz, MeOH-d₄): δ = 1.38-1.64 (m, 2H, Arg H^{β}), 1.10-1.15 (m, 2H, Arg H^{γ}), 2.00 (s, 3H, Acetyl CH₃), 2.96 (m, 2H Arg H^{δ}), 3.00-3.09 (m, 2H Phe H^{β}), 3.24-3.41 (m, 2H Trp H^{β}), 3.04-3.23 (m, 2H His H^{β}), 4.01 (m, 1H Arg H^{α}), 4.73 (m, 1H His H^{α}), 4.51 (m, 1H Phe H^{α}), 4.71 (m, 1 H Trp H^{α}), 7.04-7.67 (br 5H Trp ring protons), 7.21, 8.76 (2H, His ring protons), 7.25-7.33 (br, 5H Phe ring protons).

Another compound useful as positive control in methods for identification of compounds modulating a cellular response mediated through the melanocortin 4 receptor (MC4R) is alfa-MSH of the sequence Ac-Ser-Tyr-Ser-Met-Glu-His-Phe-Arg-Trp-Gly-Lys-Pro-Val-Gly-OH.

### Synthesis of Ac-His-(D)phe-Arg-Trp-Gly-PEGA₁₉₀₀

These resin beads were used as positive controls in some of the below mentioned examples.

An overview of the synthesis is given in figure 4B.

PEGA₁₉₀₀ library resin (100 mg, 0.02 mmol) was swollen in dry DMF (3 mL) and treated with Fmoc-Gly (17.84 mg, 0.06 mmol, 3 equiv) in presence of TBTU (18.5 mg, 0.058 mmol, 2.88 equiv) and NEM (10.2 µL, 0.08 mmol, 4 equiv). After 3 h at room temperature, the resin was washed with DMF (10×), MeOH (10×), DCM (10×) and dried in vacuo. The resin was negative to Kaiser amine test and a quantitative reaction was observed by measuring the Fmoc group on the resin (5 mg) with 20% Piperidine/DMF solution (8 mL) for 30 min at room temperature. The PEGA1900 library resin had previously been coupled to an adhesion peptide as described ib Example 5a, in the section "Synthesis of adhesion peptide".

The resin was swollen in dry DMF (1 mL) and the Fmoc group was removed by 20% Piperidine/DMF (1 mL) for 20 min at room temperature. The resin was washed with DMF (10×) and the amino acids Fmoc-Trp(Boc), Fmoc-Arg(Pmc), Fmoc-(D)Phe and Fmoc-His(Trt) (3 equiv) were attached successively in presence of TBTU (2.88 equiv) and NEM (4 equiv). After the incorporation of all amino acids, the Fmoc protection was removed by 20% piperidine in DMF (1 mL, 20 min) and the resin was washed with DMF (10×). The peptide on the resin was then acetylated with aceticanhydride/pyridine/DMF (2:4:4) (1 mL) and washed with DMF (10×), MeOH (10×), DCM (10×) and dried in vacuo. The side chain protection of the peptide was removed by treating with a solution of TFA (90%), water (5%), ethanedithiol (2%), triisopropyl silane (2%) and thioanisole (1%) for 3 h at room temperature and the resin was washed with DCM (10×), DMF (10×) and water (10×).

### Synthesis of Fmoc-Dap(N₃)OH

An overview of the synthesis is given in figure 4C

Fmoc-Dap-OH (980 mg, 3 mmol) was dissolved in 80% aqueous acetic acid (9 mL) and CuSO₄.5H₂O (15 mg, 0.06 mmol, 0.02 equiv) in water (1 mL) was added. The pH of the solution was adjusted to 9-10 with K₂CO₃. Water (15 mL), MeOH (32 mL) and trifluoromethanesulfonyl azide (6 mmol) in DCM (25 mL) was added and the pH was readjusted to 9-10 with K₂CO₃. The two-phase system was stirred vigorously for 20 h. The layers were separated by addition of DCM and the organic phase was washed with water (2 × 40 mL) and then the combined aqueous phases were acidified with 3 M HCl (aqueous) to a pH 2. The aqueous phase was extracted with DCM (4 × 50 mL) and the combined organic phases were dried over sodium sulfate, filtered and concentrated under vacuo (0.934 g, 88.2%).
HPLC: t_{R} = 10.08 min
ESI-MS: calcd (M+H)⁺ = 353.34 Da; found (M+H)⁺ = 353.1
¹H NMR (250 MHz, CDCl₃): δ = 3.75 (d, 2H), 4.14-4.9 (t, 1H), 4.36-4.39 (d, 2H), 4.50-4.54 (m, 1H), 5.50-5.54 (2H, NH and OH), 7.22-7.28 (4H, aromatic ring), 7.51-7.54 (d, 2H, aromatic ring), 7.68-7.71 (d, 2H, aromatic ring).

### Example 3

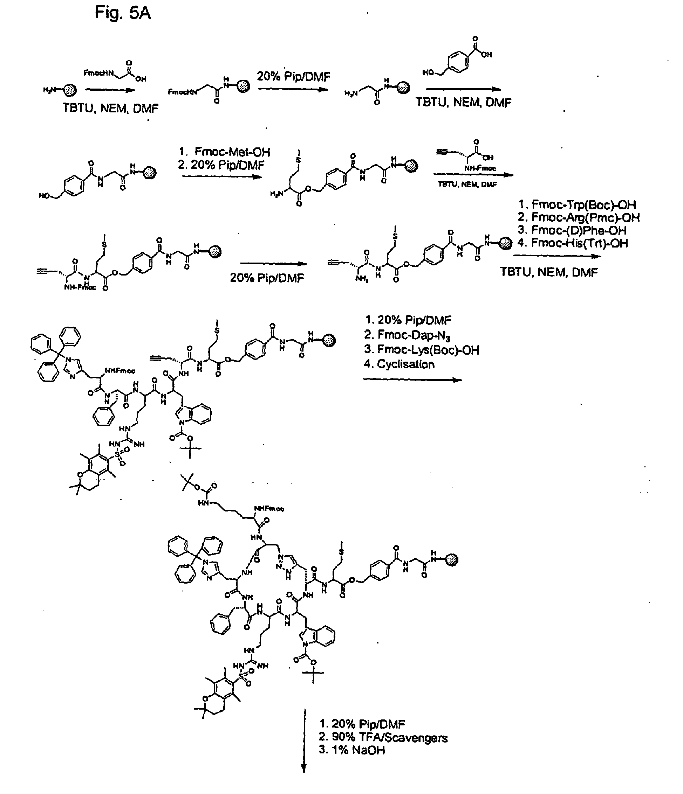

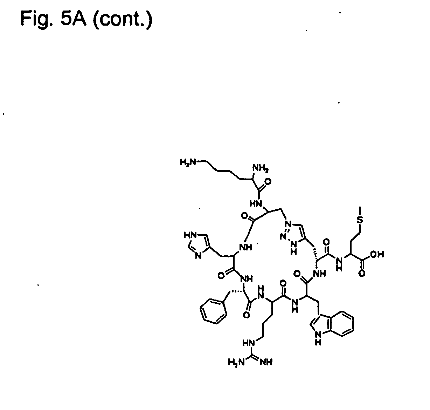

This example describes synthesis of resin beads comprising a cyclic compound, which is capable of activating for example the melanocortin 4 receptor. An overview of the synthesis is given in figure 5A.

### Synthesis of Fmoc-Lys(Boc)-Dap(N₃)-His(Trt)-(D)phe-Arg(Pmc)-Trp(Boc)-Pra-Met-HMBA-Gly-PEGA

PEGA-red-resin (150 mg, 0.24 mmol) was swollen in dry DMF (5 mL) and treated with Fmoc-Gly (215 mg, 0.72 mmol, 3 equiv) in presence of TBTU (222 mg, 0.69 mmol, 2.88 equiv) and NEM (121.8 µL, 0.96 mmol, 4 equiv). After 3 h at room temperature, the resin was washed with DMF (10×), MeOH (10×), DCM (10×) and dried in vacuo. The resin was negative to Kaiser amine test and a quantitative reaction was observed by measuring the Fmoc group on the resin (5 mg) with 20% piperidine/DMF solution (8 mL) for 30 min at room temperature.

The resin was swollen in dry DMF (5 mL), Fmoc group was removed by 20% Piperidine/DMF and treated with HMBA linker (109.5 mg, 0.72 mmol, 3 equiv) in presence of TBTU (222 mg, 0.69 mmol, 2.88 equiv) and NEM (121.8 µL, 0.96 mmol, 4 equiv). After 3 h at room temperature, the resin was washed with DMF (10×), MeOH (10×), DCM (10×) and dried in vacuo. The resin was negative to Kaiser amine test

The resin was swollen in dry DCM (2 mL), Fmoc-Met (267.5 mg, 0.72 mmol, 3 equiv), MSNT (213.4 mg, 0.72 mmol, 3 equiv) and Melm (43 µL, 0.54 mmol, 2.25 equiv) were added. After 1 h, the resin was filtered and washed with DCM (10×), MeOH (10×) and DMF (10×). The Fmoc group was removed by 20% Piperidine/DMF (1 mL) for 20 min at room temperature. The resin was washed with DMF (10×) and the amino acids Fmoc-Pra, Fmoc-Trp(Boc), Fmoc-Arg(Pmc), Fmoc-(D)Phe, Fmoc-His(Trt), Fmoc-Dap(N₃) and Fmoc-Lys(Boc) (3 equiv) were attached successively in presence of TBTU (2.88 equiv) and NEM (4 equiv). After the incorporation of all amino acids, the resin was washed with DMF (10×), MeOH (10×), DCM (10×) and dried in vacuo.

### Cyclisation of Fmoc-Lys(Boc)-Dap(N₃)-His(Trt)-(D)Phe-Arg(Pmc)-Trp(Boc)-Pra-Met-HMBA-Gly-PEGA

a. The peptidyl resin (20 mg) was treated with a solution of TFA (90%), water (5%), ethanedithiol (2%), triisopropyl silane (2%) and thioanisole (1%) for 3 h at room temperature for removing all the side chain protection groups. The resin was washed with DCM (10×), MeOH (10×) and DMF (10×). The Fmoc group was removed by 20% Piperidine/DMF (2 mL) and the resin was washed with DMF (10×), MeOH (10×), DCM (10×) and THF (10×). DIPEA (61 µL, 0.35 mmol, 50 equiv) and Cul (2.66 mg, 0.014 mmol, 2 equiv) in THF (300 µL) were added to the resin. The reaction was left for 16 h and then washed with THF, water, DMF, MeOH, DCM and dried in vacuo.
b. The peptidyl resin (20 mg) was treated with DIPEA (61 µL, 0.35 mmol, 50 equiv) and Cul (2.66 mg, 0.014 mmol, 2 equiv) in THF (300 µL) were added to the resin. The reaction was left for 16 h and then washed with THF, water, DMF, MeOH, DCM and dried in vacuo.
c. Deprotection of the cyclic peptide. A solution of TFA (90%), water (5%), ethanedithiol (2%), triisopropyl silane (2%) and thioanisole (1%) were added to the resin for removing all the side chain protection groups (3 h at room temperature). The resin was washed with DCM (10×), MeOH (10×) and DMF (10×). The Fmoc group was removed by 20% Piperidine/DMF (2 mL) and the resin was washed with DMF (10×), MeOH (10×), DCM (10×) and dried in vacuo.

### Cleavage of peptide from the resin

The resin was treated with 0.1 M NaOH (100 µL) for 2 h at room temperature. The resin was filtered and the filtrate was neutralised with 0.1 M HCl (100 µL).
(a) Yield= 8.1 mg (82.5%)
(b) Yield= 7.8 mg (79%)
HPLC: t_{R} = 10.89 min
ES MS/MS: calcd (M+H)⁺ = 1112.29 Da; found (M+H)⁺ = 1112.56 Da
¹H NMR (600 MHz, DMSO-d₆): 1.259-1.273 (m, 2H Arg H^{γ}), 1.311-1.332 (m, 2H Lys H^{γ}), 1.508-1.514 (m, 2H Lys H^{δ}), 1.416-1.616 (m, 2H Arg H^{β}), 1.650-1.669 (m, 2H Lys H^{β}), 1.852-1.979 (m, 2H Met H^{β}), 2.022 (s, 3H Met -CH₃), 2.461 (t, 2H Met H^{γ}), 2.484-2.577 (m, 2H Pra H^{β}), 2.671-2.848 (m, 2H His H^{β}), 2.721-2.944 (m, 2H Phe H^{β}), 2.728-2.734 (t, 2H Lys H^{ε}), 2.961-3.157 (m, 2H Trp H^{β}), 2.998-3.004 (m, 2H Arg H^{δ}), 3.366-3.568 (m, 2H Dap H^{β}), 3.794 (m, 1 H Lys H^{α}), 4.282 (m, 1 H Arg H^{α}), 4.309 (m, 1H Met H^{α}), 4.417 (m, 1H Pra H^{α}), 4.521 (m, 1H Dap H^{α}), 4.568 (m, 1H Trp H^{α}), 4.584 (m, 1H His H^{α}), 4.662 (m, 1H Phe H^{α}), 7.164-7.239 (br, 5H Phe ring protons), 7.201, 8.211 (2H, His ring protons), 7.447 (s, 1H Arg -NH), 6.955-7.312 (br, 5H Trp ring protons), 8.240 (s, 1H Triazole ring proton), 8.094 (1H Trp amide H), 8.185 (1 H Met amide H), 8.213 (1H Phe amide H), 8.250 (1H Pra amide H), 8.329 (1H Arg amide H), 8.408 (1H His amide H), 8.805 (1H Dap amide H), 10.697 (1H Trp ring NH).

### Example 4

### Synthesis of cyclic peptide library

The cyclic peptide library of example 4 is for example useful for identification of compounds capable of modulating a cellular response mediated through the melacortin 4 receptor.

### Fmoc-Lys(Boc)-Dap(N₃)-Aa1-Aa2 -Aa3-Aa4-Pra-Met-HMBA-Gly-PEGA-NH-Gly-Alloc

PEGA resin (1.5 g, 0.3 mmol) is swollen in dry DMF (15 mL) and treated with a mixture of Fmoc-Gly (268 mg, 0.9 mmol, 3 equiv) and Alloc-Gly (143 mg, 0.9 mmol, 3 equiv) by preactivation with TBTU (277 mg, 0.86 mmol, 2.88 equiv) and NEM (152 µL, 1.2 mmol, 4 equiv) and slow addition of the activated mixture to the resin. After 3 h at room temperature, the resin is washed with DMF (10×), MeOH (10×), DCM (10×) and dried in vacuo. The resin is negative to Kaiser amine test and a 1:2 ratio of Alloc:Fmoc is observed by measuring the Fmoc group on the resin (5 mg) with 20% Piperidine/DMF solution (15 mL) for 30 min at room temperature and determination of the absorption of the eluate at 305 nm.

The resin is swollen in dry DMF (15 mL), Fmoc group is removed by 20% Piperidine/DMF and treated with HMBA linker (92 mg, 0.6 mmol, 3 equiv) in presence of TBTU (185 mg, 0.58 mmol, 2.88 equiv) and NEM (102 µL, 0.8 mmol, 4 equiv). After 3 h at room temperature, the resin is washed with DMF (10×), MeOH (10×), DCM (10×) and dried in vacuo. The resin is negative to Kaiser amine test.

The resin is swollen in dry DCM (20 mL), Fmoc-Met-OH (223 mg, 0.6 mmol, 3 equiv), MSNT (178 mg, 0.6 mmol, 3 equiv) and Melm (36 µL, 0.45 mmol, 2.25 equiv) were added. After 1 h, the resin is filtered and washed with DCM (10×), MeOH (10×) and DMF (10×). The Fmoc group is removed by 20% Piperidine/DMF (15 mL) for 20 min at room temperature. The resin is washed with DMF (10×) and Fmoc-Pra is attached to the resin in presence of TBTU and NEM. The resin is transferred to a 20 well multiple column peptide synthesiser and distributed equally in to each wells. Amino acids Fmoc-Aa4-OH, Fmoc-Aa3-OH, Fmoc-Aa2-OH, Fmoc-Aa1-OH, Fmoc-Dap(N₃) and Fmoc-Lys(Boc) (3 equiv) are attached successively in presence of TBTU (2.88 equiv) and NEM (4 equiv). After the incorporation of all amino acids, the resin is washed with DMF (10×), MeOH (10×), DCM (10×) and dried in vacuo.

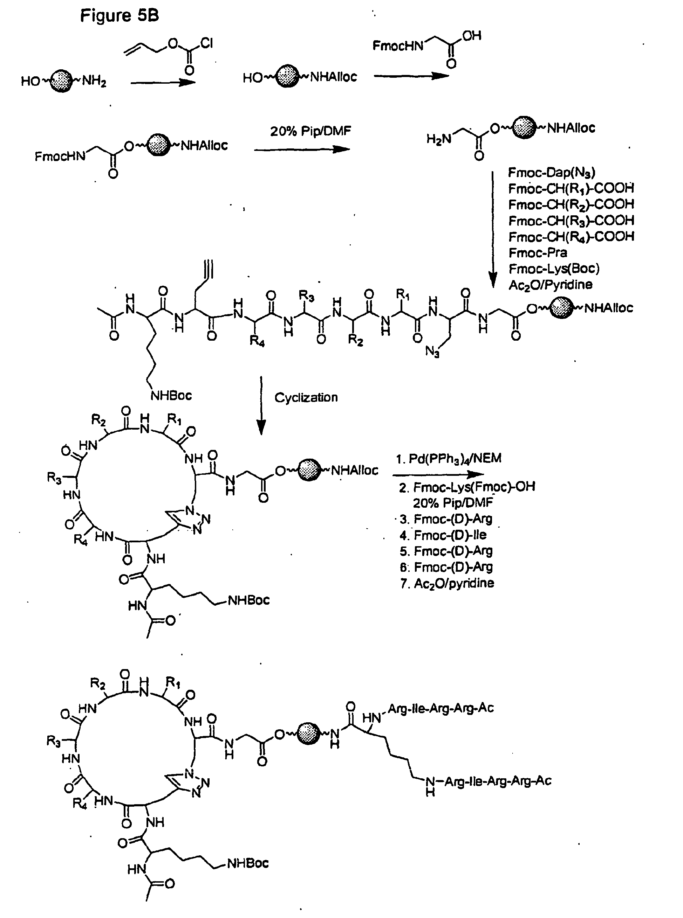

The synthesis is illustrated in figure 5B.

Fmoc-NH-CH(R₁)-CO may be any natural amino acids coupled to Fmoc

Fmoc-NH-CH(R₂)-CO; Fmoc-NH-CH(R₃)-CO; Fmoc-NH-CH(R₄)-CO may be any of the following amino acids coupled to Fmoc: Cys,Phe, His, Lys, Met, Pro, Arg, Ser, Thr, Val, Trp, Tyr, Homophenyl alanine, Tic, 4-Phenyl pyrrolidone 2-carboxylic acid, 1-Aminocyclohexane carboxylic acid, 4-Pyridyl alanine, (D)-Om Hyp, 4-Phenyl peperidine carboxylic acid.

### Cyclisation of Fmoc-Lys(Boc)-Dap(N₃)-Aa1-Aa2- Aa3-Aa4-Pra-Met-HMBA-Gly-PEGA-NH-Alloc

The peptidyl resin is treated with a solution of TFA (90%), water (5%), ethanedithiol (2%), triisopropyl silane (2%) and thioanisole (1%) for 3 h at room temperature for removing all the side chain protection groups. The resin is washed with DCM (10×), MeOH (10×) and DMF (10×). The Fmoc group is removed by 20% piperidine/DMF (15 mL) and the resin is washed with DMF (10×), MeOH (10×), DCM (10×) and THF (10×). DIPEA (1.75 mL, 10 mmol, 50 equiv) and Cul (76.2 mg, 0.4 mmol, 2 equiv) in THF (10 mL) are added to the resin. The reaction is left for 16 h and then washed with THF, water, DMF, MeOH, DCM and dried in vacuo.

The cyclisation process is illustrated in figure 5B

### Synthesis of adhesion peptide ((D)Arg-(D)Arg-(D)Ile-(D)Arg-Gly) on Cyclic peptide library beads

### a. Alloc deprotection

Pd(PPh₃)₄ (346.5 mg, 0.3 mmol, 3 equiv) is dissolved in acetic acid (5%) and NEM (2.5%) in chloroform (15 mL) and degassed by purging with Ar for 10 min. The reaction mixture is added to the lyophilised resin under Ar atmosphere and kept for 20 min at room temperature.

### b. Synthesis of adhesion peptide

The resin is washed with DMF (10×) and Fmoc-Lys(Boc) (141 mg, 0.3 mmol, 3 equiv) is attached using TBTU (92 mg, 0.288 mmol, 2.88 equiv) and NEM (51 µL, 0.4 mmol, 4 equiv). The resin is washed with DMF (10×) and the α-Fmoc and side chain Boc protections are removed by 20% piperidine (15 mL) and 30% TFA in DCM (20 mL) respectively. The resin is again treated with Fmoc-Lys(Boc) (282 mg, 0.6 mmol, 3 equiv) and TBTU (184 mg, 0.576 mmol, 2.88 equiv) and the Fmoc and Boc protections are removed by 20% piperidine in DMF and 30% TFA in DCM. The resin is washed with DMF (10×) and two residues of Ahx are attached by adding TBTU activated Fmoc-Ahx (425 mg, 1.2 mmol, 3 equiv). The amino acids Fmoc-Gly, Fmoc-(D) Arg(Pmc) and Fmoc-(D) Ile (3 equiv) are attached according to the sequence in presence of TBTU (2.88 equiv) and NEM (4 equiv). After the incorporation of all amino acids, the N-terminal Fmoc group is removed by 20% piperidine in DMF (15 mL) and the resin is washed with DMF (10×), MeOH (10×), DCM (10×) and dried in vacuo.

The peptidyl resin is treated with a solution of TFA (90%), water (5%), ethanedithiol (2%), triisopropyl silane (2%) and thioanisole (1%) for 3 h at room temperature for removing all the side chain protection groups. The resin is washed with DCM (10×), MeOH (10×) and DCM (10×) and dried in vacuo.

### Example 5

### Library of oligocyclic ureas as peptidomimetics.

This library is for example useful for identification of compounds modulating a cellular response mediated through a G-protein coupled receptor.

Synthesising combinatorial library of potential urea GPCR agonist via SPPS and the Pictet-Spengler reaction:

### Experimental:

### General:

All chemicals described, apart from the building block O-Pfp carbamates are commercially available and used without further purification. The building block O-Pfp carbamates are prepared as described in: Diness, F.; Beyer, J.; Meldal, M.; J. Combi. Chem. and QSAR. 2004, 23, 1-15. All solvents are HPLC-grade. PEGA₉₀₀ - resin is purchased from VersaMatrix A/S. Each washing step lasts 2 min unless otherwise stated.

### Coupling of HMBA linker to PEGA₉₀₀ - resin:

Dry PEGA₉₀₀ - resin is swelled in DCM and washed with DMF (3×). 3.0 eq. HMBA, 2.9 eq. TBTU and 3.0 eq. NEM are mixed in appropriate DMF and allowed to react for 10 min. The mixture is added to resin and after 2h the resin is washed with DMF (6×), DCM (6×) and lyophilised.

### General Procedure for Coupling of Amino Acid to HMBA-linker

Dry PEGA₉₀₀ - resin with HMBA-linker is swelled in DCM. 3.0 eq. Fmoc-protected amino acid, 2.25 eq. Melm and 3.0 eq. MSNT are mixed in appropriate amount of DCM and added to resin. After 1h the resin is washed with DCM (3x) and the coupling is repeated as above once. After coupling for 1 h the resin is washed with DCM (6×), DMF (6×), DCM (6×) and lyophilised.

### General SPPS Coupling Procedure

The terminal amino acid on the resin is Fmoc-deprotected by treatment with 20% piperidine in DMF (1×2 min + 1×18 min) followed by washing with DMF (6×). 3.0 eq. Fmoc-protected amino acid, 2.9 eq. TBTU and 3.0 eq. NEM are mixed in appropriate amount of DMF and allowed to react for 10 min. The mixture is added to the resin and after 2h the resin is washed with DMF (6×).

### General Building Block Coupling Procedure

The terminal amino acid on the PEGA₉₀₀ - resin with HMBA linker and tetrapeptide is Fmoc-deprotected by treatment with 20% piperidine in DMF (1×2 min + 1 × 17 min) followed by washing with DMF (6×). 3.0 eq. building block -O-Pfp carbamate is dissolved in appropriate amount of DMF and the solution is added to resin. After ended coupling the resin is washed with DMF (6×), DCM (6×) and lyophilised.

### General Pictet-Spengler Reaction Procedure

Dry PEGA₉₀₀ - resin with HMBA linker, peptide and building block is swelled in 10% TFA (aq) (1 × 1 h and 1×11 h). The resin is washed with H₂O until washing water has pH = 6-7 and washed with DMF (6×), DCM (6×) and lyophilised.

### General Side Chain Deprotection Procedure

Dry PEGA₉₀₀ - resin with HMBA linker, peptide and Pictet-Spengler product is swelled in H₂O and the side chains are deprotected with 95% TFA (aq) (2×15 min). The resin is washed with H₂O until washing water had pH = 5-7. The resin is then washed with DMF (6×), DCM (6×) and lyophilised.

### General HMBA Cleavage Procedure

Dry PEGA₉₀₀ - resin with HMBA linker and attached compound is swelled in water and NaOH (aq.) 0.1 M is added. After 2h HCl (aq.) 0.1 M is used for neutralisation and then AcN was added until the H₂O/AcN ratio is 1:1 by volume. The resin is filtered off and the liquid is used direct for RP-HPLC or/and Q-TOF MS analysis.

### Synthesising the combinatorial library:

Dry PEGA₉₀₀ - resin (1.0 g, 0.2 mmol) is coupled with HMBA linker as described in "Coupling of HMBA linker to PEGA₉₀₀ - resin" an equimolar mixture of Fmoc-glycine and Alloc glycine is the coupled to the HMBA functionalised PEGA₉₀₀ - resin as described in "General Procedure for Coupling of amino acid to HMBA linker". An analytical sample is cleaved by "General HMBA Cleavage Procedure" and tested by RP-HPLC. The Fmoc-Gly-HMBA-PEGA₉₀₀ - resin is swelled in DCM, washed with DMF (6×) and divided into the wells of a 20-welled peptide synthesiser. The 10 different amino acids are coupled to the glycine using "General SPPS Coupling Procedure". The resin from all the wells is mixed again and divided into the wells of a 20-welled peptide synthesiser and the 10 different amino acids are coupled to the terminal amino acid using "General SPPS Coupling Procedure". The resins from all the wells are mixed again and divided into the wells of a 20-welled peptide synthesiser and the 10 different trypthophan derivatives acids are coupled to the terminal amino acid using "General SPPS Coupling Procedure". The resins from all the wells is mixed again and divided into the wells of a 20-welled peptide synthesiser and the 10 different building blocks are coupled to the terminal amino acid using "General Building Block Coupling Procedure". The resin from all the wells is mixed again and the Pictet-Spengler reaction is performed as described in "General Pictet-Spengler Reaction Procedure". The Alloc group is removed from amino groups with 5mol% Pd(P(Ph₃))₄ in DMF containing 1% morpholinium acetate. Boc/tBu/Pcm protected adhesion peptide 4(2eqv) is coupled using TBTU/NEM preactivation (5 min, 0°C) for 14 h, until Kaiser test showdcomplete reaction. This is followed by Boc-, Bu*^{t}*- and Pmc- deprotection as described in "General Side Chain Deprotection Procedure". Finally analytical samples are cleaved from single beads by "General HMBA Cleavage Procedure" and tested by Q-TOF MS and MSMS analysis.

The structure of the resulting library members is given below. 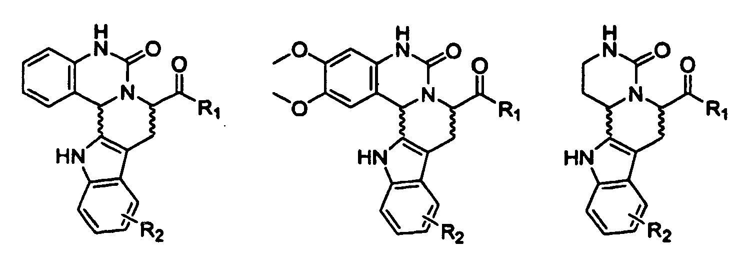 R₁ = dipeptide. Any combination of Gly, L-Trp, L-Arg, D-Arg, L-His, L-Phe, O-Phe, L-Lys, L-Asn, 4-amino-L-Phe
R₂ = H, 5-OH, 5-Br, 6-F, 7-N₃, 5-OMe 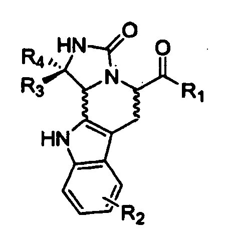 R₁ = dipeptide. Any combination of Gly, L-Trp, L-Arg, D-Arg, L-His, L-Phe, D-Phe, L-Lys, L-Asn, 4-amino-L-Phe
R₂ = H, 5-OH, 5-Br, 6-F, 7-N₃, 5-OMe
R₃=R₄=Me
or
R₃ = H and R₄ = H, iPr, H₂N-CH₂, Ph-CH₂, (4-HO-)Ph-CH₂ or indo-2-ly-CH₂
or
R₃=Phe and R₄ = H

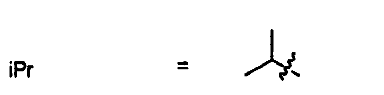

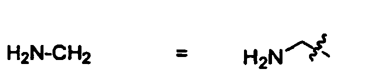

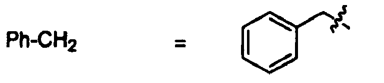

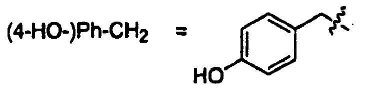

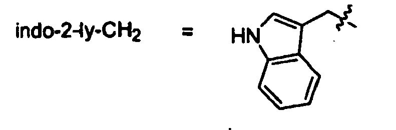

### Example 5a

Library of oligocyclic ureas as peptidomimetics 2.

This library is for example useful for identification of compounds modulating a cellular response mediated through a G-protein coupled receptor.

Synthesising combinatorial library of potential urea GPCR agonist via SPPS and the Pictet-Spengler reaction:

### Experimental:

### General:

All chemicals described, apart from the building block O-Pfp carbamates are commercially available and used without further purification. The building block O-Pfp carbamates are prepared as described in: Diness, F.; Beyer, J.; Meldal, M.; J. Combi. Chem. and QSAR. 2004, 23,1-15. All solvents are HPLC-grade. PEGA₉₀₀ - resin is purchased from VersaMatrix A/S, Denmark. Each washing step lasts 2 min unless otherwise stated.

### General SPPS Coupling Procedure

The terminal amino acid on the resin is Fmoc-deprotected by treatment with 20% piperidine in DMF (1×2 min + 1×18 min) followed by washing with DMF (6×). 3.0 eq. Fmoc-protected amino acid or HMBA, 2.9 eq. TBTU and 4.0 eq. NEM are mixed in appropriate amount of DMF and allowed to react for 10 min. The mixture is added to the resin and after 2h the resin is washed with DMF (6×).

### General Procedure for Coupling of Amino Acid to HMBA-linker

Dry PEGA₁₉₀₀ - resin with HMBA-linker is swelled in DCM. 3.0 eq. Fmoc-protected amino acid, 2.25 eq. Melm and 3.0 eq. MSNT are mixed in appropriate amount of DCM and added to resin. After 1h the resin is washed with DCM (3x) and the coupling is repeated as above once. After coupling for 1h the resin is washed with DCM (6×), DMF (6×), DCM (6×) and lyophilised.

### General Building Block Coupling Procedure

The terminal amino acid on the PEGA₁₉₀₀ - resin with HMBA linker and tetrapeptide is Fmoc-deprotected by treatment with 20% piperidine in DMF (1×2 min + 1×17 min) followed by washing with DMF (6×). 3.0 eq. building block -O-Pfp carbamate is dissolved in appropriate amount of DMF and the solution is added to resin. After ended coupling the resin is washed with DMF (6×), DCM (6×) and lyophilised.

### General Pictet-Spengler Reaction Procedure

Dry PEGA₉₀₀- resin with HMBA linker, peptide and building block is swelled in 10% TFA (aq) (1×1 h and 1×11 h). The resin is washed with H₂O until washing water has pH = 6-7 and washed with DMF (6×), DCM (6×) and lyophilised.

### General Side Chain Deprotection Procedure

Dry PEGA₁₉₀₀ - resin with HMBA linker and attached compounds is swelled in H₂O and the side chains are deprotected with 95% TFA (aq) (2×15 min). The resin is washed with H₂O until washing water had pH = 5-7. The resin is then washed with DMF (6x), DCM (6×) and lyophilised.

### General HMBA Cleavage Procedure

Dry PEGA₁₉₀₀ - resin with HMBA linker and attached compounds is swelled in water and NaOH (aq.)-0:1 M is added. After 2h HCl (aq.) 0.1 M is-used for neutralisation and then AcN was added until the H₂O/AcN ratio is 1:1 by volume. The resin is filtered off and the liquid is used direct for RP-HPLC or/and Q-TOF MS analysis.

### Synthesising the combinatorial library:

Dry PEGA₁₉₀₀- resin (1.0 g, 0.2 mmol) is coupled with an equimolar mixture of Fmoc-glycine and Alloc glycine as described in "General SPPS Coupling Procedure". HMBA is coupled as described in "General SPPS Coupling Procedure". Fmoc-glycine is the coupled to the HMBA functionalised PEGA₁₉₀₀ - resin as described in "General Procedure for Coupling of amino acid to HMBA linker". The Fmoc-Gly-HMBA-Gly-PEGA₁₉₀₀-Gly-Alloc resin is swelled in DCM, washed with DMF (6x) and divided into the wells of a 20-welled peptide synthesiser. The 20 different natural L-amino acids are coupled to the glycine using "General SPPS Coupling Procedure". The resin from all the wells is mixed again and divided into the wells of a 20-welled peptide synthesiser and the 20 different natural L-amino acids are coupled to the terminal amino acid using "General SPPS Coupling Procedure". The resins from all the wells are mixed again and divided into 10 wells of a 20-welled peptide synthesiser and the 10 different Fmoc-protected tryptophane derivatives (shown in Table 6) are coupled to the terminal amino acid using "General SPPS Coupling Procedure". The resins from all the wells is mixed again and divided into 8 wells of a 20-welled peptide synthesiser and the 8 different building blocks (shown in Table 6) are coupled to the terminal amino acid using "General Building Block Coupling Procedure". The resin from each well is transferred into a syringe with a filter in the bottom. The Alloc group is removed from the resin bound glycine by using 5mol% Pd(P(Ph₃))₄ in chloroform containing 5% AcOH and 2.5% NEM under argon for 12 h. The resin is then washed with chloroform (6x), 0.5% Et₂NCS₂Na-3H₂O and 0.5% DIPEA in DMF (6x) and DMF (10x). 1.5 eq. protected adhesion peptide (AP4), 1.4 eq. TBTU and 2.0 eq. NEM are mixed in appropriate amount of DMF and allowed to react for 10 min. The mixture is added to the resin and added to resin. When the Kaiser test shows complete reaction the resin is washed with DMF (6x) and DCM (6x). The Pictet-Spengler reaction is performed as described in "General Pictet-Spengler Reaction Procedure". This is followed by side chain deprotection as described in "General Side Chain Deprotection Procedure". Finally analytical samples are cleaved from single beads by "General HMBA Cleavage Procedure" and tested by Q-TOF MS and MSMS analysis.

**Table 6**

| Tryptophane derivatives | Building Blocks |
|---|---|
| 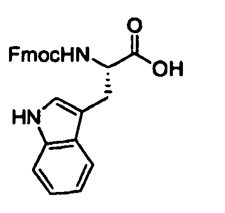 | 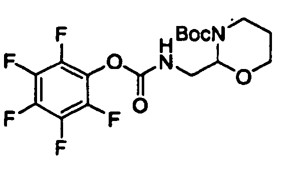 |
| | 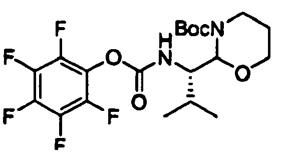 |
| 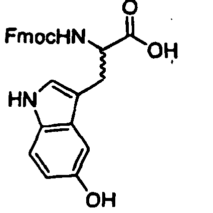 | |
| 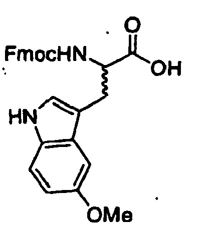 | 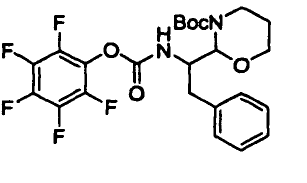 |
| 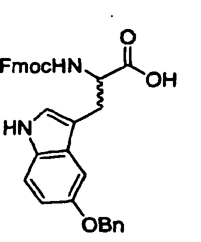 | 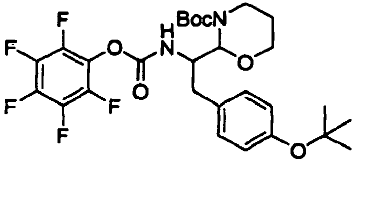 |
| 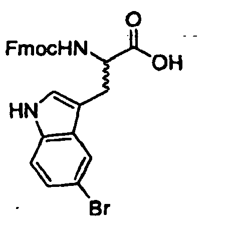 | 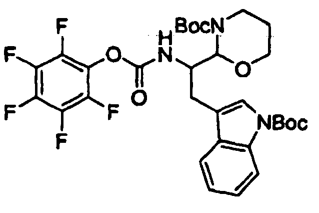 |
| 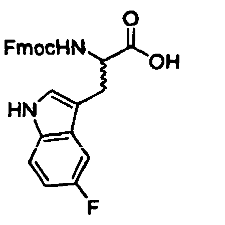 | 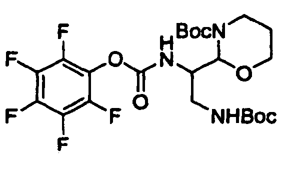 |
| 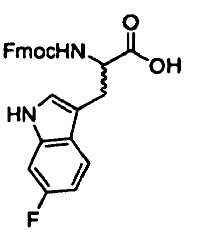 | 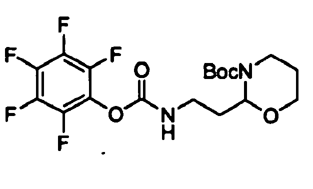 |
| 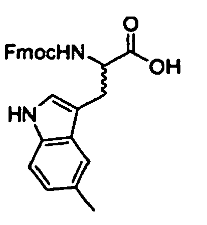 | 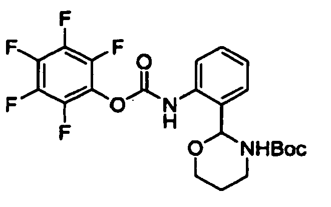 |
| 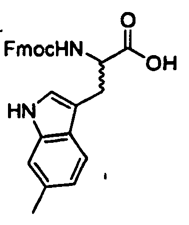 | |
| 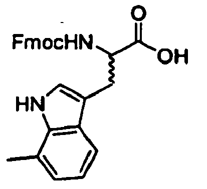 | |

The structure of the resulting library members is given below. 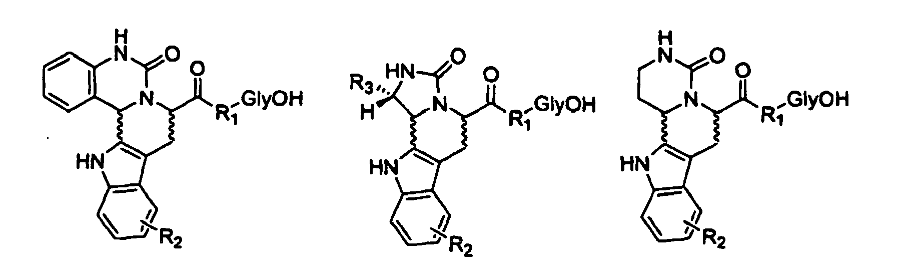 R₁ = dipeptide. Any combination of the 20 natural occuring L amino acides
R₂ = H, 5-OH, 5-OMe, 5-OBn, 5-Br, 5-F, 6-F, 5-Me. 6-Me, 7-Me
R₃ =H, iPr. H₂N-CH₂, Ph-CH₂, (4-HO-)Ph-CH₂ or indo-2-ly-CH₂

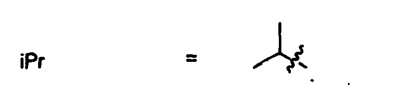

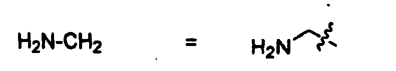

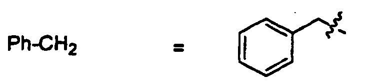

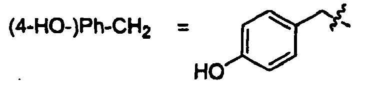

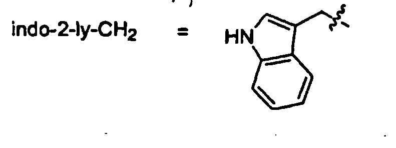

### Synthesis of protected adhesion peptide

PEGA₉₀₀ - resin is swelled in DMF. 3.0 eq. HMBA, 2.9 eq. TBTU and 4.0 eq. NEM are mixed in appropriate amount of DMF and allowed to react for 5 min. The mixture is added to resin and after 2h the resin is washed with DMF (6x), DCM (6x) and lyophilised. The resin is swelled in DCM and 3.0 eq. Fmoc-Gly-OH, 2.25 eq. Melm and 3.0 eq. MSNT are mixed in appropriate amount of DCM and added to resin. After 1 h the resin is washed with DCM (3x) and the coupling is repeated as above once. After coupling for 1 h the resin is washed with DCM (6x), DMF (6x), DCM (6x) and lyophilised. The resin is swelled in DMF and a sequence of Fmoc-gln(trt)-OH, Fmoc-arg(Pmc)-OH, Fmoc-ile-OH, Fmoc-arg(Pmc)-OH, Fmoc-lys(Boc)-OH and Boc-ala-OH is coupled as described in "General SPPS Coupling Procedure°. The resin is then washed with DMF (6x), DCM (6x) and lyophilised. The final peptide (Boc-ala-arg(Pmc)-lys(Boc)-arg(Pmc)-ile-arg(Pmc)-gln(trt)-GlyOH) is cleaved from the resin as described in "General HMBA Cleavage Procedure".

### Example 6a

Library of multi-heterocyclic peptidomimetics for GPCR receptors (Library 6a).

This library is for example useful for identification of compounds modulating a cellular response mediated through a G-protein coupled receptor.

### Library design and synthesis

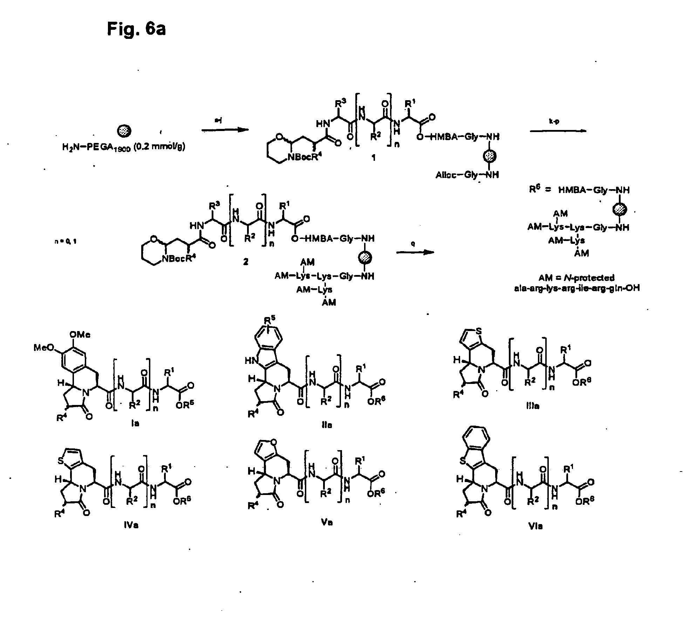

All Pictet-Spengler reaction methodology has been developed and tested on the synthesis resin PEGA₈₀₀,¹ wherefore the analogous library resin PEGA₁₉₀₀ is chosen for the library synthesis. In order to screen for active compounds, the library is prepared following a "one-bead-two-compounds" strategy. This is accomplished by treating the amino-functionalized resin with a mixture of Fmoc-Gly-OH:Alloc-Gly-OH (10:1) activated by the TBTU procedure² to provide orthogonal reaction sites for (a) split-and-mix library synthesis (via the Fmoc handle); and (b) attachment of an adhesion molecule (AM) (via the Alloc handle). The library synthesis of Pictet-Spengler reaction precursor 1 is carried out according to standard Fmoc amino acid coupling protocols for solid-phase peptide synthesis (Fig. 6a). Due to the requirement of acidic reaction conditions for the Pictet-Spengler reaction step (q), the base labile HMBA (hydroxymethylbenzoic acid) linker is employed. Prior to attachment of HMBA to H₂N-Gly-PEGA₁₉₀₀ via the TBTU activation procedure, the Fmoc protecting group is removed by standard piperidine treatment. The HMBA linker provides a convenient cleavage site for quantitative release from the solid support via basic hydrolysis. Cleavage of product from a single bead is routinely achieved by treating the bead with 0.1 M NaOH (aq) overnight, thus providing amounts of material sufficient for structure elucidation via QTOF ES-MSMS analysis. After splitting the resin portion into 10 different wells, the hydroxy handle of the linker is esterified by treatment with 10 MSNT-activated Fmoc amino acids (Fmoc-AA₁-OH)_{,}³ thus attaching the first amino acid residue of the peptidomimetic sequence. Subsequent analogous split-and-mix synthesis and 3 cycles of Fmoc deprotection/TBTU-mediated couplings of 10 Fmoc amino acids as the second amino acid residue (Fmoc-AA₂OH), 15 Fmoc amino acids incorporating the reactive aromatic side-chain (Fmoc-AA₃-OH), and 7 masked aldehyde building blocks (R⁴-MABB-OH) (Table 5a), prepared as previously reported,^{4,5} afford the Pictet-Spengler reaction precursor **1**. In this coupling sequence, one fifth of the resin is withdrawn prior to the coupling of Fmoc-AA₂-OH (steps e and f), and remixed with the remaining resin from step g and forth. Ultimately, this affords a library composed of tripeptoidal (n=0) and tetrapeptoidal (n=1) substructures. The Alloc protecting group of **1** is removed with Pd(PPh₃)₄, and subsequent TBTU coupling of Fmoc-Lys(Fmoc)-OH/Fmoc deprotection (x 2) provided the amino handles for attachment of the adhesion molecule AM, which is accomplished via the TBTU activation procedure. The adhesion molecule is synthe-sized via standard solid-phase peptide synthesis, and purified by preparative HPLC prior to attachment to resin. To finalize the library synthesis, the resin **2** is treated with 10% TFA (aq), which simultaneously facilitates the intramolecular N-acyliminium Pictet-Spengler reaction and removal of the Boc-protecting groups in the side-chains of AA₁ (R¹) and AA₂ (R²). As a consequence of the structurally diverse aromatic heterocycles undergoing the intramolecular N-acyliminium Pictet-Spengler reaction, the library is graphically represented by the six sublibraries (**Ia-VIa**) below (Fig. 6a). Theoretically, the library is composed by 11270 different compounds (32890 when all stereoisomers are counted).

An overview of the synthesis of a combinatorial library via the intramolecular N-acyliminium Pictet-Spengler reaction ^{a, b} is given in figure 6a. The amino acids and building blocks used for the library synthesis are indicated in table 5a.

Reagents and conditions: (a) Fmoc-Gly-OH:Alloc-Gly-OH (9:1), TBTU, NEM, DMF; (b) 20% piperidine (DMF); (c) HMBA, TBTU, NEM, DMF; (d) Fmoc-AA₁-OH, MSNT, Melm, CH₂Cl₂; (e) 20% piperidine (DMF); (f) Fmoc-AA₂-OH, TBTU, NEM, DMF; (g) 20% piperidine (DMF); (h) Fmoc-AA₃-OH, TBTU, NEM, DMF; (i) 20% piperidine (DMF); (j) R⁴-MABB-OH, TBTU, NEM, DMF; (k) Pd(PPh₃)₄ (CHCl₃:AcOH:NEM (925:50:25); (I) Fmoc-Lys(Fmoc)-OH, TBTU, NEM, DMF; (m) 20% piperidine (DMF); (n) Fmoc-Lys(Fmoc)-OH, TBTU, NEM, DMF; (o) 20% piperidine (DMF); (p) AM-OH, TBTU, NEM, DMF; (q) 10% TFA (aq); ^{a} Sublibraries **Ia**, **IIIa**, **IVa, Va** and **VIa** each consists of 700 different compounds (1300 when all stereoisomers are counted) with n=1, and 70 different compounds (130 when all stereoisomers are counted) with n=0; ^{b} Sublibrary **IIa** consists of 7000 different compounds (23400 when all stereoisomers are counted) with n=1, and 700 different compounds (2340 when all stereoisomers are counted) with n=0.

**Table 5a. Amino acids and building blocks for combinatorial library synthesis**

| | | | |
|---|---|---|---|
| 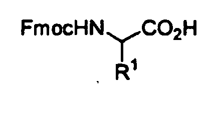 | 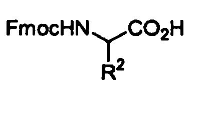 | 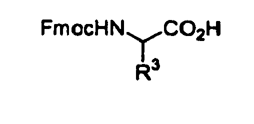 | 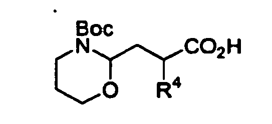 |

| Fmoc-AA₁-OH | Fmoc-AA₂-OH | Fmoc-AA₃₋OH | rac-R⁴-MABB-OH |
|---|---|---|---|
| **AA₁** | **AA₂** | **AA₃ (Sublibrary structure)** | **R⁴** |
| D-Phe | Phe | L-3,4-Dimethoxyphe (**Ia**) | H |
| D-Tyr(t-Bu) | Tyr(t-Bu) | Trp (**IIa**) | Me |
| D-Arg(Boc)₂ | Arg(Boc)₂ | D/L-(5-Br)Trp (**IIa**) | i-Bu |
| D-Lys(Boc) | Lys(Boc) | L-(5-OH)Trp (**IIa**) | Bn |
| D-His(Boc) | His(Boc) | D/L-(5-MeO)Trp (**IIa**) | Ph |
| D-Trp | Trp | D/L-(4-Me)Trp (**IIa**) | 4-Br-Ph |
| L-(1-Np)Ala | L-(1-Np)Ala | D/L-(5-Me)Trp (**IIa**) | 3-CF₃-Ph |
| L-Homophe | L-Homophe | D/L-(6-Me)Trp (**IIa**) | |
| L-(3-CN)Phe | L-(3-CN)Phe | D/L-(5-BnO)Trp (**IIa**) | |
| L-(4-CF₃)Phe | L-(4-CF₃)Phe | D/L-(5-F)Trp (**IIa**) | |
| | | D/L-(6-F)Trp (**IIa**) | |
| | | L-(2-Thi)Ala (**IIIa**) | |
| | | L-(3-Thi)Ala (**IVa**) | |
| | | L-(2-Fur)Ala (**Va**) | |
| | | L-(3-BzThi)Ala (**VIa**) | |

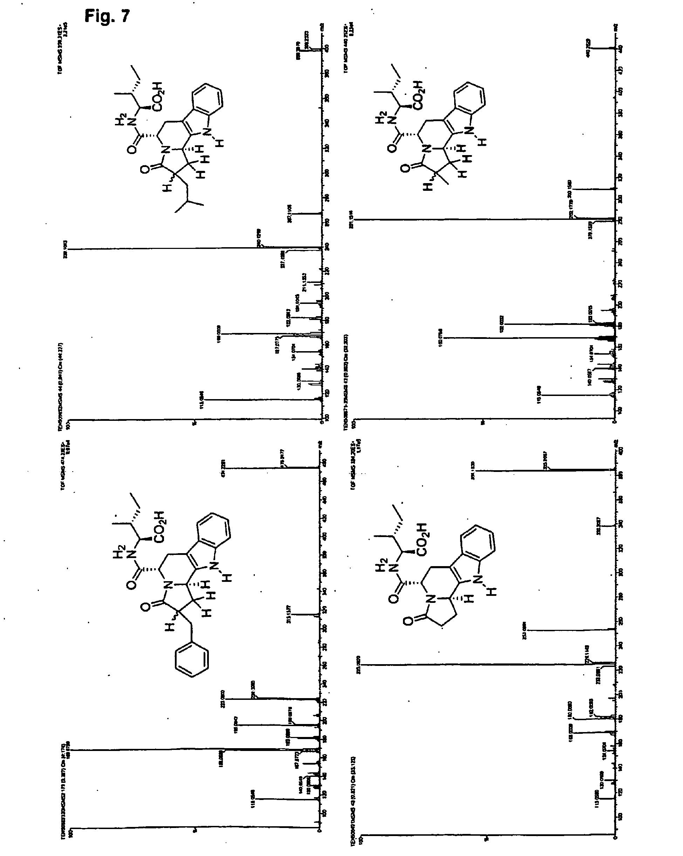

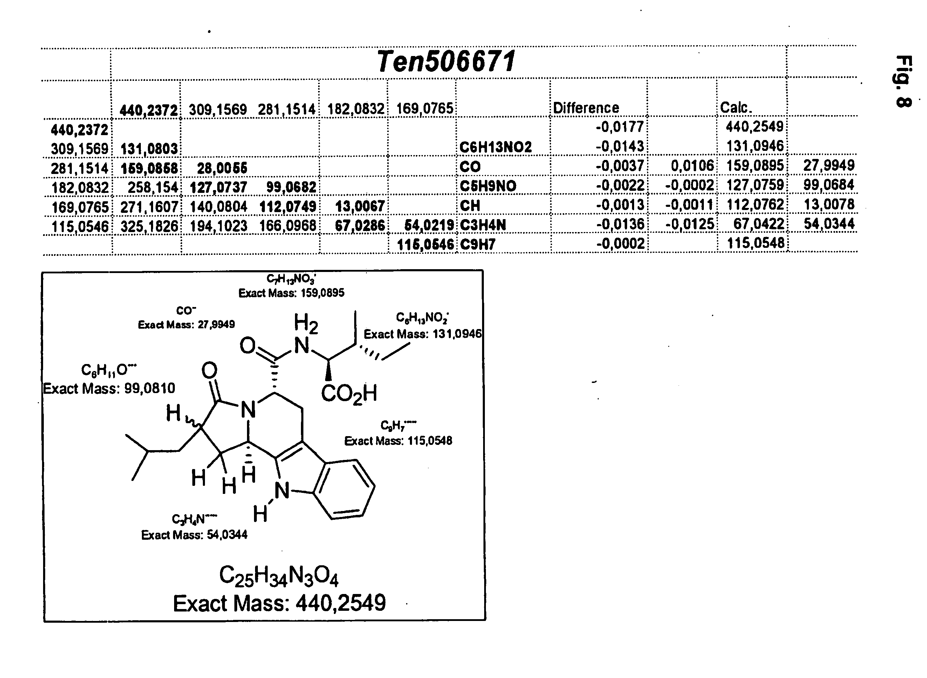

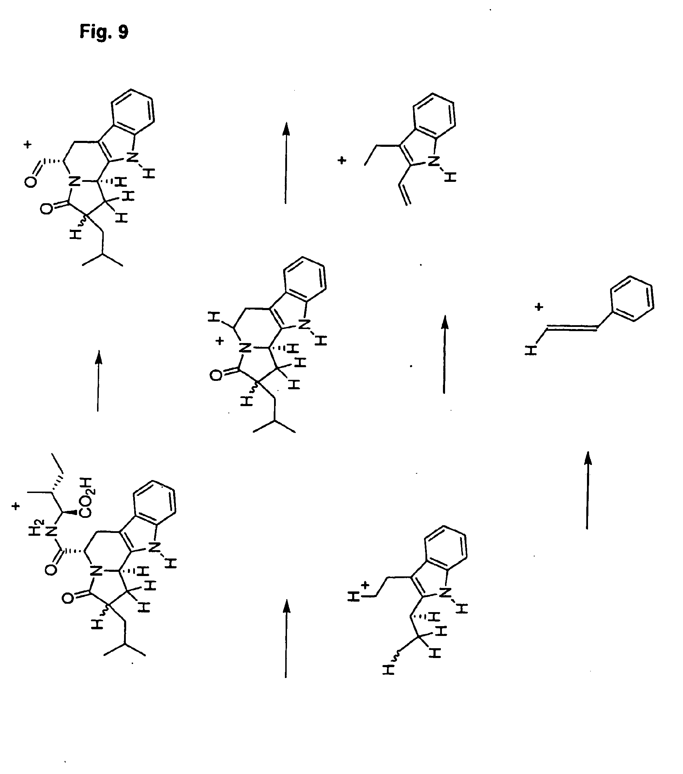

**General Methods.** All solvents are of HPLC quality and stored over molecular sieves. Solid-phase organic combinatorial chemistry is routinely carried out using a 20-well peptide synthesizer equipped with sintered teflon filters (50 µm pores), teflon tubing, and valves, which allow suction to be applied below the wells. For all reactions on solid support, PEGA₁₉₀₀ resin (0.2 mmol/g, VersaMatrix A/S) is used. Prior to use, the resin is washed with methanol (× 6), DMF (× 6), and CH₂Cl₂ (× 6). All commercially available reagents are used as received without further purification. Analysis of all solid-phase reactions is performed after cleaving the products as their free acids from the resin. A single bead is treated with 0.1 M aqueous NaOH (10 µL) in a 0.5 mL Eppendorf tube overnight, then diluted with CH₃CN (20 µL), before filtering the solution, thereby providing a sample for ES MSMS analysis on a MicroMass QTOF Global Ultima mass spectrometer (mobile phase 50% CH₃CN (aq), 0.1 µL/min) employing a linear ramping of the collision energy. Spectra (fig. 7) are analyzed by generating the exact mass differences between fragment ions and tabulated (fig. 8) to provide the fragmentation pathway (fig. 9) and therefore structure of the compound released from the single bead.

### Solid-phase synthesis of Combinatorial library (6a).

Attachment of Fmoc-Gly-OH/Alloc-Gly-OH to the amino-functionalized PEGA₁₉₀₀ resin (1.00 g) is carried out by premixing Fmoc-Gly-OH (0.62 mmol, 185 mg):Alloc-Gly-OH (0.07 mmol, 9.9 mg) (9:1, 3.0 equiv in total), N-ethyl morpholine (NEM, 0.92 mmol, 106 mg, 4.0 equiv), and N-[(1H-benzotriazol-1-yl)-(dimethylamino)methytene]-N-methylmethanaminium tetrafluoroborate N-oxide (TBTU, 0.66 mmol, 213 mg, 0.88 equiv) for 5 min in DMF. The resulting solution is added to the DMF preswollen resin and allowed to react for 5 h, followed by washing with DMF (× 6), and CH₂Cl₂ (× 6). Completion of the reaction is monitored using the Kaiser test. Prior to attachment of the HMBA linker via the procedure above, Fmoc-deprotection was accomplished with 20% piperidine in DMF, first for 2 min, and then for 18 min, followed by washing with DMF (× 6). Coupling of the first amino acid (Fmoc-AA₁-OH) to the HMBA derivatized resin is accomplished by treating the freshly lyophilized resin, split in 20 (2 × 10) wells via dry CH₂C₁₂, with a mixture of the Fmoc-AA₁-OH (4.5 equiv), Melm (3.4 equiv), and MSNT (4.5 equiv) in CH₂Cl₂:THF (5:1).³ The coupling is carried out for 1 h. When split in 20 wells, each well is assumed to hold ca. 50 mg resin, and accordingly added reagents relative to 0.01 mmol of material on the solid phase. Excess reagents are removed with suction below each well, followed by washing with dry DMF (× 1), and dry CH₂Cl₂ (× 1), before repeating the MSNT coupling of Fmoc-AA₁-OH once. Subsequent split-and-mix peptide syntheses with Fmoc-AA₂-OH, Fmoc-AA₃-OH, and R⁴-MABB-OH, respectively, are accomplished following the coupling procedure described above for the attachment of Fmoc-Gly-OH (via TBTU and NEM in DMF).² The usual washing protocol followed each coupling and deprotection step, and all couplings are checked via the Kaiser test. The Alloc group of 1 is removed by treating the resin with Pd(PPh₃)₄ (0.06 mmol, 69 mg, 3.0 equiv) in CHCl₃:AcOH:NEM (925:50:25) for 2 h. Washing is carried out with CHCl₃ (× 6), a mixture of 5% sodium diethyldithiocarbamate trihydrate and 5% DIPEA in DMF (× 2), and DMF (× 10). The free amino group of the resin (ca. 0.02 mmol) is coupled with Fmoc-Lys(Fmoc)-OH (0.06 mmol, 35 mg, 3.0 equiv.) via the TBTU activation procedure, using TBTU (0.058 mmol, 19 mg, 2.88 equiv), and NEM (0.08 mmol, 9 mg, 4.0 equiv). Following Fmoc-deprotection with 20% piperidine in DMF, first for 2 min, and then for 18 min, followed by washing with DMF (× 6), the two newly liberated amino handles are coupled with Fmoc-Lys(Fmoc)-OH (0.12 mmol, 71 mg, 3.0 equiv pr amino handle) via the TBTU activation procedure, using TBTU (0.115 mmol, 37 mg, 2.88 equiv.) and NEM (0.16 mmol, 18 mg, 4.0 equiv). Another round of Fmoc-deprotection with 20% piperidine in DMF, first for 2 min, and then for 18 min, followed by washing with DMF (x 6), provided four amino handles, which are coupled to the adhesion molecule AM-OH (0.24 mmol, 534 mg, 3.0 equiv) via the TBTU activation procedure, using TBTU (0.23 mmol, 73 mg, 2.88 equiv.) and NEM (0.32 mmol, 37 mg, 4.0 equiv). The resin is washed with DMF (x 6), and CH₂Cl₂ (× 6), and lyophilized overnight. Finally, the library synthesis is finished by treating the resin with 10% TFA (aq) for 24 h. followed by washing with water (× 6), DMF (× 6), and CH₂Cl₂ (× 6). The resin is lyophilized overnight, and kept in the freezer (-18 °C).

### Example 6b

### Library of multi-heterocyclic peptidomimetics for GPCR receptors (Library 6b).

This library is for example useful for identification of compounds modulating a cellular response mediated through a G-protein coupled receptor.

### Library design and synthesis

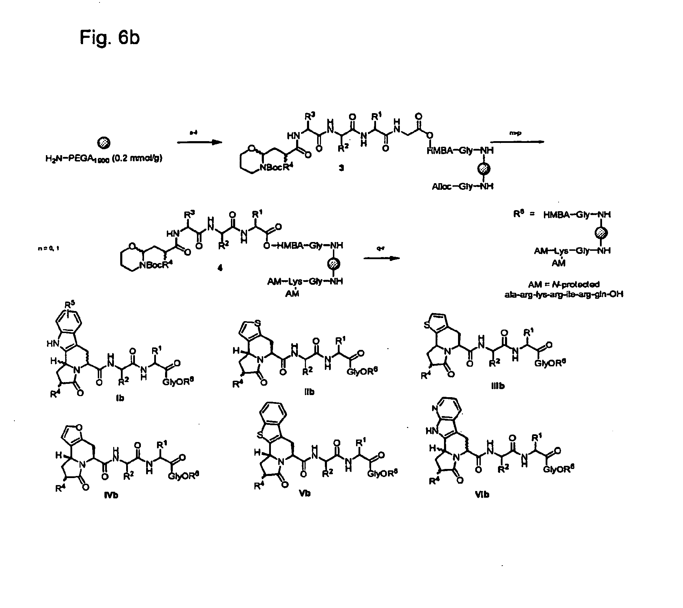

All Pictet-Spengler reaction methodology has been developed and tested on the synthesis resin PEGA₈₀₀,¹ wherefore the analogous library resin PEGA₁₉₀₀ is chosen for the library synthesis. In order to screen for active compounds, the library is prepared following a "one-bead-two-compounds" strategy. This is accomplished by treating the amino-functionalized resin with a mixture of Fmoc-Gly-OH:Alloc-Gly-OH (1:1) activated by the TBTU procedure² to provide orthogonal reaction sites for (a) split-and-mix-library synthesis (via the Fmoc handle); and (b) attachment of an adhesion molecule (AM) (via the Alloc handle). The library synthesis of Pictet-Spengler reaction precursors 3 is carried out according to standard Fmoc amino acid coupling protocols for solid-phase peptide synthesis (Figure 6b). Due to the requirement of acidic reaction conditions for the Pictet-Spengler reaction step (q), the base labile HMBA (hydroxymethylbenzoic acid) linker is employed. Prior to attachment of HMBA to H₂N-Gly-PEGA₁₉₀₀ via the TBTU activation procedure, the Fmoc protecting group is removed by standard piperidine treatment. The HMBA linker provides a convenient cleavage site for quantitative release from the solid support via basic hydrolysis. Cleavage of product from a single bead is routinely achieved by treating the bead with 0.1 M NaOH (aq) overnight, thus providing amounts of material sufficient for structure elucidation via QTOF ES-MSMS analysis. The hydroxy handle of the linker is esterified by treatment with MSNT-activated Fmoc-Gly-OH³ thus placing glycine as the first amino acid residue of the peptidomimetic sequence. Subsequent analogous split-and-mix synthesis and 4 cycles of Fmoc deprotection/TBTU-mediated couplings of 20 Fmoc amino acids as the first amino acid residue (Fmoc-AA₁-OH), 20 Fmoc amino acids as the second amino acid residue (Fmoc-AA₂-OH), 15 Fmoc amino acids incorporating the reactive aromatic side-chain (Fmoc-AA₃-OH), and 6 masked aldehyde building blocks (R⁴-MABB-OH) (table 5b), prepared as previously reported,^{4,5} afford the Pictet-Spengler reaction precursor 3. The Alloc protecting group of 3 is removed with Pd(PPh₃)₄, and subsequent TBTU coupling of Fmoc-Lys(Fmoc)-OH/Fmoc deprotection provided the amino handles for attachment of the adhesion molecule AM, which is accomplished via the TBTU activation procedure. The adhesion molecule is synthesized via standard solid-phase peptide synthesis, and purified by preparative HPLC prior to attachment to resin. To finalize the library synthesis, the resin 4 is treated with 10% TFA (aq) to facilitate the intramolecular N-acyliminium Pictet-Spengler reaction and TFA:CH₂Cl₂:H₂O:MeSPh:(CH₂SH)₂:TIPS (66.5:20:5:5:2.5:1) to remove residual protecting groups in the side-chains of AA₁ (R¹) and AA₂ (R²). As a consequence of the structurally diverse aromatic heterocycles undergoing the intramolecular N-acyliminium Pictet-Spengler reaction, the library is graphically represented by the six sublibraries (**Ib-VIb**) below (Figure 6b). Theoretically, the library is composed by 38400 different compounds (118800 different compounds when all stereoisomers are counted).

An overview of the synthesis of a combinatorial library via the intramolecular N-acyliminium Pictet-Spengler reaction ^{a, b, c} is given in figure 6b. The amino acids and building blocks used for the library synthesis are indicated in table 5b.

Reagents and conditions: (a) Fmoc-Gly-OH:Alloc-Gly-OH (1:1), TBTU, NEM, DMF; (b) 20% piperidine (DMF); (c) HMBA, TBTU, NEM, DMF; (d) Fmoc-Gly-OH, MSNT, Melm, CH₂Cl₂; (e) 20% piperidine (DMF); (f) Fmoc-AA₁-OH, TBTU, NEM, DMF; (g) 20% piperidine (DMF); (h) Fmoc-AA₂-OH, TBTU, NEM, DMF; (i) 20% piperidine (DMF); (i) Fmoc-AA₃-OH, TBTU, NEM, DMF; (k) 20% piperidine (DMF); (I) R⁴-MABB-OH, TBTU, NEM, DMF; (m) Pd(PPh₃)₄ (CHCl₃:AcOH:NEM (925:50:25); (n) Fmoc-Lys(Fmoc)-OH, TBTU, NEM, DMF; (o) 20% piperidine (DMF); (p) AM-OH, TBTU, NEM, DMF, 20 h; (q) 10% TFA (aq); (r) TFA:CH₂Cl₂:H₂:H₂O:MeSPh:(CH₂SH)₂:TIPS (66.5:20:5:5:2.5:1).^{a} Sublibrary **Ib** consists of 26400 different compounds (92400 when all stereoisomers are counted). ^{b} Sublibraries **IIb**, **IIIb**, **IVb**, and **Vb** each consists of 2400 different compounds (4400 when all stereoisomers are counted). Sublibrary **VIb** consists of 2400 different compounds (8800 when all stereoisomers are counted).

**Table 5b. Amino acids and building blocks for combinatorial library synthesis**

| | | | |
|---|---|---|---|
| 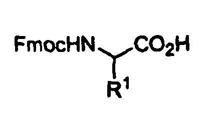 | 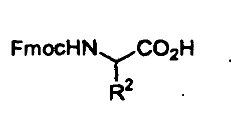 | 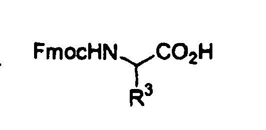 | 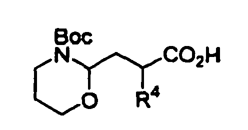 |

| Fmoc-AA₁-OH | Fmoc-AA₂-OH | Fmoc-AA₃-OH | rac-R⁴-MABB-OH |
|---|---|---|---|
| **AA₁** | **AA₂** | **AA₃ (Sublibrary structure)** | **R⁴** |
| His(Boc) | His(Boc) | Trp (**Ib**) | H |
| Asp(t-Bu) | Asp(t-Bu) | D/L-(5-Br)Trp (**Ib**) | Me |
| Arg(Pmc) | Arg(Pmc) | L-(5-OH)Trp (**Ib**) | i-Bu |
| Phe | Phe | D/L-(5-MeO)Trp (**Ib**) | Bn |
| Ala | Ala | D/L-(4-Me)Trp (**Ib**) | Ph |
| Cys(Trt) | Cys(Trt) | D/L-(5-Me)Trp (**Ib**) | CH₂OH |
| Gly | Gly | D/L-(6-Me)Trp (**Ib**) | |
| Gln(Trt) | Gln(Trt) | D/L-(5-BnO)Trp (**Ib**) | |
| Glu(t-Bu) | Glu(t-Bu) | D/L-(5-F)Trp (**Ib**) | |
| Lys(Boc) | Lys(Boc) | D/L-(6-F)Trp (**Ib**) | |
| Leu | Leu | L-(2-Thi)Ala (**IIb**) | |
| Met | Met | L-(3-Thi)Ala (**IIIb**) | |
| Asn(Trt) | Asn(Trt) | L-(2-Fur)Ala (**IVb**) | |
| Ser(t-Bu) - | Ser(t-Bu) | L-(3-BzThi)Ala (**Vb**) | |
| Tyr(t-Bu) | Tyr(t-Bu) | D/L-(7-Aza)Trp (**VIb**) | |
| Thr(f-Bu) | Thr(*t*-Bu) | | |
| Ile | Ile | | |
| Trp(Boc) | Trp(Boc) | | |
| Pro | Pro | | |
| Val | Val | | |

**General Methods**. All solvents are of HPLC quality and stored over molecular sieves. Solid-phase organic combinatorial chemistry is routinely carried out using a 20-well peptide synthesizer equipped with sintered teflon filters (50 µm pores), teflon tubing, and valves, which allow suction to be applied below the wells. For all reactions on solid support, PEGA₁₉₀₀ resin (0.24 mmol/g, VersaMatrix A/S) is used. Prior to use, the resin is washed with methanol (× 6), DMF (× 6), and CH₂Cl₂ (× 6). All commercially available reagents are used as received without further purification.

Analysis of all solid-phase reactions is performed after cleaving the products as their free acids from the resin. A single bead is treated with 0.1 M aqueous NaOH (10 µL) in a 0.5 mL Eppendorf tube overnight, then diluted with CH₃CN (20 µL), before filtering the solution, thereby providing a sample for ES MSMS analysis on a MicroMass QTOF Global Ultima mass spectrometer (mobile phase 50% CH₃CN (aq), 0.1 µL/min) employing a linear ramping of the collision energy. Spectra (fig. 7) are analyzed by generating the exact mass differences between fragment ions and tabulated (fig. 8) to provide the fragmentation pathway (fig. 9) and therefore structure of the compound released from the single bead.

**Solid-phase synthesis of combinatorial library (6b)**. Attachment of Fmoc-Gly-OH/Alloc-Gly-OH to the amino-functionalized PEGA₁₉₀₀ resin (0.24 mmol/g, 1.68 mmol, 7.00 g). The resin swelled in DMF is added solutions (i) +(ii) of TBTU-activated N-protected glycines; (i) Fmoc-Gly-OH (1.5 equiv., 2.52 mmol, 749 mg) + NEM (2.0 equiv., 3.36 mmol, 426 µL) + TBTU (1.44 equiv., 2.42 mmol, 809 mg) in 5 mL DMF (activation in the usual way); and (ii) Alloc-Gly-OH (1.5 eq, 2.52 mmol, 401 mg) + NEM (2.0 eq, 3.36 mmol, 426 µL) + TBTU (1.44 eq, 2.42 mmol, 809 mg) in 5 mL DMF (activation in the usual way). Both solutions are simultaneously added to the resin in 100 × 50 µL portions with vigorous shaking, maintaining the rate at 1 addition from each solution pr. minute. After addition of solutions (i) and (ii), the reaction mixture is further shaken for 30 min. followed by washing with DMF (x 6), and CH₂Cl₂ (× 6) in a syringe fitted with a Teflon filter. Completion of the reaction is monitored using the Kaiser test. Prior to attachment of the HMBA linker via the procedure above, Fmoc-deprotection is accomplished with 20% piperidine in DMF, first for 2 min, and then for 18 min, followed by washing with DMF (x 6). Coupling of the first amino acid (Fmoc-Gly-OH) to the HMBA derivatized resin is accomplished by treating the freshly lyophilized resin (0.84 mmol) with a mixture of the Fmoc-Gly-OH (4 eq, 3.4 mmol, 999 mg), Melm (8 eq, 6.8 mmol, 533 µL), and MSNT (4 eq, 3.4 mmol, 996 mg) in dry CH₂Cl₂ (30 mL).³ The coupling is carried out for 2h, then the resin is washed with dry DMF (× 1), and dry CH₂Cl₂ (× 1), before repeating the MSNT coupling of Fmoc-Gly-OH once. The resin is washed with DMF (× 6) and CH₂Cl₂ (× 6) prior to lyophilization for removal of all solvent traces. A batch of resin (1.00 g) is subjected to split-and-mix peptide syntheses with Fmoc-AA₁-OH, Fmoc-AA₂-OH, Fmoc-AA₃-OH, and R⁴-MABB-OH, respectively, following the coupling procedure described above for the attachment of Fmoc-Gly-OH (via TBTU and NEM in DMF).² The usual washing protocol follows each coupling and deprotection step, and all couplings are checked via the Kaiser test. The Alloc group of 3 is removed by treating the resin batch twice with Pd(PPh₃)₄ (3.0 equiv., 0.36 mmol, 416 mg) in CHCl₃:AcOH:NEM (925:50:25) for 3h. Washing was carried out with CHCl₃ (× 6) and DMF (× 10). The free amino group of the resin (0.12 mmol) was coupled with Fmoc-Lys(Fmoc)-OH (3.0 equiv., 0.36 mmol, 210 mg) via the TBTU activation procedure, using TBTU (2.88 equiv., 0.348 mmol, 114 mg) and NEM (4.0 equiv., 0.48 mmol, 54 mg). Following Fmoc-deprotection with 20% piperidine in DMF, first for 2 min, and then for 18 min, followed by washing with DMF (× 6), newly liberated amino handle is coupled to the adhesion molecule AM-OH (1.5 equiv., 0.36 mmol, 801 mg) via the TBTU activation procedure, using TBTU (2.88 equiv., 0.691 mmol, 222 mg) and NEM (4.0 equiv., 0.96 mmol, 122 µL). The resin was washed with DMF (× 6), and CH₂Cl₂ (× 6), and lyophilized overnight. The library synthesis was finished by first treating the resin with 10% TFA (aq) for 24 h, followed by washing with water (× 6), DMF (× 6), and CH₂Cl₂ (× 6), and finally with TFA:CH₂Cl₂:H₂O:MeSPh:(CH₂SH)₂:TIPS (66.5:20:5:5:2.5:1) for 5h, before washing with CH₂Cl₂ (× 6), DMF (× 6), water (× 6), DMF (x 6), and CH₂Cl₂ (× 6). The resin was lyophilized overnight, and stored in the freezer (-18 °C).

### References

(1) Meldal, M. Tetrahedron Left. 1993, 33,3077-3080.
(2) Knorr, R.; Trzeciak, A.; Bannwarth, W.; Gillessen, D. Tetrahedron Lett. 1989, 30, 1927-1930.
(3) Blankemeyer-Menge, B.; Nimtz, M.; Frank, R. Tetrahedron Lett. 1990, 31, 1701-1704.
(4) Groth, T.; Meldal, M. J. Comb. Chem. 2001, 3, 34-44.
(5) Nielsen, T. E.; Meldal, M. J. Org. Chem. 2004, 69, 3765-3773.

### Example 7:

### Gs coupled receptor (MC4R): Agonist assay (Cre-GFPreporter assay detected with a Fluorescence Activated Bead Sorter)

### Cre-GFP:

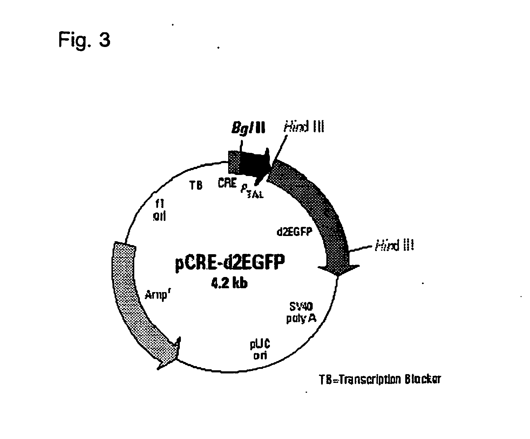

Cre-GFP is commercially available from clontech (pCre-d2eGFP) The vector contains three copies of Cre-binding sequence fused to a TATA-like promoter. The vector is holding a neomycin resistance gene. A map of the vector is shown in figure 3.

### MC4R:

PCR amplified MC4R encoding DNA is introduced into the gateway Entry Vector (pENTR) by topoisomarase-mediated ligation. The DNA is subsequently recombined into Destination Vector pDEST12.2. (pDEST12.2MC4R)

### Cell line establishment:

U2OS cells are transfected with pDEST1 .2.2MC4R using standard procedure for Fugene6 transfection. Cells are put under G418 selection for 4 weeks to obtain a cell line stably expressing MC4R.

The U2OS cell line stably expressing the human MC4R (melanocortin4 receptor) is further transfected with Cre-GFP the day before culturing them on PEGA beads displaying adhesion peptide and respectively 1)Negative control (PEGA beads with adhesion peptide, but no library compound), 2)Positive control (PEGA beads of example 2) and 3)Library compounds. The three cultures are handled separately in each their culture flask.

### Bead/cell preparation:

Cells are trypsinized and mixed with the PEGA beads in growth medium (DMEM containing 10% FCS, in the proportion 4000 cells/bead and app. 50ml growth medium/5000 beads1) Positive control: 50ml Growth medium + 5000 positive control beads + 2x10E7 cells.
2) Negative control: 50ml Growth medium + 5000 negative control beads + 2x10E7 cells.
3) Screening library (eg. 100.000 compounds): 1000ml Growth medium + 100.000 library beads + 4x10E8 cells.

The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause. Beads, now covered with cells, are allowed to sediment for 10 min (no centrifugation needed) and the growth medium is removed using a 50ml pipette. 10m1 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Bead sorting:

A Fluorescence Activated Bead Sorter (FABS) equipped with a multiline Argon laser 488 nm excitation line and 500-650 nm emission filter and sorting capability into 96 well plate is used to identify and isolate positive hit beads.
The FABS is calibrated to identify and isolate positive hit beads (increased GFP fluorescence) by determining the dynamic range of the assay using positive control beads prepared as described in Example 2 as Smax (maximum response) and negative control beads comprising only cell adhesion peptide as Smin (minimum response). A cut off at 30% response compared to negative control beads is set as threshold for a positive hit bead.
Positive hits are separated into each their well of a 96 well plate and are hereafter ready for compound elucidation, re-synthesis and re-test as well as test for effects in other assays.

This assay may also be performed using HEK cells essentially as described herein below in Example 7a, except that the HEK cells should be transfected with the Cre-GFP and pDEST1.2.2MC4R constructs. Positive resin beads may also preferably be selected using a fluorescence microscope, as described in Example 7a.

### Example 7a

### Gs coupled receptor (MC4R): Agonist assay (MC4R-GFP internalization: microscopy)

### Construction of MC4R-GFP:

996 bp of MC4R ORF sequence without stop codon is inserted into pGFP2-N1 vector (Biosignal Packard Cat.# 6310013-001) with cloning sites EcoRl/BamHl.

### Cell line establishment:

Hek293 cells are transfected with MC4R-GFP using standard procedure for Fugene6 transfection. Cells are put under zeocin selection for 4 weeks to obtain a cell line stably expressing the MC4R-GFP.

### Cell/bead preparation:

Cells were cultured on respectively 1) Negative control beads (prepared as described in example 1). 2) Positive control beads (prepared as described in example 2; Ac-His-(D)phe-Arg-Trp-Gly-PEGA₁₉₀₀) and Library beads (prepared as described in example 6b). Each batch of beads was handled separately.
Cells were trypsinized and mixed with Negative control beads/Positive control beads/Library beads in growth medium (Hams F12 containing 5% FCS)
- Add 500 beads in 500ul Hams to a 14ml Nunc tube
- Add 2500ul cell suspension 1x10E6/ml Hams w. 5% FCS
- Leave tube vertically in incubator (37 degrees, 5% CO2) for 16-24hrs - rock tube gently every 15min for the first hour
- Remove medium. Wash loose cells away by gently adding and removing 4ml Hams x2 (Turn the tube upside down and back again - as soon as beads have sedimented suck away medium)
- Add 2ml Hams w. FCS 5%
- Incubate o/n at 37 degrees, 5% CO2
- Decant beads to a 1 well Lab-Tek Chambered Coverglass (#155361)

### Hit identification and isolation

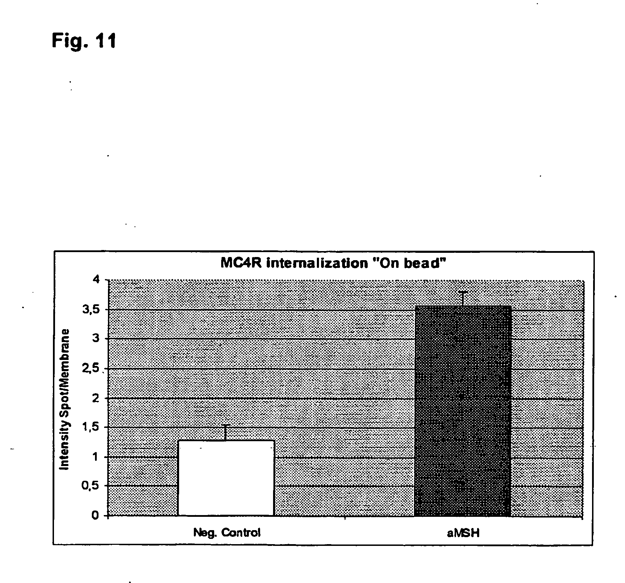

The LabTek 1 well chambered coverglass was placed on a Zeiss Axiovert 200 fluorescence microscope equipped with filters optimal for GFP fluorescence. The microscope was further more equipped with a micromanipulator (Eppendorf Transferman NK2)) capable of picking out single beads. Using 40x objective chambers were scanned for positive hit beads, which appeared as cells with green dots located in the cytoplasma in contrast to negative beads where GFP is located in the plasma membrane of the cells. Positive and negative control beads were used to set cut off for positive hit beads. Such hit beads were picked out using the micromanipulator. MC4R-GFP internalization was quantified and the results are shown in fig. 11.

### Example 8:

### Gs coupled receptor (MC4R): Agonist assay (Multiplexed Cre-YFP reporter and MC4R-GFPintemalization: FABS and microscopy)

### Construction of pCRE-d2EYFP:

A 732 bp of EYFP fragment from pd2EYFP-1 (Clontech Cat.#6912-1) is ligated to a 3.5 kb fragment from pCRE-d2EGFP (Clontech Cat #6034-1). Both fragments are excised from the two vectors by a common restriction enzyme digestion.

### Construction of MC4R-GFP:

996 bp of MC4R ORF sequence without stop codon is inserted into pGFP2-N1 vector (Biosignal Packard Cat.# 6310013-001) with cloning sites EcoRl/BamHl.

### Cell line establishment:

U2OS cells are transfected with MC4R-GFP using standard procedure for Fugene6 transfection. Cells are put under zeocin selection for 4 weeks to obtain a cell line stably expressing the MC4R-GFP.
The U2OS cell line stably expressing the human MC4R-GFP (melanocortin4 receptor-GFP) is further transfected with Cre-YFP the day before culturing them on PEGA beads displaying adhesion peptide and respectively 1)Negative control (PEGA beads with adhesion peptide, but no library compound), 2)Positive control (PEGA beads of example 2) and 3)Library compounds. The three cultures are handled separately in each their culture flask.

### Bead/cell preparation:

Cells are trypsinized and mixed with beads in growth medium (DMEM containing 10% FCS, in the proportion 4000 cells/bead and app. 50ml growth medium/5000 beads.
1) Positive control: 50ml Growth medium + 5000 positive control beads + 2x10E7 celts.
2) Negative control: 50ml Growth medium + 5000 negative control beads + 2x10E7 cells.
3) Screening library (eg. 100.000 compounds): 1000ml Growth medium + 100.000 library beads + 4x10E8 cells

The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Bead sorting for Cre-YFP response:

A Fluorescence Activated Bead Sorter (FABS) equipped with 514 nm excitation laser line and 528-572 nm emission filter is used to identify and isolate positive hit beads.
The FABS is calibrated to identify and isolate positive hit beads (increased YFP fluorescence) by determining the dynamic range of the assay using positive control beads as Smax (maximum response) and negative control beads as Smin (minimum response). A cut off at 30% response compared to negative control beads is set as threshold for a positive hit bead.
Positive hit beads are isolated into a 1 well Nunc chamber and are hereafter ready to test for receptor internalisation.

### MC4R-GFPintemalisation: Microscope analysis

The Nunc chamber with positive Cre-YFP hits is placed on an imaging microscope (Zeiss Axiovert 200M) equipped with filters allowing separation of YFP and GFP. Further more the microscope is equipped with a micromanipulator (Eppendorf Transferman NK2)) capable of picking out single beads. Using 20× objective the chamber is scanned for positive MC4R-GFPinternalisation (appear as intracellular spots as compared to membrane distribution in non positive MC4R-GFP intemalisation) and such hit beads are picked out using the micromanipulator for compound structure elucidation.

A multiplexed screening like this is expected to give very low rate of false positive hits since hits picked out for structure elucidation are giving rise to both specific receptor activation (internalisation of receptor) as well as a functional response (activation of transcription of Cre-YFP).

### Example 9:

### Gs coupled receptor (MC4R): Agonist assay (Cre-YFP reporter and HA-MC4R internalization: FABS and Microscopy)

### Construction of pCRE-d2EYFP:

A 732 bp of EYFP fragment from pd2EYFP-1 (Clontech Cat.#6912-1) is ligated to a 3.5 kb fragment from pCRE-d2EGFP (Clontech Cat #6034-1). Both fragments are excised from the two vectors by a common restriction enzyme digestion.

### Construction of HA-MC4R:

999 bp of MC4R ORF sequence is first inserted into pCMV-HA vector (Clontech Cat#6003-1) with cloning sites EcoRI/XhoI, then the fusion fragment of HA-MC4R is further cloned into pcDNA3.1/Zeo (Invitrogen Cat.#V86520) with the cloning sites HindIIIXhoI.

### Cell line establishment:

U2OS cells are transfected with HA-MC4R using standard procedure for Fugene6 transfection. Cells are put under zeocin selection for 4 weeks to obtain a cell line stably expressing the HA-MC4R.
The U2OS cell line stably expressing the human HA-MC4R (melanocortin4 receptor-GFP) is further transfected with Cre-YFP the day before culturing them on PEGA beads displaying adhesion peptide and respectively 1)Negative control (PEGA beads with adhesion peptide, but no library compound), 2)Positive control (PEGA beads of example 2) and 3)Library compounds. The three cultures are handled separately in each their culture flask.

### Bead/cell preparation:

Cells are trypsinized and mixed with beads in growth medium (DMEM containing 10% FCS, in the proportion 4000 cells/bead and app. 50ml growth medium/5000 beads.
1) Positive control: 50ml Growth medium + 5000 positive control beads + 2x10E7 cells.
2) Negative control: 50ml Growth medium + 5000 negative control beads + 2x10E7cells.
3) Screening library (eg. 100.000 compounds): 1000ml Growth medium + 100.000 library beads + 4x10E8 cells
The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Bead sorting for Cre-YFP response:

A (Fluorescence Activated Bead Sorter (FABS) equipped with a multiline Argon laser adjusted to the 514 nm excitation line and 528-572 nm emission filter is used to identify and isolate positive hit beads.
The FABS is calibrated to identify and isolate positive hit beads (increased YFP fluorescence) by determining the dynamic range of the assay using positive control beads as Smax (maximum response) and negative control beads as Smin (minimum response). A cut off at 30% response compared to negative control beads is set as threshold for a positive hit bead.
Positive hits are isolated into a 1 well Nunc chamber and are hereafter ready to test for receptor internalisation.

### HA-MC4R internalisation: Microscope analysis

Beads isolated as positive hits in Cre-YFP transcription reporter assay by FABS are treated with Triton-x to permeabilize cells followed by incubation with HA-tag polyclonal antibody followed by staining with appropriate TRITC conjugated secondary antibody. A Nunc 1 well chamber holding the labelled beads are placed on an imaging microscope (Zeiss Axiovert 200M) equipped with filters allowing separation of YFP and TRITC. Further more the microscope is equipped with a micromanipulator (Eppendorf Transferman NK2)) capable of picking out single beads. Using 20x objective the chamber is scanned for positive HA- internalisation (appear as intracellular spots as compared to membrane distribution in non positive HA-MC4Rintemalisation and such hit beads are picked out for compound structure elucidation.

A multiplexed screening like this is expected to give very low rate of false positive hits since hits picked out for structure elucidation is giving rise to both specific receptor activation (observed as internalisation of receptor) as well as a functional response (observed as transcription of Cre-YFP construct).

### Example 10:

### Gs coupled receptor (MC4R): Agonist assay (Cre-YFP reporter and HA-MC4R internalization: FABS+FABS)

### Construction of pCRE-d2EYFP:

A 732 bp of EYFP fragment from pd2EYFP-1 (Clontech Cast.#6912-1) is ligated to a 3.5 kb fragment from pCRE-d2EGFP (Clontech Cat #6034-1). Both fragments are excised from the two vectors by a common restriction enzyme digestion.

### Construction of HA-MC4R:

999 bp of (MC4R ORF sequence is first inserted into pCMV-HA vector (Clontech Cat#6003-1) with cloning sites EcoRI/XhoI, then the fusion fragment of HA-MC4R is further cloned into pcDNA3.1/Zeo (Invitrigen Cat.#V86520) with the cloning sites HindIII/XhoI.

### Cell line establishment:

U2OS cells are transfected with HA-MC4R using standard procedure for Fugene6 transfection. Cells are put under zeocin selection for 4 weeks to obtain a cell line stably expressing the HA-MC4R.
The U2OS cell line stably expressing the human HA-MC4R (HA-melanocortin4 receptor) is further transfected with Cre-YFP the day before culturing them on PEGA beads displaying adhesion peptide and respectively 1)Negative control (PEGA beads with adhesion peptide, but no library compound), 2)Positive control (PEGA beads of example 2) and 3)Library compounds. The three cultures are handled separately in each their culture flask.

### Bead/cell preparation:

Cells are trypsinized and mixed with beads in growth medium (DMEM containing 10% FCS, in the proportion 4000 cells/bead and app. 50ml growth medium/5000 beads.
1) Positive control: 50ml Growth medium + 5000 positive control beads + 2x10E7 cells.
2) Negative control: 50ml Growth medium + 5000 negative control beads + 2x10E7 cells.
3) Screening library (eg. 100.000 compounds): 1000my Growth medium + 100.000 library beads + 4x10E8 cells
The three culture flasks are placed on a Magnetic stirring platform (Techne) designed for cell culture in suspension and incubated at 37°, 5% CO2 for 16 - 24 hrs using spinning interval 30 rpm, 3 min stirring, 10main pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Bead sorting for Cre-YFP response:

A Fluorescence Activated Bead Sorter (FABS) equipped with 514 nm laser excitation line and 528-572 nm emission filter is used to identify and isolate positive hit beads.
The FABS is calibrated to identify and isolate positive hit beads (increased YFP fluorescence) by determining the dynamic range of the assay using positive control beads as Smax (maximum response) and negative control beads as Smin (minimum response). A cut off, at 30% response compared to negative control beads is set as threshold for a positive hit bead.
Positive hits are isolated into a 10 ml tube and are hereafter ready to test for receptor internalisation.

### HA-MC4R internalisation: FABS analysis

A Fluorescence Activated Bead Sorter (FABS) equipped with 568 nm laser line excitation and 584-640 nm emission filter is used to identify and isolate positive hit beads.

Beads isolated as positive hits in Cre-YFP transcription reporter assay by FABS as well as positive and negative control beads are incubated with HA-tag polyclonal antibody followed by staining with appropriate TRITC conjugated secondary antibody.
The FABS is calibrated to identify and isolate positive hit beads by determining the dynamic range of the assay using positive control beads as Smax (maximum response) and negative control beads as Smin (minimum response). A cut off at 30% response compared to negative control beads is set as threshold for a positive hit bead. Positive hits are giving less TRITC fluorescence than negative hits caused by receptor internalization resulting in inability of the TRITC conjugated sec. antibody to reach the HA-tag (no permeabilization of the plasma membrane).
Positive hits are separated into each well of a 96 well plate and are hereafter ready for compound elucidation, re-synthesis and re-test as well as test for effects in other assays.

A multiplexed screening like this is expected to give very low rate of false Positive hits since hits picked out for structure elucidation is giving rise to both specific receptor activation as well as a functional response

### Example 11

### Gs coupled receptor(β2AR): Antagonist assay (Cre-reporter)

### Cre-GFP:

Cre-GFP (c-AMP Response Element-Green Fluorescent Protein) commercially available from clontech (pCre-d2eGFP) The vector contains three copies of Cre-binding sequence fused to a TATA-like promoter. The vector is holding a neomycin resistance gene. A map of the vector is shown in figure 3. β2 adrenergic receptor

### (β2AR):

A 1776 bp cDNA fragment containing β2AR ORF sequence is PCR-amplified from human kidney and fetal brain cDNA libraries( Clontech Cat#639305, 6393029) using primers designed from β2AR mRNA sequence (accession # NM_000024), and cloned into pCR-XL-TOPO vector (invitrogen). A 1274 bp of β2AR gene containing a kozak sequence and a stop codon is further cloned into pcDNA3.1/zeo(+) vector (invitrogen) with the restriction sites Hindlll/Xhol. The β2AR gene is sequencing confirmed.

### Cell line establishment:

U2OS cells are transfected with β2AR using standard procedure for Fugene6 transfection. Cells are put under zeocin selection for 4 weeks to obtain a cell line stably expressing the β2AR.
The U2OS cell line stably expressing the human β2AR is further transfected with Cre-GFP the day before culturing them on PEGA beads displaying adhesion peptide and respectively 1)Negative control (PEGA beads with adhesion peptide, but no library compound), 2)Positive control (PEGA beads displaying adhesion peptide and isoproterenol) and 3)Library compounds. The three cultures are handled separately in each their culture flask.

### Bead/cell preparation:

Cells are trypsinized and mixed with beads in growth medium (DMEM containing 10% FCS), added Isoproterenol 10uM, in the proportion 4000 cells/bead and app. 50ml growth medium w. isoproterenol 10uM/5000 beads.
1) Positive control: 50ml Growth medium w. proterenol 10uM + 5000 positive control beads + 2x10E7 cells.
2) Negative control: 50ml Growth medium w. proterenol 10uM + 5000 Negative control beads + 2x10E7 cells.
3) Screening library (eg. 100.000 compounds): 1000ml Growth medium w. proterenol 10uM + 100.000 library beads + 4x10E8 cells
The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Ceiis are now preserved and fixed to the beads

### Bead sorting:

A Fluorescence Activated Bead Sorter (FABS) equipped with 488 nm laser excitation line and 500-550 nm emission filter is used to identify and isolate positive hit beads (=inhibition of isoproterenol induced Cre-GFP transcription (= decreased fluorescence compared to negative control).
The FABS is calibrated to identify and isolate positive hit beads by determining the dynamic range of the assay using positive control beads as Smax (maximum inhibition = minimal fluorescence) and negative control beads as Smin (minimum inhibition = maximal fluorescence). A cut off at 30% inhibition compared to negative control beads is set as threshold for a positive hit bead.
Positive hits are separated into each their well of a 96 well plate and are hereafter ready for compound elucidation, re-synthesis and re-test as well as test for effects in other assays.

### Example 12:

### Gi coupled receptor (CCR5): Agonist assay (Cre-GFP reporter)

### Cre-GFP:

Cre-GFP is commercially available from clontech (pCre-d2eGFP) The vector contains three copies of Cre-binding sequence fused to a TATA-like promoter. The vector is holding a neomycin resistance gene. A map of the vector is shown in figure 3. C-C Chemokine Receptor5 (CCR5):

### Accession no. AAB57793

U2OS cells are transfected with CCR5 using standard procedure for Fugene6 transfection. Cells are put under zeocin selection for 4 weeks to obtain a cell line stably expressing the CCR5.
The U2OS cell line stably expressing the human CCR5 is further transfected with Cre-GFP the day before culturing them on PEGA beads displaying adhesion peptide and respectively 1)Negative control (PEGA beads with adhesion peptide, but no library compound), 2)Positive control (PEGA beads with adhesion peptide and RANTES) and 3)Library compounds. The three cultures are handled separately in each their culture flask.

### Bead/cell preparation:

Cells are trypsinized and mixed with beads in DMEM containing 10% FCS, 10uM forskolin and 500uM IBMX, in the proportion 4000 cells/bead and app. 50ml DMEM/5000 beads.
1) Positive control: 50ml DMEM + 5000 positive control beads + 2x10E7 cells.
2) Negative control: 50ml DMEM + 5000 negative control beads + 2x10E7 cells.
3) Screening library (eg. 100.000 compounds): 1000ml DMEM + 100.000 library beads + 4x10E8 cells
The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Bead sorting:

A Fluorescence Activated Bead Sorter (FABS) equipped with 488 nm laser excitation line and 500-550 nm emission filter and sorting capability into 96 well plate is used to identify and isolate positive hit beads.
The FABS is calibrated to identify and isolate positive hit beads (decreased GFP fluorescence compared to negative control) by determining the dynamic range of the assay using positive control beads (RANTES) as Smax (maximum response = minimal fluorescence) and negative control beads as Smin (minimum response = maximal fluorescence). A cut off at 30% response compared to negative control beads is set as threshold for a positive hit bead.
Positive hits are separated into each their well of a 96 well plate and are hereafter ready for compound elucidation, re-synthesis and re-test as well as test for effects in other assays.

### Example 13:

### Gq coupled receptor (Muscarinic M1): Antagonist assay (Ca++ mobilization using Fluo-4)

### Ca++ antagonist assay:

This assay is designed to identify muscarinic M1 antagonist compounds. The read out is changes in intracellular Ca++ conc. detected using the Fluo-4 probe from Molecular probes (see description elsewhere). Positive hits are compounds that inhibit Carbacol (muscarinic M1 agonist) induced increase in intracellular Ca++.

U2OS cells are transfected with Muscarinic M1 receptor using standard procedure for Fugene6 transfection. Cells are put under zeocin selection for 4 weeks to obtain a cell line stably expressing the Muscarinic M1 receptor.
U2OS cells expressing the Muscarinic M1 receptor are culturedon PEGA beads displaying adhesion peptide and respectively 1)Negative control (Beads comprising only cell adhesion compound), 2)Positive control (beads comprising cell adhesion compound and Atropine) and 3)Library compounds. The three cultures are handled separately in each their culture flask.

### Bead/cell preparation:

Cells are trypsinized and mixed with beads in DMEM containing 10% FCS in the proportion 4000 cells/bead and app. 50ml growth medium/5000 beads.
1) Positive control: 50ml Growth medium + 5000 positive control beads + 2x1 0E7 cells.
2) Negative control: 50ml Growth medium + 5000 negative control beads + 2x10E7 cells.
3) Screening library (eg. 100.000 compounds): 1000ml Growth medium + 100.000 library beads + 4x10E8 cells

The three culture flasks are placed on a Magnetic stirring platform (Techne) designed for cell culture in suspension and incubated at 37°, 5% CO2 for 16 - 24 hrs using spinning interval 30 rpm-, 3 min stirring, 10min pause.

Measurement of changes in the cytoplasmic free calcium concentration [Ca²⁺]ᵢ Beads, now covered with cells, are allowed to sediment for 10 min (no centrifugation needed) and the growth medium is removed using a 50ml pipette. 10ml Krebs Ringer buffer (KRW :KrebsRingerWollheim, pH 7.4: NaCl 0.14 M, KCL 3.6 mM, NaH₂PO₄,H₂O 0.5 mM, MgSPO₄, 7H₂O 0.5.mM, NaHCO₃,2H₂O 1.5 mM, D-Glucose 6 mM, CaCl₂ 1.5 mM, HEPES 10 mM) added 1uM Fluo-4 (Molecular Probes F-14201) + 0.02% Pluronic (Molecular Probes F-127) per 5000 beads is added, mixed gently and cells/beads are incubated at 37°c for 30 min. Beads are hereafter washed w. 10ml KRW/5000 beads x3 by allowing sedimentation for 10 min between each wash. The Fluo-4 loaded cells are now ready for detection of changes in [Ca²⁺]ᵢ The fluorescence is monitored in either a Fluorescence Activated Bead Sorter (FABS) (COPAS from Union Biometrica, US) that is equipped with multiple laser excitation lines (476 nm, 483 nm, 488 nm, 496 nm, 514 nm, 520 nm, 568 nm, 647 nm, 676 nm) or a fluorescence plate-reader (Polarstar Optima from BMG Labtech, Germany) that is equipped with a flash Xenon blitz lamp. Fluo4 fluorescence is detected in FABS by exciting with 488 nm and collecting the emitted light on to a PMT through a 530±30 nm emission filter, and on the plate-reader the cells are excited through a 490±5 nm excitation filter and the emission collected through a 510±5 nm emission filter. For calculation of the exact [Ca²⁺]ᵢ the fluorescence intensity is converted to [Ca²⁺]ᵢ by using the equation [Ca²⁺]ᵢ=K_{D}[(F-Fₘᵢₙ)/(Fₘₐₓ-F)] where the dissociation constant K_{D} is 345 nM, F is fluorescence intensity, Fₘᵢₙ is total fluorescence in the absence of Ca²⁺ and Fₘₐₓ is total fluorescence when Fluo3 is saturated with Ca²⁺. To obtain Fₘᵢₙ the cells are pre-incubated in a calcium low buffer (PH 7.4: NaCl 0.14 M, KCL 3.6 mM, NaH₂PO₄,H₂O 0.5 mM, MgSO₄, 7H₂O 0.5.mM, NaHCO₃,2H₂O 1.5 mM, D-Glucose 6 mM, EGTA 1.5 mM, HEPES 10 mM) and is challenged with 1 uM ionomycin immediately before the fluorescence detection. Similarly Fₘₐₓ is obtained by suspending the cells in a calcium saturated buffer (pH 7.4: NaCl 0.14 M, KCL 3.6 mM, NaH₂PO₄,H₂O 0.5 mM, MgSO₄, 7H₂O 0.5.mM, NaHCO₃,2H₂O 1.5 mM, D-Glucose 6 mM, CaCl₂ 1.5 mM, HEPES 10 mM) and challenged with 1 uM ionomycin immediately before detection.
In several of our screening assay we do not use exact ion [Ca²⁺]ᵢ, but express the response of screening compounds as relative to control compounds (see below).

### Bead sorting for Fluo-4 signal:

A Fluorescence Activated Bead Sorter (FABS) equipped with 488 nm laser excitation line and 528-572 nm emission filter and injection capability is used to identify and isolate positive hit beads (=inhibition of Carbachol induced Ca++ response = decreased fluorescence compared to negative control).
The FABS is calibrated to identify and isolate positive hit beads by determining the dynamic range of the assay using positive control beads as Smax (maximum inhibition = minimal fluorescence) and negative control beads as Smin (minimum inhibition = maximal fluorescence). Carbacol 1uM is injected into the flow steam resulting in an increase in fluorescence for negative control beads and an unchanged or minor increase in fluorescence for positive control beads. A cut off at 30% inhibition compared to negative control beads is set as threshold for a positive hit bead. Positive hit beads may preferably be identified using a plate reader essentially as described in Example 13a herein below.

Positive hits are separated into each their well of a 96 well plate and are hereafter ready for compound elucidation, re-synthesis and re-test as well as test for effects in other assays.

### Example 13a:

### Gq coupled receptor (Muscarinic M1): Antagonist assay (Ca++ mobilization using Fluo-4)

### Ca++ antagonist assay:

This assay is designed to identify muscarinic M1 antagonistic compounds. The readout is changes in intracellular Ca++ conc. detected using the Fluo-4 probe from Molecular probes. Positive hits are compounds that inhibit Carbacol (muscarinic M1 agonist) induced increase in intracellular Ca++.
For calculation of the exact [Ca²⁺]ᵢ the fluorescence intensity is converted to [Ca²⁺]ᵢ by using the equation [Ca²⁺]ᵢ=K_{D}[(F-Fₘᵢₙ)/(Fₘₐₓ-F)] where the dissociation constant K_{D} is 345 nM, F is fluorescence intensity, Fₘᵢₙ is total fluorescence in the absence of Ca²⁺ and Fₘₐₓ is total fluorescence when Fluo4 is saturated with Ca²⁺. To obtain Fₘᵢₙ the cells are pre-incubated in a calcium low buffer (pH 7.4: NaCl 0.14 M, KCL 3.6 mM, NaH₂PO₄,H₂O 0.5 mM, MgSO₄, 7H₂O 0.5.mM, NaHCO₃, 2H₂O 1.5 mM, D-Glucose 6 mM, EGTA 1.5 mM, HEPES 10 mM, probenecid 2mM) and is challenged with 1 uM ionomycin immediately before the fluorescence detection. Similarly Fₘₐₓ is obtained by suspending the cells in a calcium saturated buffer (pH 7.4: NaCl 0.14 M, KCL 3.6 mM, NaH₂PO₄,H₂O 0.5 mM, MgSO₄, 7H₂O 0.5.mM, NaHCO₃,2H₂O 1.5 mM. D-Glucose 6 mM, CaCl₂ 1.5 mM, HEPES 10 mM, probenecid 2mM) and challenged with 1 uM ionomycin immediately before detection.

In general it is not required to use exact ion [Ca²⁺]ᵢ. Rather, the response of screening compounds may be expressed as relative to control compounds (see below).

### Cell line establishment:

BHK cells are transfected with the muscarinic M1 receptor using standard procedure for Fugene6 transfection. Cells are put under G418 selection for 4 weeks to obtain a cell line stably expressing the muscarinic M1 receptor.

### Cell/bead preparation:

BHK cells expressing the Muscarinic M1 receptor are cultured on Negative control beads (prepared as described in example 1) using the following procedure:
- Trypsinize cells and adjust cell conc. to 1x10E6/ml Hams F12 growth medium
- Add 500 beads in 500ul Hams to a 14ml Nunc tube
- Add 2500ul cell suspension 1x10E6/ml Hams w. 5% FCS
- Leave tube vertically in incubator (37 degrees, 5% CO2) for 16-24hrs - rock tube gently every 15min for the first hour
- Remove medium. Wash loose cells away by gently adding and removing 4ml Hams F12 twice (Turn the tube upside down and back again - as soon as beads have sedimented suck away medium)
- Add 2ml Hams F12 w. FCS 10%
- Incubate o/n at 37 degrees, 10% CO2

Measurement of changes in the cytoplasmic free calcium concentration [Ca²⁺]ᵢ:
- Beads, now covered with cells, are allowed to sediment for 10 min (no centrifugation needed) and the growth medium is removed using a pipette.
- 2ml Krebs Ringer buffer (KRW ;Krebs RingerWollheim, pH 7.4: NaCl 0.14 M, KCL 3.6 mM, NaH₂PO₄,H₂O 0.5 mM, MgSO₄, 7H₂O 0.5.mM, NaHCO₃,2H₂O 1.5 mM, D-Glucose 6 mM, CaCl₂ 1.5 mM, HEPES 10 mM, probenecid 2mM) added 1 uM Fluo-4 (Molecular Probes F-14201) + 0.02% Pluronic (Molecular Probes F-127) per 500 beads is added, mixed gently and cells/beads are incubated at 37°c for 30 min.
- Beads are hereafter washed w. 5ml KRW/500 beads x2 by allowing sedimentation for 10 min between each wash. The Fluo-4 loaded cells are now ready for detection of changes in [Ca²⁺]ᵢ.

The fluorescence is monitored in a fluorescence plate-reader (Polarstar Optima from BMG Labtech, Germany) equipped with a flash Xenon blitz lamp and 490±5 nm excitation filter and 510±5 nm emission filter. The plate reader is furthermore equipped with a dispenser allowing injection of agonist.
The measurement can equally well be performed on a microscope equipped with a fluorescence illuminator (E.g. HBO 100W lamp) and 480/30 nm excitation filter, 505nm LP dicroic mirror and 535/40nm emission filter.
Approx. 50 beads covered with BHK cells expressing the M1 receptor now loaded with the fluorescent Ca++ indicator Fluo-4 are pipetted into each well of a 96 well plate. The plate is placed in the fluorescence plate reader and Carbacol 1uM is injected resulting in an increase in fluorescence for negative control beads.

This assay can be used to screen for Carbacol inhibitors. Positive hits are compounds that inhibit the Carbacol (muscarinic M1 agonist)

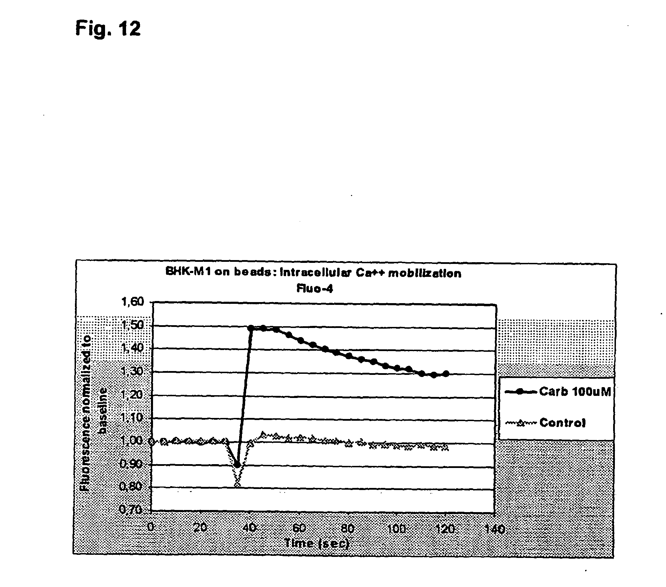

Fig. 12 shows the ilntracellular Ca++ mobilization in BHK-M1 cells on beads treated with Carbamylcholin 100uM versus control (buffer).

### Example 14

**Gs coupled receptor (MC4R): Agonist assay** (Cre-GFP reporter assay detected with fluorecence plate reader or fluorescence imaging equipment)

### Cre-GFP:

Cre-GFP is commercially available from clontech (pCre-d2eGFP) The vector contains three copies of Cre-binding sequence fused to a TATA-like promoter. The vector is holding a neomycin resistance gene. A map of the vector is shown in figure 3.

### MC4R:

PCR amplified MC4R encoding DNA is introduced into the gateway Entry Vector (pENTR) by topoisomarase-mediated ligation. The DNA is subsequently recombined into Destination Vector pDEST12.2. (pDEST12.2MC4R)

### Cell line establishment:

U2OS cells are transfected with pDEST1.2.2MC4R using standard procedure for Fugene6 transfection. Cells are put under G418 selection for 4 weeks to obtain a cell line stably expressing the MC4R.
The U2OS cell line stably expressing the human MC4R (melanocortin4 receptor) is further transfected with Cre-GFP the day before culturing them on PEGA beads displaying adhesion peptide and respectively 1)Negative control (PEGA beads with adhesion peptide, but no library compound), 2)Positive control (PEGA beads of example 2) and 3)Library compounds. The three cultures are handled separately in each their culture flask.

### Bead/cell preparation:

Cells are trypsinized and mixed with the PEGA beads in growth medium (DMEM containing 10% FCS, in the proportion 4000 cells/bead and app. 50ml growth medium/5000 beads.
1) Positive control: 50ml Growth medium + 5000 positive control beads + 2x10E7 cells.
2) Negative control: 50ml Growth medium + 5000 negative control beads + 2x10E7 cells.
3) Screening library (eg. 100.000 compounds): 1000ml Growth medium + 100.000 library beads + 4x10E8 cells
The three culture flasks are placed on a Magnetic stirring platform (Techne) designed for cell culture in suspension and incubated at 37°, 5% CO2 for 16 - 24 hrs using spinning interval 30 rpm, 3 min stirring, 10min pause.
Beads, now covered with cells, are allowed to sediment for 10 min (no centrifugation needed) and the growth medium is removed using a 50ml pipette. 10ml 99% EtOH per 5000 beads is added, mixed gently and left for 15 min. Beads are washed w. 10ml PBS/5000 beads x3 by allowing sedimentation for 10 min between each wash. Cells are now preserved and fixed to the beads

### Plate reader assay:

Control beads as well as library beads are seeded in 384 well black plates (eg. Nunc) with clear bottom app. 20 beads per well. Positive and negative controls are placed in dedicated wells in 2 times 4 replicates in each end of the plate. Negative control = 20 negative control beads, positive control = one positive control bead + 19 negative control beads. The plates are measured in a fluorescence plate reader (PolarStar Optima from BMG) using 490 +-6nm excitation filter and 510 +-5nm-emission filter. Positive control wells are used to determine Smax (maximum response) = 100% activity and negative control wells to determine Smin (minimum response) = 0% activity. Beads from wells showing activity > 30% are collected in a tube for reseeding in a new 384 well plate, this time having one bead per well. Smin = one negative control bead and Smax = one positive control bead. Read plates in plate reader and identify hits beads using same procedure as described above.

### Image acquisition analysis:

Control beads as well as library beads are seeded in 384 well black plates (eg. Nunc) with clear bottom app. 20 beads per well. Positive and negative control beads are placed in dedicated wells in 2 times 4 replicates in each end of the plate. Negative control = 20 negative control beads, positive control = one positive control bead + 19 negative control beads. Plates are placed on a microscope (Zeiss Axiovert 200M) equipped with filters allowing fluorescence imaging of eGFP (excitation: 490nm, emission: 510nm), 10x objective and motorized stage. One image is acquired for each well followed by image analysis (Metamorph) for identification of hit beads (green). Beads from hit wells are seeded in a new 384 well plate this time having one bead per well. Smin = one negative control bead and Smax = one positive_control bead. Image acquisition and analysis described above is repeated and final hit beads are identified.

Alternatively, approximately 5000 beads are seeded into Lab-Tech Chambered Coverglass System (#155361; Nalge Nunc INternational), imaging acquisition analysis is performed using the fluorescence equipment described above, and individual beads that display the required fluorescence properties are Isolated using a micromanipulator system (Eppendorf Injectman NK). This method is preferred.

### Example 14a:

### Gs coupled receptor (MC4R) agonist screening (Cre-YFP reporter assay detected using a Fluorescence Microscope)

### pCre-d2YFP:

A 732 bp of EYFP fragment from pd2EYFP-1 (Clontech Cat.#6912-1) is ligated to a 3.5 kb fragment from pCRE-d2EGFP (Clontech Cat #6034-1). Both fragments are excised from the two vectors by a common restriction enzyme digestion.

### MC4R:

PCR amplified MC4R encoding DNA is introduced into the gateway Entry Vector (pENTR) by topoisomarase-mediated ligation. The DNA is subsequently recombined into Destination Vector pDEST12.2. (pDEST12.2MC4R)

### Cell line establishment:

Hek293 cells are co-transfected with pDEST1.2.2MC4R and Cre-YFP (using standard procedure for Fugene6 transfection) and cells are cultured on PEGA beads displaying adhesion peptide and respectively 1)Negative control (PEGA beads with adhesion peptide) and 2)Positive control (PEGA beads of example 2) by mixing appr. 400 beads with 400.000 cells in 1ml Hams F12 medium containing 10% FCS in a 1.8ml Eppendorf tube. Tubes are shaked gently every 15 min for 2 hrs. Cells/beads are incubated in a CO2 incubator (5% CO2,m 37 degrees) for 20 hrs. The level of CRE-YFP expression was detected using a Zeiss Axiovert 200M microscope equipped with appropriate filters for YFP detection (Excitation 500/20 nm, Dicroic 515 LP EM 535/30).

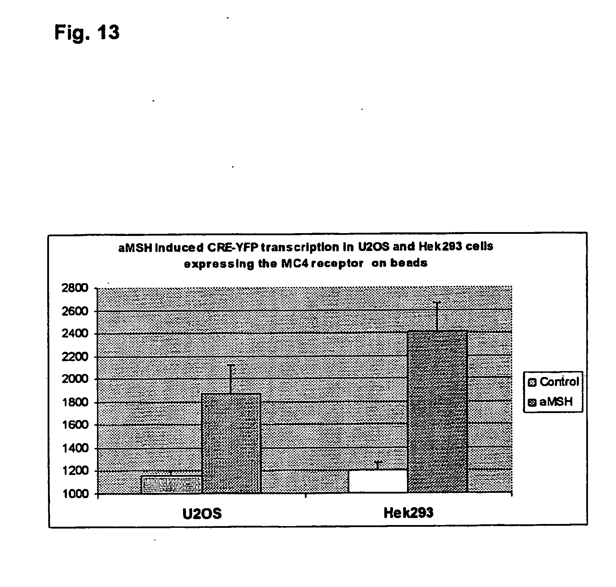

Higher signal was observed for the Hek293 cells compared to the U2OS, why Hek293 were used for further experiments (see fig. 13).

### Library screening:

### Synthesis of Library- and control beads:

Two libraries were synthesized according to examples 6a and 6b. Control beads were synthesized as described in example 5a section "Synthesis of adhesion peptide".

### Cell line establishment:

Hek293 cells were co-transfected with pDEST1.2.2MC4R and pCRE-d2EYFP and put under G418 selection for 3 weeks. Hereafter cells were FACSorted (Fluorescence Activated Bead sorted) for high YFP expression after stimulation with aMSH 100nM and 0.4uM TSA (Tricostatin A) for 20 hrs. Cells were propagated and subcultured for 2 month and FACSorted again for high aMSH/TSA induced YFP expression.

### Cell/bead preparation:

Cells were cultured on respectively 1) Negative control beads (prepared as described in example 1), 2) Positive control beads (prepared as described in example 2) and Library beads (prepared as described in example 6b ). Each batch of beads was handled separately.
Cells were trypsinized and mixed with Negative control beads/Positive control beads/Library beads in growth medium (Hams F12 containing 5% FCS):

### Control beads

- Add 500 beads in 500ul Hams to a 14ml Nunc tube
- Add 2500ul cell suspension 1x10E6/ml Hams w. 5% FCS
- Leave tube vertically in incubator (37 degrees, 5% CO2) for 16-24hrs - rock tube gently every 15min for the first hour
- Remove medium. Wash loose cells away by gently adding and removing 4ml Hams x2 (Turn the tube upside down and back again - as soon as beads have sedimented suck away medium)
- Add 2ml Hams w. FCS 5% and TSA (Tricostatin A) 0.4uM
- Incubate o/n at 37 degrees, 5% CO2
- Decant beads to a 1well Lab-Tek Chambered Coverglass (#155361)

### Library beads

- Add 10.000 beads in 5ml Hams to a 50ml Nunc tube
- Add 25ml cell suspension 2x10E6/ml Hams w. 5% FCS
- Leave tube vertically in incubator (37 degrees, 5% CO2) for 16-24hrs - rock tube gently every 15min for the first hour
- Remove medium. Wash ioose cells away by gentry adding and removing 25ml Hams x2 (Turn the tube upside down and back again - as soon as beads have sedimented suck away medium)
- Add 25ml Hams w. FCS 5% and TSA (Tricostatin A) 0.4uM
- Incubate o/n at 37 degrees, 5% CO2
- Decant beads to 2x 1well Lab-Tek Chambered Coverglass (#l 55361)

### Hit identification and isolation:

The LabTek one well chambered coverglass was placed on a Zeiss Axiovert 200 fluorescence microscope equipped with filters optimal for YFP fluorescence. The microscope was further more equipped with a micromanipulator (Eppendorf Transferman NK2)) capable of picking out single beads. Using 10x objective chambers were scanned for positive hit beads, which appeared as green dotted beads caused by cells expressing CRE-YFP. Positive and negative control beads were used to set cut off for positive hit beads. Such hit beads were picked out using the micromanipulator for further test in specificity assay (Receptor internalization) before final structure elucidation.
Microscope detection was prefered for this screening campaign. The throughput of this microscope-based method was app. 40.000 beads per day. 90,000 beads were screened in totally. 35 hits were identified and isolated, 15 were structure elucidated and resynthesized.

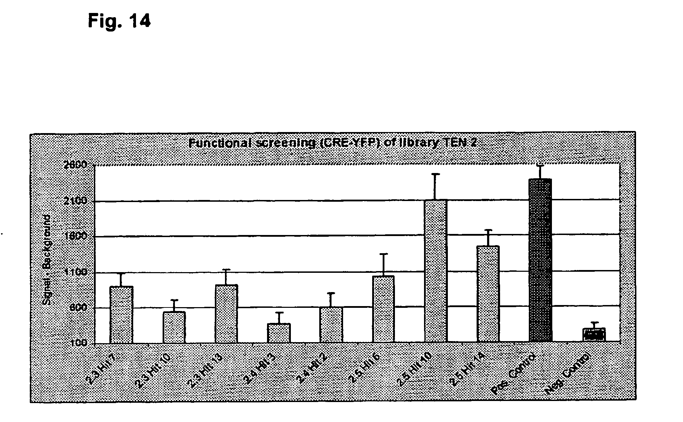

Signal obtained from a sub-fraction of identified hits is shown in the graph of fig. 14.

### Structure elucidation and resynthesis:

Hit beads are structure elucidated using the method described in example 15. As an illustrative example identification of hit designated TEN-636-33-26 is described om example 15b. Other hits may be identified using a similar method.

### Specificity screening:

Hits are tested for MC4 receptor specificity using a Hek 293 cell line stably expressing the MC4R-GFP as described in example 7a under cell line establishment.
Cells are seeded in Hams F12 w. 10% FCS in an 8 well Lab-tek Chambered Coverglass to give 75% confluency 24 hrs after seeding. Cells are challenged with hit compounds for 30 min at 37 degrees. The chamber is placed on a Zeiss Axiovert 200M equipped with filters suited for GFP fluorescence and cells are inspected for MC4R-GFP internalization using image acquisition (20x) followed by image analysis. Negative and positive controls are Hams F12 respectively αMSH 100nM.

### Selectivity screening (β2-adrenergic receptor (β2-AR - GFP internalization:

Hits are further tested for receptor selectivity using a Hek293 cell line stably expressing the β2-adrenergic receptor fused to GFP (β2-AR - GFP). Cells are tested as described above for "Specificity determination".
MC4R specific hits are those showing a positive response in CRE-YFP reporter assay, a positive response in the MC4R-GFP internalization assay (specificity) and a negative response in β2-AR - GFP internalization assay (selectivity).

### Example 15

### Identification of compound

Once a resin bead is selected, the library compound comprised within the bead is identified. The selected, single resin bead is treated with 0.1 M aqueous NaOH (10 µL) in a 0.5 mL Eppendorf tube overnight, then diluted with CH₃CN (20 µL), before filtering the solution, thereby providing a sample for ES MSMS analysis on a MicroMass QTOF Global Ultima mass spectrometer (mobile phase 50% CH₃CN (aq), 0.1 µL/min) employing a linear ramping of the collision energy. The spectra are analyzed by generating the exact mass differences between fragment ions and tabulated to provide the fragmentation pathway and from that the structure of the compound released from the selected bead is elucidated.

### Example 15b. Identification of hit TEN-636-33-26 (From Ibrary prepared as described in example 6b) from GPCR assay

Hits selected in the GPCR assay call for unambiguous structure assignment. High purity of compounds generated on the solid support during library synthesis are preferred for single bead analysis. For example for the chemistry utilized in Example 6a-b (indsæt evt. ref til scaffold-patent), it has demonstrated that quantities of material cleaved from single synthesis and library beads can be analyzed and identified by QTOF MSMS (ES). The signal arising from the molecular ion (M+H) is first detected, and MSMS is subsequently carried out to obtain a specific fragmentation pattern.

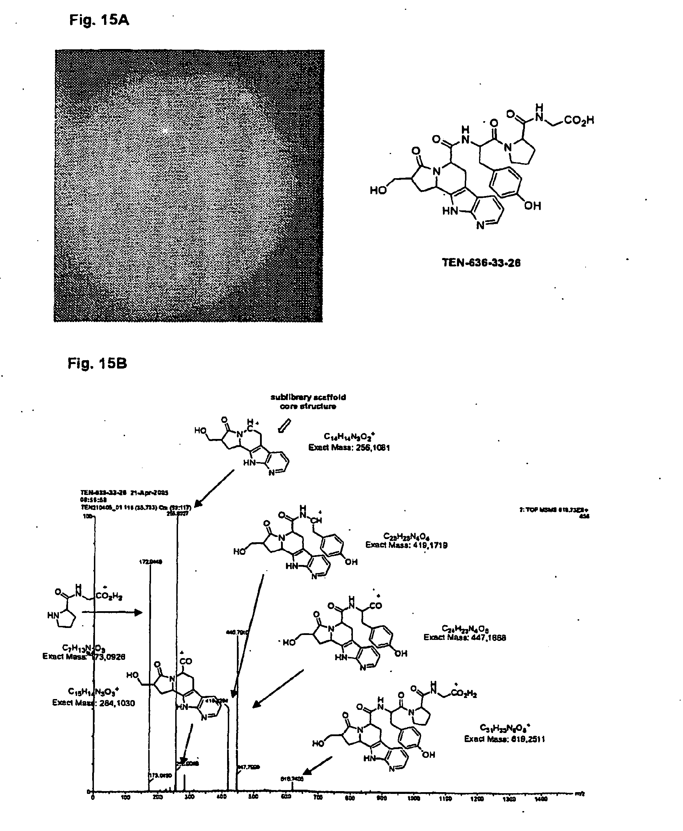

A hit (bead) selected in the assay (Figure 15a) is carefully washed with 10% TFA (aq) and MiliQ water by successive rounds of decantation. The bead is placed in a 1.5 mL Eppendorf tube and treated with 0.1 M NaOH (3 mL) prepared in the usual way from solid NaOH pellets and MiliQ water. Hydrolysis is effected during 2h to 24h in a sealed tube. After hydrolysis, 0.1 M HCl (3 mL) is added to neutralize the alkaline cleavage mixture, followed by addition of MiliQ water (40 µL). Prior to loading, the wells of the OASIS elution plate was carefully washed with 3 x CH₃CN:H₂O (4:1, 0.1% HCOOH), then 3 x H₂O (0.1% HCOOH), and 3 x H₂O. The selected well is loaded with the sample solution by applying gentle suction. Salts are then washed out with water (70 µL) and 0.1% HCOOH (70 mL), before eluting the compound with CH₃CN:H₂O (4:1, 0.1% HCOOH) (200 µL). The eluent is removed on the speed vac, and the resulting residue is taken up in CH₃CN:H₂O (19:1, 0.05% TFA) (50 µL) before analysis by QTOF LC/MSMS (Figure 15b).

### Identification of compound (from libraries described in examples 6a-b)

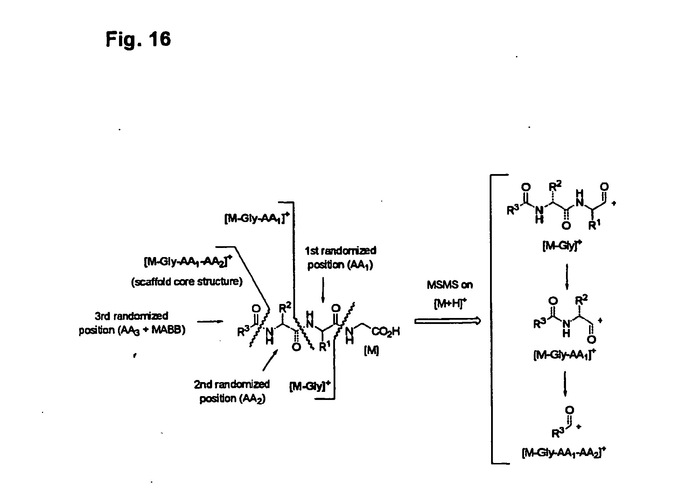

Libraries containing heterocyclic scaffolds attached to peptide sequences (see Example 6a-b) are very well applicable to single-bead MSMS analysis (material cleaved from single library beads). This compound class generally displays a high propensity to afford unique detectable fragments corresponding to the heterocyclic scaffold core structures. Recognizing this peak in MSMS analysis of the anticipated molecular ion, and relying on the general tendency of peptides to fragment at amide bonds, a predictable fragmentation pattern is emerging (See Figure 16), since each randomized position of amino acids is given by their unique masses (with the pairs of leucine/isoleucine and glutamine/lysine as the only exceptions).

### Example 16:

### Multiple GPCR receptors

### β2-adrenergic receptor (β2AR) - GFP (for internalization studies):

Hek293 cells are transfected with β2-adrenergic receptor (β2AR)-GFP using standard procedure for Fugene6 transfection. Cells are put under zeocin selection for 4 weeks to obtain a cell line stably expressing β2-adrenergic receptor (β2AR) -GFP.

### Bead/cell preparation

Hek293 cells stably expressing β2AR-GFP are seeded in a Nunc 8 well chambered coverglass in Hams F12 w. 10% FCS and incubated at 37 degrees, 5% CO₂ for 20 hrs.
Cells are stimulated with isoproterenol 100uM (positive control) and medium (negative control) for 30 min. Cells are imaged on a Zeiss Axiovert 200M fluorescence microscope equiped with optimal filters for GFP.
For negative control the β2-adrenergic receptor (β2AR) - GFP is localized in the membrane whereas for positive control β2-adrenergic receptor (β2AR) - GFP is localized in intracellular spots as an indication of receptor activation and consequently internalization.

Compounds modulating the β2AR can be identified in a similar manner by growing above-mentioned cells on resin beads comprising library compounds, such as the resin beads used in example 14a.

### Abreviations:

HGF: Hepatocyte Growth Factor
NGF: Nerve Growth Factor
PDGF: Platelet Derived Growth Factor
FGF: Fibroblast Growth Factor
EGF: epidermal Growth Factor
GH: Growth hormone
TRE: TPA Response Element
SRE: serum response element
CRE: cAMP response element
AcN: acetonitril;
Boc: *tert*-butoxycarbonyl;
Bu*^{t}*: *tert*-butyl;
DCM: dichlormethane;
DMF: dimethylformamide;
Fmoc: 9-fluorenylmethoxycarbonyl:
HMBA: 4-hydroxymethylbenzoic acid;
Q-TOF MS: quadrupole time-of-flight mass spectrometry;
Melm: *N-*methyl imidazole;
MSNT: 1-(mesitylene-2-sulphonyl)-3-nitro-1H-1,2,4-triazole;
NEM: 4-ethyl morpholine;
PEGA: polyethylene glycol-polydimethyl acrylamide resin;
Pfp: pentafluorophenyl;
Pmc: 2,2,5,7,8-pentamethylchroman-6-sulfonyl;
RP-HPLC: reversed phase high pressure liquid chromatography;
SPPS: solid phase peptide synthesis;
TBTU: *O*-(benzotriazol-1-yl-*N,N,N',N*'-tetramethyluronium tetrafluoroborate;
TFA: trifluoro acetic acid;
Thi: thienyl
Fur: furanyl
BzThi: benzothienyl

## Claims

1. A method of identifying a compound modifying at least one cellular response, wherein each cellular response is linked to different reporter systems generating detectable outputs, said method comprising the steps of:
(a) Providing multiple solid supports capable of supporting adherence and growth of cells, wherein each solid support is coupled to a member of a library of test compounds and wherein at least two solid supports comprise different library members; and
(b) Attaching cells comprising said reporter system(s) onto said solid support;
wherein
- cells are directly attached to the solid support, and/or
- at least 10 % of the solid supports comprise cell adhesion molecules as well as said library member, and cells adhere to said cell adhesion molecules; and
(c) Screening said solid supports for solid supports comprising cells meeting at least one predetermined selection criterion, wherein said selection criterion is linked directly or indirectly to said detectable output; and
(d) Selecting solid supports comprising cells meeting said at least one selection criterion; and
(e) Identifying said library member, thereby identifying a compound modifying said at least one cellular response.

2. The method according to claim 1, wherein said adherence is mediated through a cell adhesion compound coupled to said solid support, wherein said cell adhesion compound enables said solid support to support growth of cells.

3. The method according to any of claims 1 and 2, wherein the solid supports are resin beads.

4. The method according to any of claims 1 and 2, wherein the solid supports are spots or regions on a surface or a plated gel or a membrane.

5. The method according to claim 2, wherein said cell adhesion compound is a peptide with an overall positive netcharge.

6. The method according to claim 5, wherein said cell adhesion compound is selected from the group consisting of SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21. SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54. SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67. SEQ ID 68, SEQ ID 69 and SEQ ID 70.

7. The method according to claim 1, wherein said cellular response is modulation of a signal transduction pathway mediated by a cell surface molecule.

8. The method according to claim 7, wherein said cell surface molecule is a G-protein coupled receptor (GPCR).

9. The method according to claim 8, wherein said GPCR is selected from the group consisting of GPCR of table 3.

10. The method according to claim 1, wherein the cellular response is modulation of transcriptional activity.

11. The method according to claim 1, wherein the cellular response is change in the intracellular level of a compound.

12. The method according to claim 1, wherein the cellular response is relocalisation of a compound.

13. The method according to claim 1, wherein the reporter system is a system endogenous to said cells.

14. The method according to claim 1, wherein the reporter system comprises a nucleic acid comprising a nucleotide sequence encoding a detectable polypeptide operably linked to a response element, the activity of which is modulated by the cellular response.

15. The method according to any of claims 7 to 10 and 14, wherein the reporter system comprises a nucleic acid comprising a nucleotide sequence encoding a detectable polypeptide operably linked to a response element selected from the group consisting of cAMP response element (CRE) and serum response element (SRE).

16. The method according to any of claims 7 to 10 and 14, wherein the reporter system comprises a nucleic acid comprising a nucleotide sequence encoding a detectable polypeptide operably linked to transcriptional response element (TRE).

17. The method according to any of claims 1 to 16, wherein said detectable polypeptide is selected from the group consisting of fluorescent proteins and enzymes.

18. The method according to claim 1, wherein the reporter system comprises a bioluminescent moiety.

19. The method according to any of claims 1 to 18, wherein one predetermined selection criteria is a quantitative level of said bioluminiscence above or below a specific threshold.

20. The method according to any of claims 1 to 18, wherein the predetermined selection criteria is specific localisation of a fluorescent signal.

21. The method according to claim 1, wherein said cells are selected from the group consisting of mammalian cells.

22. The method according to any of claims 7 to 10 and 14 to 16, wherein the cells attached to the resin beads comprise a nucleic acid comprising a first nucleotide sequence encoding said cell surface molecule operably linked to a second nucleotide sequence not naturally associated therewith directing expression of said first sequence.

23. The method according to claim 1, wherein at least 100 resin beads comprising different library members are provided.

24. The method according to claim 1, wherein the library is selected from the group consisting of libraries of natural oligomers such as peptides, glycopeptides, lipopeptides, nucleic acids (DNA or RNA), or oligosaccharides; unnatural oligomers such as chemically modified peptides, glycopeptides, nucleic acids (DNA or RNA) or oligosaccharides; and small organic molecules.

25. The method according to claim 1, wherein the library is a library of small organic molecules..

26. The method according to claim 1, wherein compounds modifying at least two cellular responses are identified, wherein step c) involves screening said resin beads for beads comprising cells meeting at least two predetermined selection criterion, wherein each selection criterion is related to a different detectable output.

27. The method according to claim 1, wherein the resin bead comprises or consists of polyethylene glycol

28. A cell adhesion compound selected from either:
i) the group consisting of peptides of: SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID.14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 and SEQ ID 70 or
ii) a peptide comprising at least one D-form amino acid, said peptide being selected from the group consisting of:
SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 and SEQ ID 70.

29. A resin bead comprising a cell adhesion compound selected from the group consisting of SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28; SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 and SEQ ID 70.

30. The resin bead according to claim 29, wherein said resin bead comprises polyethylene glycol.

## Patentansprüche

1. Verfahren zum Identifizieren einer Verbindung, die mindestens eine zelluläre Antwort modifiziert, wobei jede zelluläre Antwort mit unterschiedlichen Reportersystemen verbunden ist, die feststellbare Ergebnisse erzeugen, wobei das Verfahren die Schritte umfasst:
(a) Bereitstellen mehrerer solider Träger, die eine Adhärenz und ein Wachstum von Zellen unterstützen können, wobei jeder solide Träger mit einem Mitglied einer Bibliothek von Testverbindungen gekoppelt vorlegt, und wobei mindestens zwei solide Träger unterschiedliche Bibliotheksmitglieder umfassen, und
(b) Anlagern von das/die Reportersystem(e) umfassende(n) Zellen auf den soliden Trägern,
wobei
- Zellen direkt auf dem soliden Träger angeheftet bzw. angelagert werden, und/oder
- mindestens 10 % der soliden Träger Zelladhäsionsmoleküle wie auch das Bibliotheksmitglied umfassen, und wobei die Zellen an den Zelladhäsionsmolekülen adhärieren;
und
(c) Durchmustern der soliden Träger auf solide Träger, die Zellen umfassen, die mindestens ein zuvor bestimmtes Auswahlkriterium erfüllen, wobei das Auswahlkriterium unmittelbar oder mittelbar mit dem feststellbaren Ergebnis verbunden ist, und
(d) Auswählen von soliden Trägern, die Zellen umfassen, die das mindestens eine Auswahlkriterium erfüllen, und
(e) Identifizieren des Bibliotheksmitglieds, wodurch eine Verbindung identifiziert wird, die die mindestens eine zelluläre Antwort modifiziert.

2. Verfahren nach Anspruch 1, wobei die Adhärenz durch eine an den soliden Träger gekoppelte Zelladhäsionsverbindung vermittelt wird, wobei die Zelladhäsionsverbindung dem soliden Träger ermöglicht, ein Wachstum von Zellen zu unterstützen.

3. Verfahren nach einem der Ansprüche 1 und 2, wobei die soliden Träger Harzkügelchen sind.

4. Verfahren nach einem der Ansprüche 1 und 2, wobei die soliden Träger Orte oder Bereiche auf einer Oberfläche oder eines plattierten Gels oder einer Membran sind.

5. Verfahren nach Anspruch 2, wobei die Zelladhäsionsverbindung ein Peptid mit einer insgesamt positiven Nettoladung ist.

6. Verfahren nach Anspruch 5, wobei die Zelladhäsionsverbindung ausgewählt ist aus der Gruppe bestehend aus SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 und SEQ ID 70.

7. Verfahren nach Anspruch 1, wobei die zelluläre Antwort in einer Modulation eines Signalübertragungsweges besteht, der durch ein Oberflächenmolekül der Zelle vermittelt wird.

8. Verfahren nach Anspruch 7, wobei das Oberflächenmolekül der Zelle ein G-Protein gekoppelter Rezeptor (GPCR) ist.

9. Verfahren nach Anspruch 8, wobei der GPCR ausgewählt ist aus der Gruppe bestehend aus Tabelle 3.

10. Verfahren nach Anspruch 1, wobei die zelluläre Antwort in einer Modulation der translationalen Aktivität besteht.

11. Verfahren nach Anspruch 1, wobei die zelluläre Antwort in einer Änderung des intrazellulären Pegels einer Verbindung besteht.

12. Verfahren nach Anspruch 1, wobei die zelluläre Antwort in einer Relokalisierung einer Verbindung besteht.

13. Verfahren nach Anspruch 1, wobei das Reportersystem ein für die Zellen endogenes System ist.

14. Verfahren nach Anspruch 1, wobei das Reportersystem eine Nukleinsäure umfasst, die eine ein erfassbares Polypeptid kodierende Nukleotidsequenz umfasst, die mit einem Response-Element funktionsbereit verbunden ist, dessen Aktivität durch die zelluläre Antwort moduliert wird.

15. Verfahren nach einem der Ansprüche 7 bis 10 und 14, wobei das Reportersystem eine Nukleinsäure umfasst, die eine ein erfassbares Polypeptid kodierende Nukleotidsequenz umfasst, die mit einem Response-Element, ausgewählt aus der Gruppe bestehend aus cAMP Response-Element (CRE) und Serum Response-Element (SRE), funktionsbereit verbunden ist.

16. Verfahren nach einem der Ansprüche 7 bis 10 und 14, wobei das Reportersystem eine Nukleinsäure umfasst, die eine ein erfassbares Polypeptid kodierende Nukleotidsequenz umfasst, die mit einem transkriptionellen Response-Element (TRE) funktionsbereit verbunden ist.

17. Verfahren nach einem der Ansprüche 1 bis 16, wobei das detektierbare Polypeptid ausgewählt ist aus der Gruppe bestehend aus fluoreszierenden Proteinen und Enzymen.

18. Verfahren nach Anspruch 1, wobei das Reportersystem einen biologisch lumineszierenden Rest umfasst.

19. Verfahren nach einem der Ansprüche 1 bis 18, wobei ein bestimmtes Auswahlkriterium in einem quantitativen Pegel der biologischen Lumineszenz oberhalb oder unterhalb eines spezifischen Schwellenwertes besteht.

20. Verfahren nach einem der Ansprüche 1 bis 18, wobei das bestimmte Auswahlkriterium in einer spezifischen Lokalisation eines fluoreszenten Signals besteht.

21. Verfahren nach Anspruch 1, wobei die Zellen ausgewählt sind aus der Gruppe bestehend aus Säugetierzellen.

22. Verfahren nach einem der Ansprüche 7 bis 10 und 14 bis 16, wobei die Zellen, die an die Harzkügelchen angelagert sind, eine Nukleinsäure umfassen, die eine ein Zelloberflächenmolekül kodierende erste Nukleotidsequenz umfasst, die mit einer zweiten Nukleotidsequenz funktionsbereit verbunden ist, die naturgemäß nicht damit verbunden vorliegt, die die Expression der ersten Sequenz regelt.

23. Verfahren nach Anspruch 1, wobei mindestens 100 Harzkügelchen mit unterschiedlichen Bibliotheksmitgliedem bereitgestellt werden.

24. Verfahren nach Anspruch 1, wobei die Bibliothek ausgewählt ist aus der Gruppe bestehend aus Bibliotheken natürlicher Oligomere, wie beispielsweise Peptiden, Glykopeptiden, Lipopeptiden, Nukleinsäuren (DNS oder RNS), oder Oligosacchariden, nicht natürlichen Oligomeren wie beispielsweise chemisch modifizierten Peptiden, Glykopeptiden, Nukleinsäuren (DNS oder RNS) oder Oligosacchariden, und kleinen organischen Molekülen.

25. Verfahren nach Anspruch 1, wobei die Bibliothek eine Bibliothek aus kleinen organischen Molekülen ist.

26. Verfahren nach Anspruch 1, wobei Verbindungen identifiziert werden, die mindestens zwei zelluläre Antworten modifizieren, wobei Schritt (c) ein Durchmustern der Harzkügelchen für Kügelchen einbezieht, die Zellen umfassen, die mindestens zwei bestimmte Auswahlkriterien verfüllen, wobei jedes Auswahlkriterium auf ein unterschiedliches detektierbares Ergebnis bezogen ist.

27. Verfahren nach Anspruch 1, wobei das Harzkügelchen Polyethylenglycol umfasst oder daraus besteht.

28. Zelladhäsionsverbindung ausgewählt unter entweder:
i) der Gruppe bestehend aus Peptiden von SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 und SEQ ID 70,
oder
ii) einem Peptid, das mindestens eine D-Form Aminosäure umfasst, wobei das Peptid ausgewählt ist aus der Gruppe bestehend aus:
SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 und SEQ ID 70.

29. Harzkügelchen umfassend eine Zelladhäsionsverbindung ausgewählt aus der Gruppe bestehend aus SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69 und SEQ ID 70.

30. Harzkügelchen nach Anspruch 29, wobei das Harzkügelchen Polyethylenglycol umfasst.

## Revendications

1. Procédé d'identification d'un composé modifiant au moins une réponse cellulaire, dans lequel chaque réponse cellulaire est liée à différents systèmes rapporteurs générant des produits détectables, ledit procédé comprenant les étapes consistant à :
(a) fournir de multiples supports solides capables de promouvoir l'adhérence et la croissance des cellules, dans lequel chaque support solide est couplé à un membre d'une bibliothèque de composés de test, et dans lequel au moins deux supports solides comprennent des membres de bibliothèque différents ; et
(b) fixer les cellules comprenant le ou lesdits systèmes rapporteurs sur ledit support solide ;
où
- les cellules sont directement fixées au support solide, et/ou
- au moins 10 % des supports solides comprennent des molécules d'adhésion cellulaires ainsi que ledit membre de bibliothèque, et les cellules adhèrent auxdites molécules d'adhésion cellulaire ; et
(c) cribler lesdits supports solides pour rechercher les supports solides comprenant des cellules répondant à au moins un critère de sélection prédéterminé, où ledit critère de sélection est lié directement ou indirectement audit produit détectable ; et
(d) choisir les supports solides comprenant les cellules répondant à au moins un critère de sélection ; et
(e) identifier ledit élément de bibliothèque, pour identifier ainsi un composé modifiant ladite au moins une réponse cellulaire.

2. Procédé selon la revendication 1, dans lequel ladite adhérence est induite via un composé d'adhésion cellulaire couplé audit support solide, où ledit composé d'adhésion cellulaire permet audit support solide de promouvoir la croissance des cellules.

3. Procédé selon l'une des revendications 1 et 2, dans lequel les supports solides sont des billes de résine.

4. Procédé selon l'une des revendications 1 et 2, dans lequel les supports solides sont des emplacements ou des régions sur une surface ou un gel sur plaque ou une membrane.

5. Procédé selon la revendication 2, dans lequel ledit composé d'adhésion cellulaire est un peptide avec une charge nette globale positive.

6. Procédé selon la revendication 5, dans lequel ledit composé d'adhésion cellulaire est choisi parmi le groupe constitué par SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27 SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56 SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69, SEQ ID 70.

7. Procédé selon la revendication 1, dans lequel ladite réponse cellulaire est la modulation d'un mode de transduction de signal induite par une molécule de surface cellulaire.

8. Procédé selon la revendication 7, dans lequel ladite molécule de surface cellulaire est un récepteur couplé aux protéines G (GPCR).

9. Procédé selon la revendication 8, dans lequel ledit GPCR est choisi parmi le groupe constitué par les GPCR du Tableau 3.

10. Procédé selon la revendication 1, dans lequel la réponse cellulaire est la modulation de l'activité de transcription.

11. Procédé selon la revendication 1, dans lequel la réponse cellulaire est le changement de la teneur intracellulaire en un composé.

12. Procédé selon la revendication 1, dans lequel la réponse cellulaire est la relocalisation d'un composé.

13. Procédé selon la revendication 1, dans lequel le système rapporteur est un système endogène auxdites cellules.

14. Procédé selon la revendication 1, dans lequel le système rapporteur comprend un acide nucléique comprenant une séquence de nucléotides codant pour un polypeptide détectable lié de façon opérationnelle à un élément de réponse, et dont l'activité est modulée par la réponse cellulaire.

15. Procédé selon l'une des revendications 7 à 10 et 14, dans lequel le système rapporteur comprend un acide nucléique comprenant une séquence de nucléotides codant pour un polypeptide détectable lié de façon opérationnelle à un élément de réponse choisi parmi le groupe constitué par l'élément de réponse au AMPc (CRE) et l'élément de réponse au sérum (SRE).

16. Procédé selon l'une des revendications 7 à 10 et 14, dans lequel le système rapporteur comprend un acide nucléique comprenant une séquence de nucléotides codant pour un polypeptide détectable lié de façon opérationnelle à un élément de réponse de transcription (TRE).

17. Procédé selon l'une des revendications 1 à 16, dans lequel ledit polypeptide détectable est choisi parmi le groupe constitué par les protéines et les enzymes fluorescents.

18. Procédé selon la revendication 1, dans lequel le système rapporteur comprend un fragment bioluminescent.

19. Procédé selon l'une des revendications 1 à 18, dans lequel un critère de sélection prédéterminé est un niveau quantitatif de ladite bioluminescence au-dessus ou en-dessous d'un seuil spécifique.

20. Procédé selon l'une des revendications 1 à 18, dans lequel le critère de sélection prédéterminé est la localisation spécifique d'un signal fluorescent.

21. Procédé selon la revendication 1, dans lequel lesdites cellules sont choisies parmi le groupe constitué par les cellules de mammifères.

22. Procédé selon l'une des revendications 7 à 10 et 14 à 16, dans lequel les cellules fixées aux billes de résine comprennent une acide nucléique comprenant une première séquence de nucléotides codant pour ladite molécule de surface cellulaire liée de façon opérationnelle à une seconde séquence de nucléotides non associée naturellement avec celle-ci, dirigeant l'expression de ladite première séquence.

23. Procédé selon la revendication 1, dans lequel on fournit au moins 100 billes de résine comprenant différents membres de bibliothèques.

24. Procédé selon la revendication 1, dans lequel la bibliothèque est choisie parmi le groupe constitué par les bibliothèques d'oligomères naturels tels que les peptides, les glycopeptides, les lipopeptides, les acides nucléique (ADN ou ARN), ou les oligosaccharides ; les oligomères non naturels tels que les peptides, glycopeptides, acides nucléides (ADN ou ARN) ou les oligopeptides modifiés chimiquement ; et les petites molécules organiques.

25. Procédé selon la revendication 1, dans lequel la bibliothèque est une bibliothèque de petites molécules organiques.

26. Procédé selon la revendication 1, dans lequel on identifie les composés modifiant au moins deux réponses cellulaires, où l'étape c) consiste à cribler lesdites billes de résine pour rechercher les billes qui comprennent des cellules répondant à au moins deux critères de sélection prédéterminés, où chaque critère de détection est lié à un produit détectable différent.

27. Procédé selon la revendication 1, dans lequel la bille de résine comprend ou est constituée de polyéthylèneglycol.

28. Composé d'adhésion cellulaire choisi soit parmi :
i) le groupe constitué par les peptides provenant des séquences suivantes :
SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 26, SEQ ID 27 SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56 SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69, SEQ ID 70
ou
ii) un peptide comprenant au moins un acide aminé de forme D, ledit peptide étant choisi parmi le groupe constitué par :
SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27 SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56 SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69, SEQ ID 70.

29. Bille de résine comprenant un composé d'adhésion cellulaire choisi parmi le groupe constitué par :
SEQ ID 1, SEQ ID 2, SEQ ID 3, SEQ ID 4, SEQ ID 5, SEQ ID 6, SEQ ID 7, SEQ ID 8, SEQ ID 9, SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27 SEQ ID 28, SEQ ID 29, SEQ ID 30, SEQ ID 31, SEQ ID 32, SEQ ID 33, SEQ ID 34, SEQ ID 35, SEQ ID 46, SEQ ID 47, SEQ ID 48, SEQ ID 49, SEQ ID 50, SEQ ID 51, SEQ ID 52, SEQ ID 53, SEQ ID 54, SEQ ID 55, SEQ ID 56 SET ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69, SEQ ID 70.

30. Bille de résine selon la revendication 29, dans laquelle ladite bille de résine comprend du polyéthylèneglycol.
